# EUROPEAN PATENT APPLICATION

(11) **EP 4 560 030 A2**
(43) Date of publication of application: **28.05.2025**
(21) Application number: 25165566.8
(22) Date of filing: 27.08.2021
(51) Int. Cl.: C12Q 1/6897

(54) **PREDICTING CANCER RELAPSE AND TREATMENT OF CANCER DISEASES**

(30) Priority: 02.09.2020 SE 2051035
(62) Divisional of application: 21766247.7
(71) Applicant: Johansson Swartling, Fredrik, 756 47 Uppsala (SE)
(72) Inventor: JOHANSSON SWARTLING, Fredrik, 756 47 Uppsala (SE); HUTTER, Sonja, 756 43 Uppsala (SE)
(74) Representative: Barker Brettell Sweden AB

(57) **Abstract**

A likelihood of cancer relapse is estimated for a subject by introducing *ex vivo* a nucleic acid molecule comprising a reporter gene under transcriptional control of a SOX9p or of a promoter operatively linked to an enhancer element comprising at least one SOX9 binding site into cancer cells obtained from a tumor sample from a subject. The cancer cells are exposed *ex vivo* to a cancer treatment and the amount of SOX9+ cancer cells following cancer treatment is determined based on expression of the reporter gene. The likelihood of cancer relapse following cancer treatment for the subject is estimated based on the determined amount of SOX9+cancer cells. A combination of a viral vector comprising a suicidal gene under transcription control of a SOX9p or of a promoter operatively linked to an enhancer element comprising at least one SOX9 binding site and a prodrug can be used in treatment of a subject suffering from a cancer disease.

## Description

### TECHNICAL FIELD

The present invention generally relates to cancer diseases, and in particular to methods of estimating the likelihood of cancer relapse and predicting a response to cancer treatment, and viral vectors and medicaments useful in treatment of cancers.

### BACKGROUND

Recurrence and metastases are major barriers to therapeutic success in cancer diseases, and relapse is responsible for a high percentage of cancer associated deaths. For instance, cancer relapse accounts for 95 % of brain cancer associated deaths.

Medulloblastoma (MB) is the most common malignant brain tumor in children. MB is classified into four subgroups, wingless (WNT), Sonic hedgehog (SHH), Group 3 and Group 4, which are clinically, transcriptionally, and genetically distinct. The most common genetic alteration in MB includes amplification or overexpression of the MYC and MYCN cancer gene family members. Leptomeningeal spread occurs in up to 40 % of the patients at time of diagnosis. Despite significant progress in MB management, recurrent disease remains almost always fatal, with recurrent and metastatic tumor cells evading both re-irradiation and high-dose chemotherapy regimens. In fact, in MB, the major cause of death is due to relapse that occurs in 20-30 % of the patients. Patients who relapse rarely survive (<10 %) despite multimodal treatment options. Patients with Group 3 and 4 MB die almost exclusively as a result of metastatic disease, which suggests that therapy-resistant clones are present in the metastatic compartments.

Glioblastoma (GBM) is the most common malignant adult brain tumor. It has dismal prognosis despite aggressive irradiation and chemotherapy. The heterogeneity of the tumors likely contributes to failure and poor outcomes of numerous clinical trials of this disease. Less than 5 % of the patients survive five years after diagnosis despite aggressive treatment. It is believed that all brain cancer relapses are caused by clonal selection and expansion of therapy-resistant clones.

Hence, recurrent cancer diseases of MB and GBM represent a tremendous treatment challenge.

Emergence of therapy resistance is a major cause of treatment failure with conventional chemotherapy and radiation. It is imperative to find novel options to target therapy-resistant tumor populations.

WO 95/09654 discloses a method of treating an adverse condition of the nervous system. The method comprises administering, to the cerebrospinal fluid of the host, an expression vehicle capable of transducing a cell in order to express a therapeutic agent in the nervous system. The expression vehicle includes a nucleic acid sequence encoding a therapeutic agent for treating an adverse condition of the nervous system, and is administered in an amount effective in treating the adverse condition of the nervous system.

WO 95/05835 discloses a method of treating localized solid tumors and papillomas in an individual, as well as metastatic carcinomas. The method comprises delivering a suicide gene, by way of a recombinant adenoviral vector or other DNA transport system, into the tumor, papilloma or wart of an individual. Subsequently, a prodrug is administered to the individual.

US 6,241,982 discloses recombinant viral vectors carrying a vector construct which directs the expression of a gene product that activates a compound with little or no cytotoxicity into a toxic product. Also a method of destroying or inhibiting pathogenic agents in a warm blooded animal is disclosed. The method comprises administering to the animal a viral vector as described above in order to inhibit or destroy the pathogenic agent.

There is still a need to identify patients likely to suffer from cancer relapse and to provide efficient treatments to such patients.

### SUMMARY

It is an objective to provide methods of estimating the likelihood of cancer relapse and predicting a response to cancer treatment.

It is another objective to provide viral vectors and medicaments useful in treatment of cancer diseases.

These and other objectives are met by embodiments as disclosed herein.

The present invention is defined in the independent claims. Further embodiments of the present invention are defined in the dependent claims.

An aspect of the invention relates to a method of estimating a likelihood of cancer relapse for a subject following cancer treatment. The method comprises introducing ex *vivo* a nucleic acid molecule comprising a reporter gene under transcriptional control of a SOX9 promoter (SOX9p) or of a promoter operatively linked to an enhancer element comprising at least one SOX9 binding site into cancer cells obtained from a tumor sample from a subject. The method also comprises exposing ex *vivo* the cancer cells to a cancer treatment. The method further comprises determining the amount of SOX9 positive (SOX9+) cancer cells following cancer treatment based on expression of the reporter gene and estimating a likelihood of cancer relapse following cancer treatment for the subject based on the determined amount of SOX9+ cancer cells.

Another aspect of the invention relates to a viral vector comprising a suicidal gene under transcriptional control of a SOX9p or of a promoter operatively linked to an enhancer element comprising at least one SOX9 binding site.

A further aspect of the invention relates to a medicament comprising a viral vector comprising a suicidal gene under transcriptional control of a SOX9p or of a promoter operatively linked to an enhancer element comprising at least one SOX9 binding site and a prodrug for use in treatment of a cancer disease in a subject. The suicidal gene encodes a prodrug activating protein capable of converting the prodrug or a metabolite thereof into a cytotoxic agent.

Yet another aspect of the invention relates to an *in vitro* method of predicting a response to a cancer treatment. The method comprises introducing ex *vivo* i) a nucleic acid molecule comprising a reporter gene under transcriptional control of a SOX9p or of a promoter operatively linked to an enhancer element comprising at least one SOX9 binding site and a suicidal gene under transcriptional control of a SOX9p or ii) a first nucleic acid molecule comprising the reporter gene under transcriptional control of a SOX9p or of a promoter operatively linked to an enhancer element comprising at least one SOX9 binding site and a second nucleic acid molecule comprising the suicidal gene under transcriptional control of a SOX9p or of a promoter operatively linked to an enhancer element comprising at least one SOX9 binding site into cancer cells obtained from a tumor sample from a subject. The suicidal gene encodes a prodrug activating protein capable of converting a prodrug or a metabolite thereof into a cytotoxic agent. The method also comprises exposing *ex vivo* the cancer cells to a cancer treatment and determining a first amount of SOX9+ cancer cells following cancer treatment based on expression of the reporter gene. The method further comprises adding *ex vivo* the prodrug to the cancer cells and determining a second amount of SOX9+ cancer cells following addition of the prodrug based on expression of the reporter gene. The method additionally comprises predicting a response of the subject to a cancer treatment based on the determined first and second amounts.

A related aspect of the invention defines a kit for use in the *in vitro* method according to above. The kit comprises i) a nucleic acid molecule comprising a reporter gene under transcriptional control of a *SOX9* promoter or of a promoter operatively linked to an enhancer element comprising at least one SOX9 binding site and a suicidal gene under transcriptional control of a SOX9 promoter or of a promoter operatively linked to an enhancer element comprising at least one SOX9 binding site, or ii) a first nucleic acid molecule comprising the reporter gene under transcriptional control of a SOX9 promoter or of a promoter operatively linked to an enhancer element comprising at least one SOX9 binding site and a second nucleic acid molecule comprising the suicidal gene under transcriptional control of a *SOX9* promoter or of a promoter operatively linked to an enhancer element comprising at least one SOX9 binding site. The kit also comprises a prodrug. The suicidal gene encodes a prodrug activating protein capable of converting the prodrug or a metabolite thereof into a cytotoxic agent.

A further aspect of the invention relates to a method of treating a subject suffering from a cancer disease. The method comprises administering a viral vector according to above to the subject prior to, during or following exposing the subject to a cancer treatment. The method also comprises administering a prodrug to the subject. The suicidal gene encodes a prodrug activating protein capable of converting the prodrug or a metabolite thereof into a cytotoxic agent.

The present invention enables identification of subjects likely to suffer from cancer relapse and have dormant and quiescent cancer cells that can escape standard cancer treatments but may convert into mobile and metastasizing cancer cells. The invention also relates to viral vectors and medicaments useful in targeting such dormant and quiescent cancer cells and can thereby be used to treat cancer diseases, including preventing or inhibiting cancer relapse.

### BRIEF DESCRIPTION OF THE DRAWINGS

The embodiments, together with further objects and advantages thereof, may best be understood by making reference to the following description taken together with the accompanying drawings, in which:

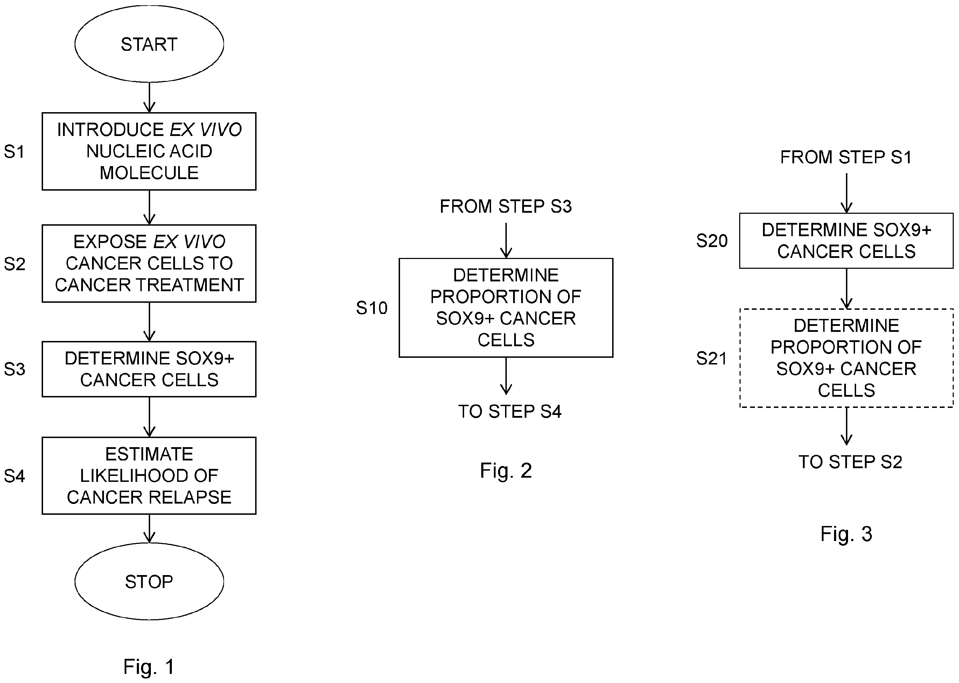

Fig. 1 is a flow chart illustrating a method of estimating a likelihood of cancer relapse according to an embodiment.
Fig. 2 is a flow chart illustrating an additional, optional step of the method in Fig. 1 according to an embodiment.
Fig. 3 is a flow chart illustrating additional, optional steps of the method in Fig. 1 according to an embodiment.

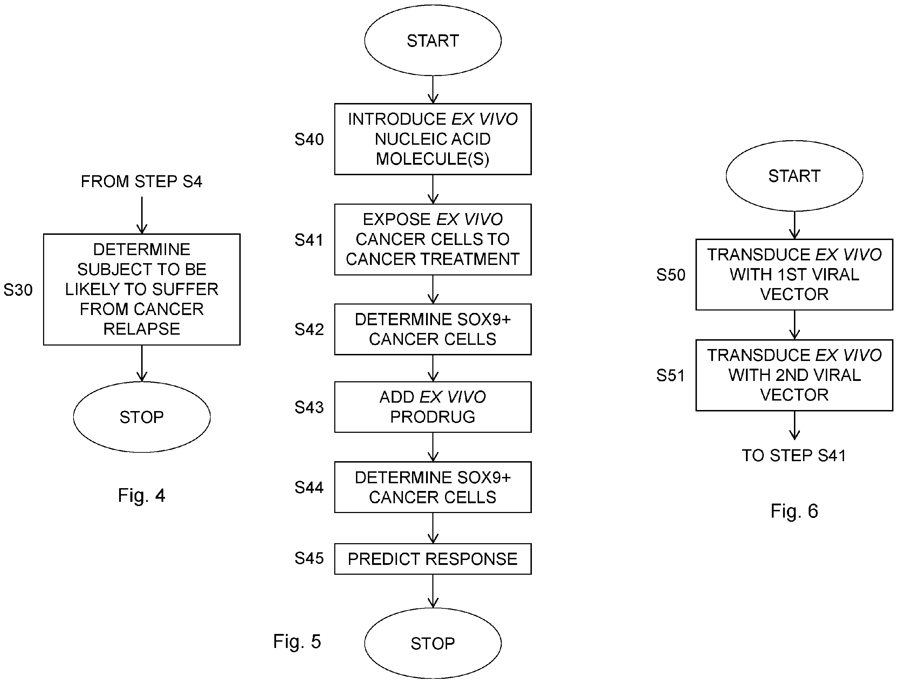

Fig. 4 is a flow chart illustrating an additional, optional step of the method in Fig. 1 according to another embodiment.
Fig. 5 is a flow chart illustrating an *in vitro* method of predicting a response to a cancer treatment according to an embodiment.
Fig. 6 is a flow chart illustrating an embodiment of the introducing step in Fig. 5;

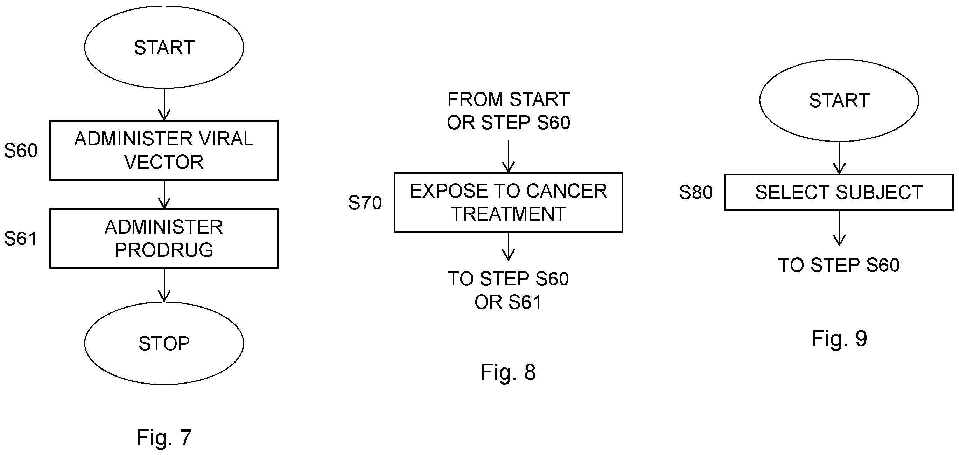

Fig. 7 is a flow chart illustrating a method of treating a subject suffering from a cancer disease according to an embodiment.
Fig. 8 is a flow chart illustrating an additional, optional step of the method in Fig. 7 according to an embodiment.
Fig. 9 is a flow chart illustrating an additional, optional step of the method in Fig. 7 according to another embodiment.

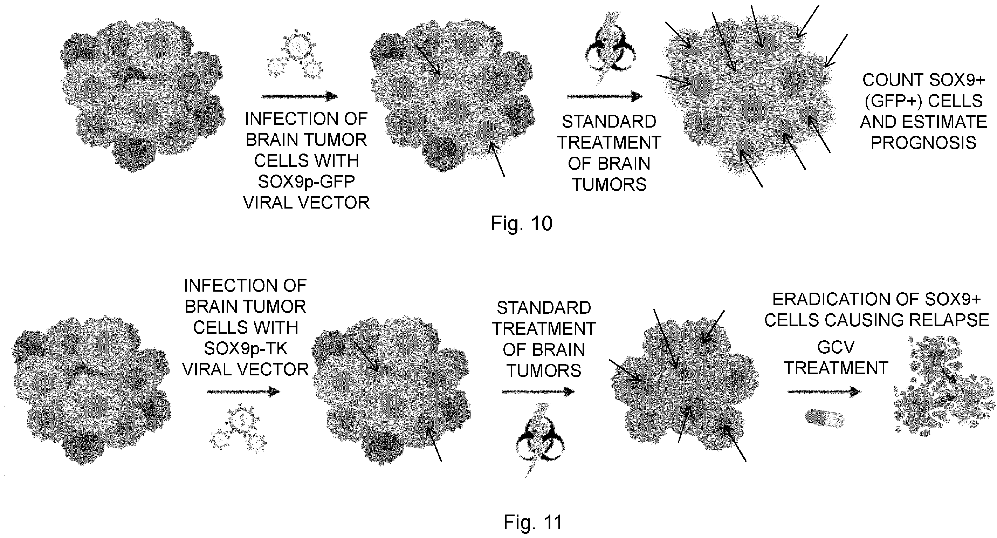

Fig. 10 is a schematic overview of labeling SOX9+ cancer cells for estimation of a likelihood of cancer relapse, SOX9+ cancer cells are marked with arrows.
Fig. 11 is a schematic overview of targeting SOX9+ cancer cells for predicting a response to cancer treatment, SOX9+ cancer cells are marked with arrows.

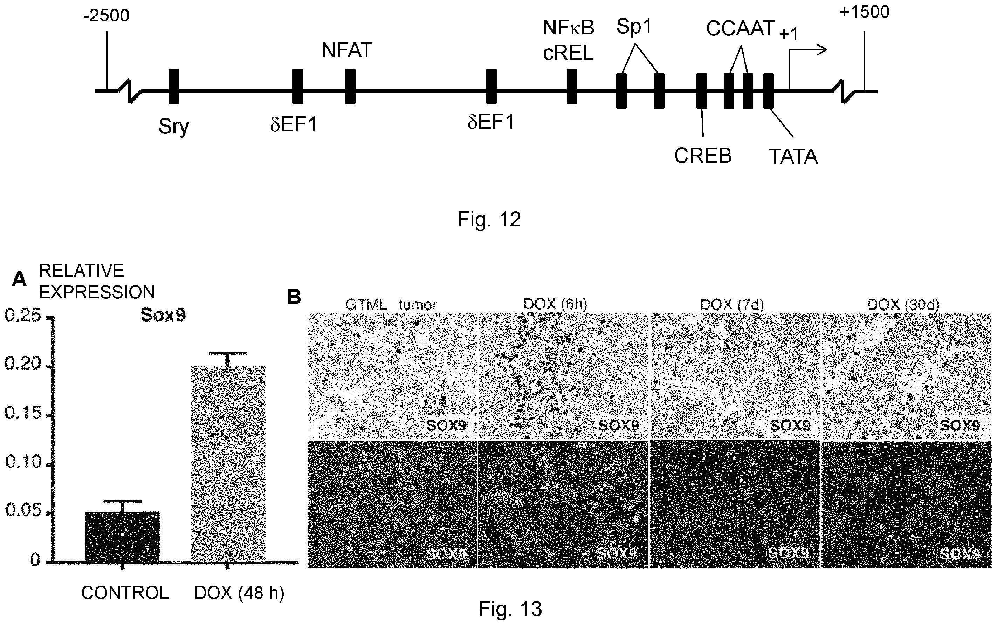

Fig. 12 is a schematic illustration of the full length human SOX9 promoter.

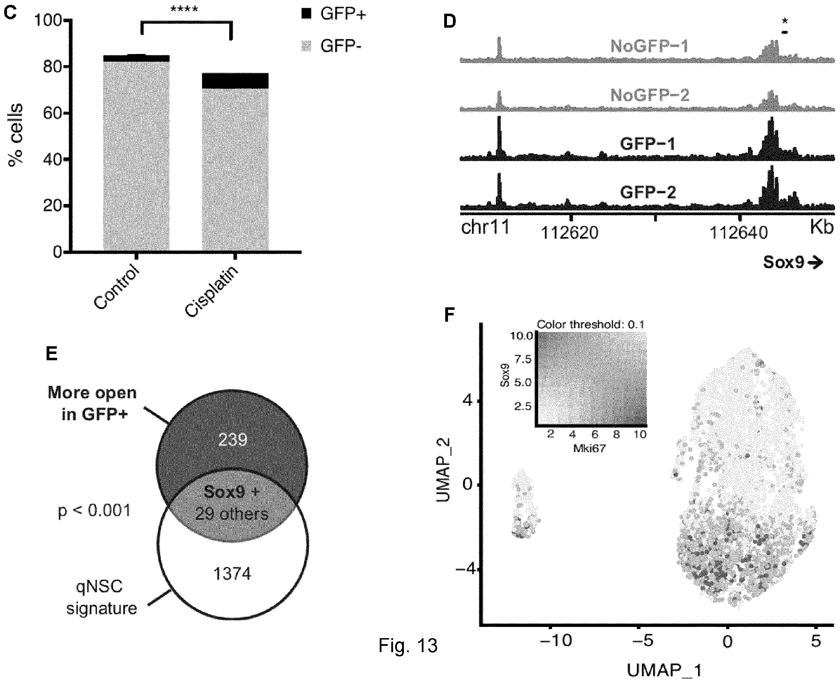

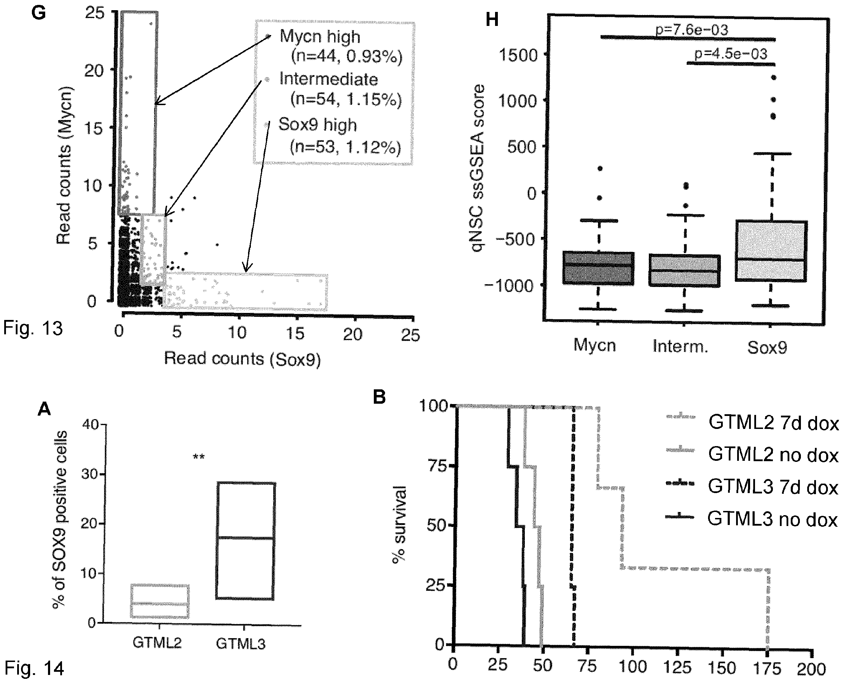

Fig. 13 SOX9 is upregulated by loss of the cancer gene MYCN and is induced in therapy-resistant dormant cells. (A) RT-PCR shows an increased expression of SOX9 in the surviving cell population of transgenic Glt1-driven Tet/dox-regulated MYCN-driven luciferase (GTML) mouse medulloblastoma cells after 48 h *in vitro* dox treatment. (B) IF staining of GTML tumor after 6 h *in vivo* dox administration that turns MYCN off. The analysis shows an increase of non-proliferating SOX9 expressing cells in the GTML model. (C) Quantification of GFP-positive cells using FACS shows that 24 h cisplatin treatment of GTML-pSOX9-GFP cells increases the number of SOX9-positive cells compared to DMSO control. (D) ATACseq of GTML-pSOX9-GFP cells FACS sorted for GFP (SOX9 expression). There is more open chromatin at the SOX9 promoter in GFP-positive cells compared to GFP-negative. (E) Genes with higher ATACseq peaks in GFP-positive GTML-pSOX9-GFP cells significantly (p<0.05; Fishers's exact test) overlap with a quiescent signature. (F) Umap plot of SOX9 and KI67 in GTML cells shows SOX9-positive cell and KI67-positive cell are exclusive from each other. (G) Single cell sequencing of GTML cells indicates that high MYCN and SOX9 expression is largely mutually exclusive. The top ~1 % of cells with highest MYCN and SOX9 expression, respectively, and ~1 % of cells with intermediary expression were selected for downstream analyses. (H) Boxplot comparing the ssGSEA scores for a quiescent NSC signature between the groups from (G), indicating that SOX9 high GTML cells appear significantly (p<0.05, Welch's t-test) more quiescent than MYCN-high and intermediate cells.

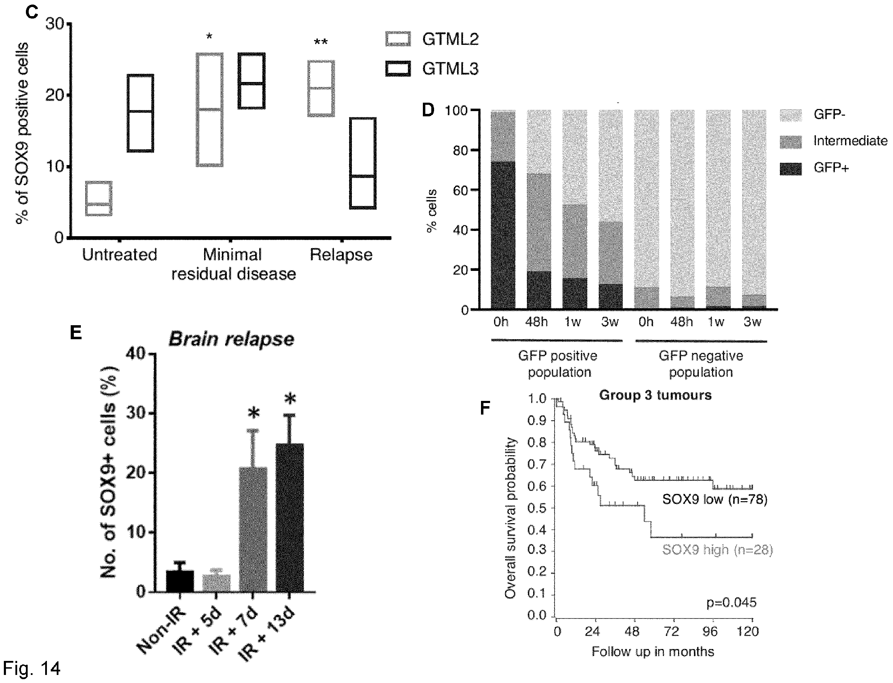

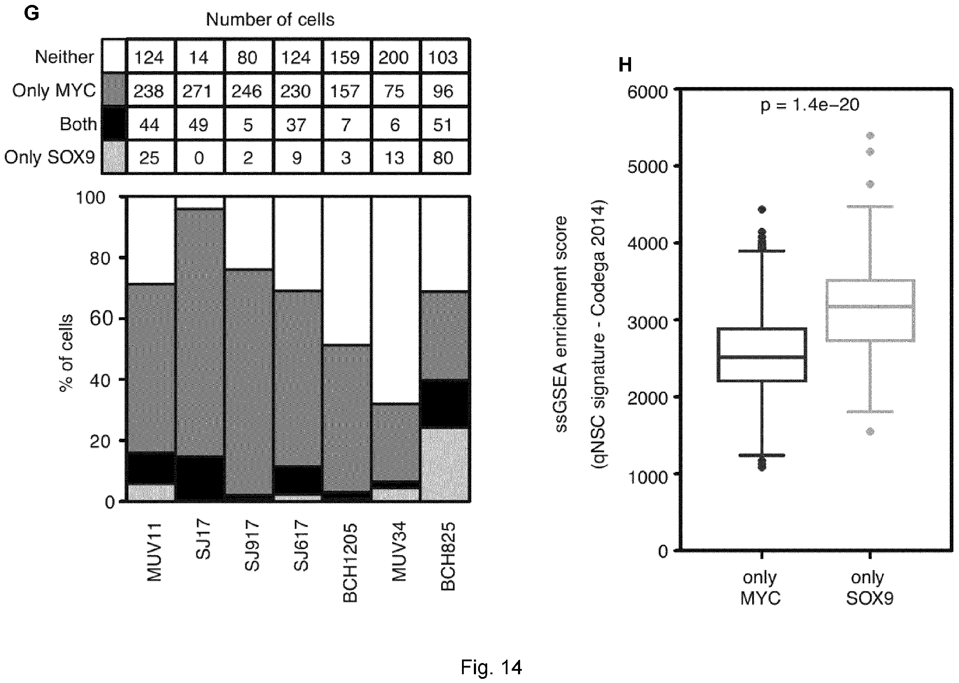

Fig. 14 SOX9 increases in residual disease models and marks quiescent cells in Group 3 MB patients. (A) Quantification of IHC for SOX9 in tumor-bearing allografted Nude mice. Tumors generated from the GTML3 cell line had significantly higher levels of SOX9 protein compared to GTML2. (B) Kaplan-Meier plot of the minimal residual disease treatment groups 7 days on Dox diet or on regular diet. Tumors generated from GTML3 cell line presented with tumors faster than GTML2. (C) Quantification of IHC for SOX9 positive cells from (B) in tumor bearing allografted Nude mice showing all Dox treatment groups; 7 days, 7 days plus 10 days after treatment and at relapse. (D) Quantification of GFP-positive, negative and intermediate cells in FACS sorted GTML-pSOX9-GFP cell line, directly after sorting, 48 h, 1 week and 3 weeks after sorting. The number of GFP-positive cells in the positive population quickly drops while it slowly increases in the negative population. (E) Following transplantation of 10⁵ cells of a GTML cell line (GTML2) into the hindbrain of adult mice, we irradiated them for 3.5 weeks (2Gy per day) using a total radiation of 36 Gy. More efficiently targeted tumors (that relapse later) of the same cell line show a significant accumulation (p<0.05; paired Student's t-test) of the number of SOX9 positive cells. (F) Kaplan-Meier plot showing 10-year overall survival in Group 3 MB with high SOX9 levels (n=28) and low SOX9 levels (n=78) from Cancer Cell 2017, 31: 737-754.e736 at last quartile cut off, excluding MB with extensive nodularity or tumors with desmoplastic histology. High SOX9 levels correlate to a poor overall survival of Group 3 MB. (G) The total numbers and percentages of cells with any (>0) expression of either MYC, SOX9, both or none in diagnostic Group 3 MBs (data from Nature 2019, 572: 74-79). (H) Cells from (F) with only SOX9 expression display a significantly (p<0.05; Welch's t-test) higher ssGSEA score for a quiescent NSC signature as compared to cells with only MYC expression.

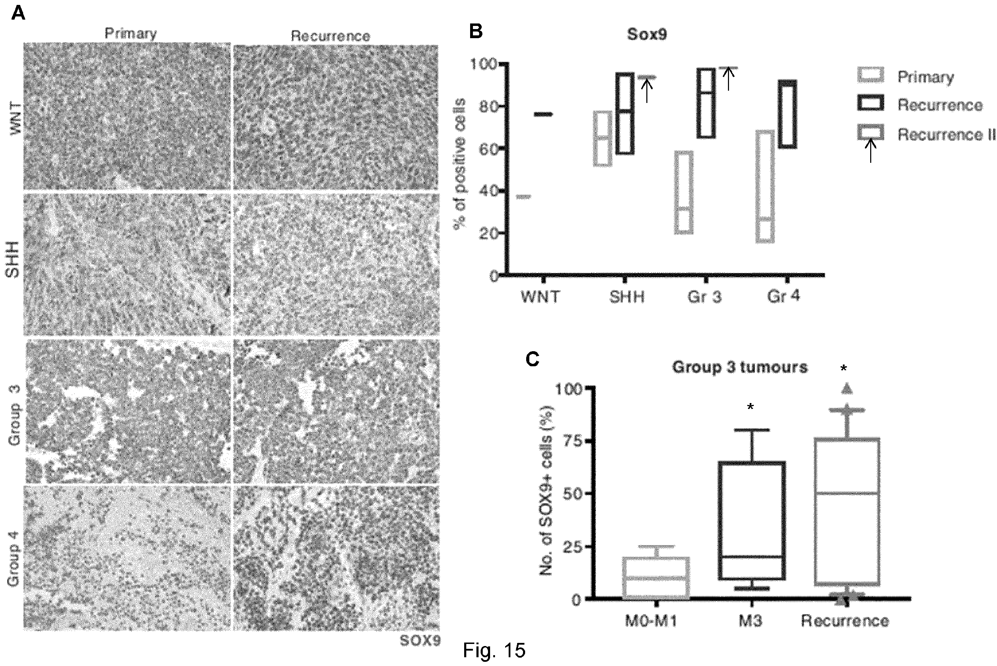

Fig. 15 SOX9 accumulates in metastatic disease and in relapses of paired primary-recurrent MB. (A) Representative images of IHC SOX9 staining in primary and recurrent tumors of all four MB subgroups. (B) Quantification of SOX9-positive cells in IHC of WNT (n=1), SHH (n=3), Group 3 (n=3) and Group 4 (n=3) MB. The number of SOX9-positive cells is significantly higher in recurrent Group 3 and 4 MB compared to primary tumor. (C) Percentage of SOX9 levels in low (M0-M1) and high (M3) stage metastatic Group 3 MB as well as recurrent tumors of the same subgroup from EMBO J 2016, 35: 2192-2212. SOX9 protein levels are higher in distant metastases (M3 stage) and recurrent Group 3 MB than in primary M0-M1 stage tumor.

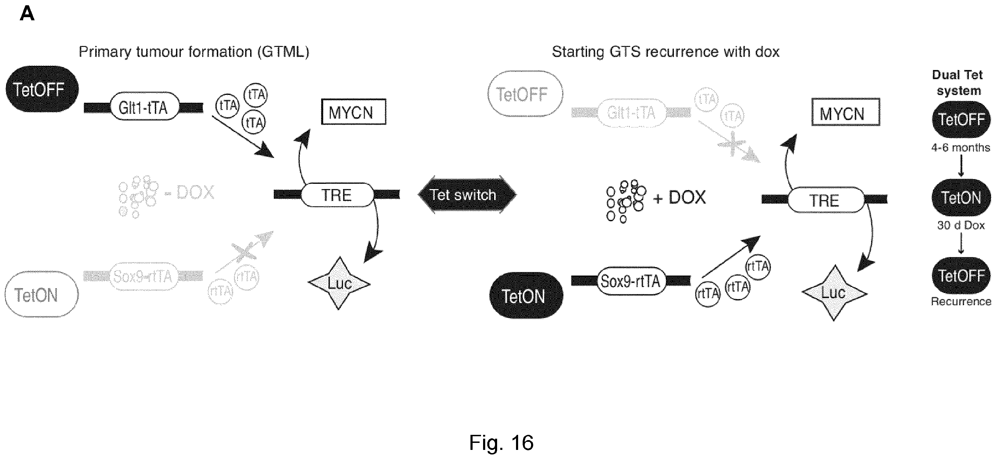

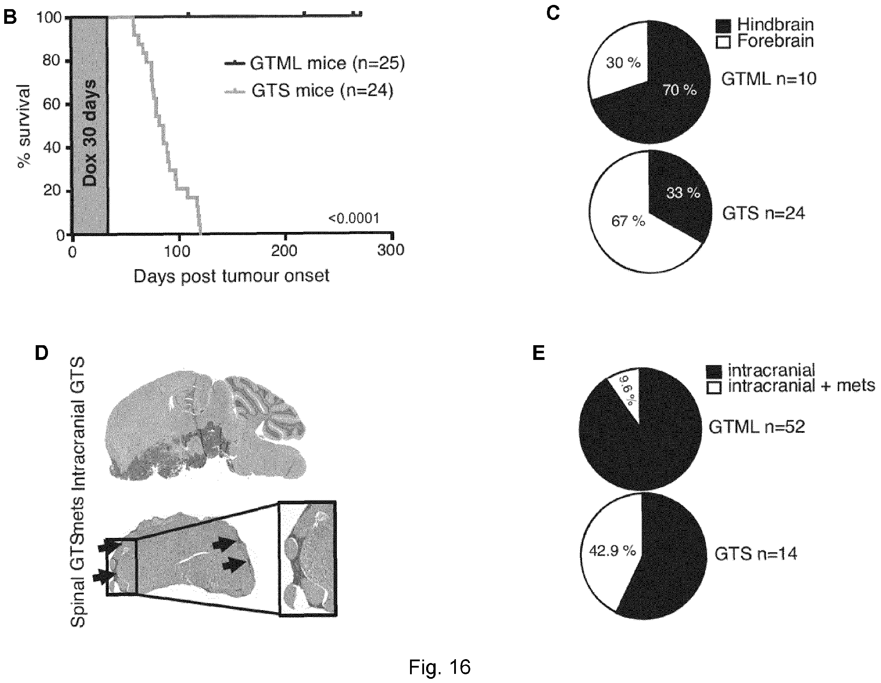

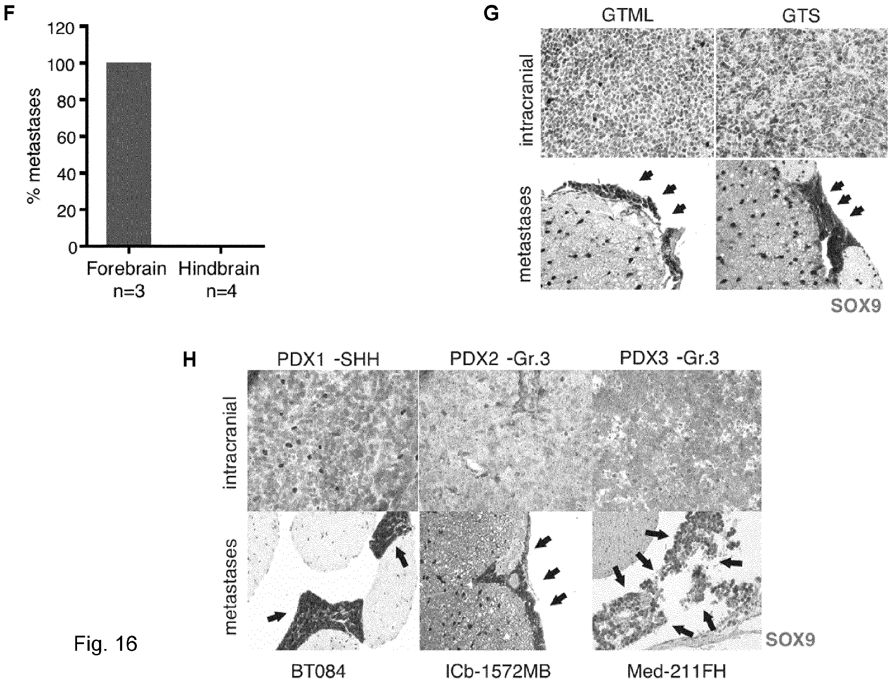

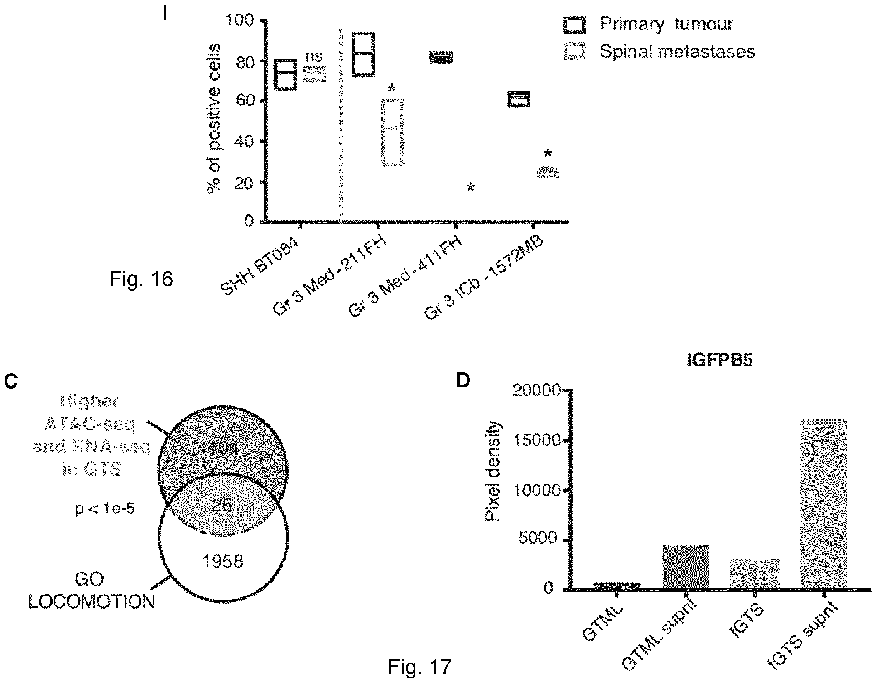

Fig. 16 The recurrence process is reproduced in a novel TetON/TetOFF transgenic GTS model. (A) Schematics of the GTS recurrence model. (left) In the absence of Dox the TML (TRE-MYCN-Luc) transgene is expressed in Glt1 positive cells leading to MB tumor formation in the GTML model. Dox administration will halt MYCN expression (tetOFF) in the Glt1-positive cell population. (right) When combining this with a tetON model, Dox will at the same time force expression of the TML transgene in SOX9-positive cells. (B) Scheme of dox treatment and mouse survival in the GTS recurrence model. Primary tumor (GTML) is treated with and cured after 30 days of Dox administration. Tumors recur after 80-120 days post primary tumor onset. (C) Quantification of tumor localization in the GTML and GTS models. 70% of GTS recurrences are located in the mouse forebrain in contrast to GTML tumors that most often are located in hindbrain. (D) H&E staining of a whole mouse brain with relapsed GTS tumor. The tumor recurrences are most often situated in the forebrain. Spinal cord metastasis can be found in the GTS relapsed tumors. Arrows are pointing at the metastatic cells surrounding the spinal cord. (E) Only 9.6% spinal metastases (mets) where identified in biopsies of H&E-stained sectioned spinal cords of GTML samples, as compared to 42.9% mets found in biopsies of GTS samples. (F) Histogram comparing occurrence of spinal metastases in GTS recurrences in hindbrain or forebrain. All spinal metastases are found in forebrain relapses. (G) (top) SOX9 protein levels in primary GTML and GTS tumor as compared to (bottom) SOX9 levels in disseminated tumor cells in corresponding spinal cords from representative individual mice with metastatic spread (marked by arrows). (H) (top) IHC of SOX9 protein in primary intracranial location (cerebellum) of transplanted PDX in NSG mice with few, scattered SOX9 positive cells as compared to (bottom) corresponding spinal cords with a majority of SOX9 positive cells from representative individual animals with confirmed metastatic spread (marked by arrows). (I) The number of Ki67-positive (dividing) cells in the primary cerebellar tumor versus Ki67-positive tumor cells in corresponding metastases in spinal cord evaluated in 3 different regions per site and PDX model. Metastases of Group 3 MB PDXs are less proliferative. Significant difference between columns (p<0.05) using Students t-test is marked by stars (*<0.05; ***<0.001). ns = not significant.

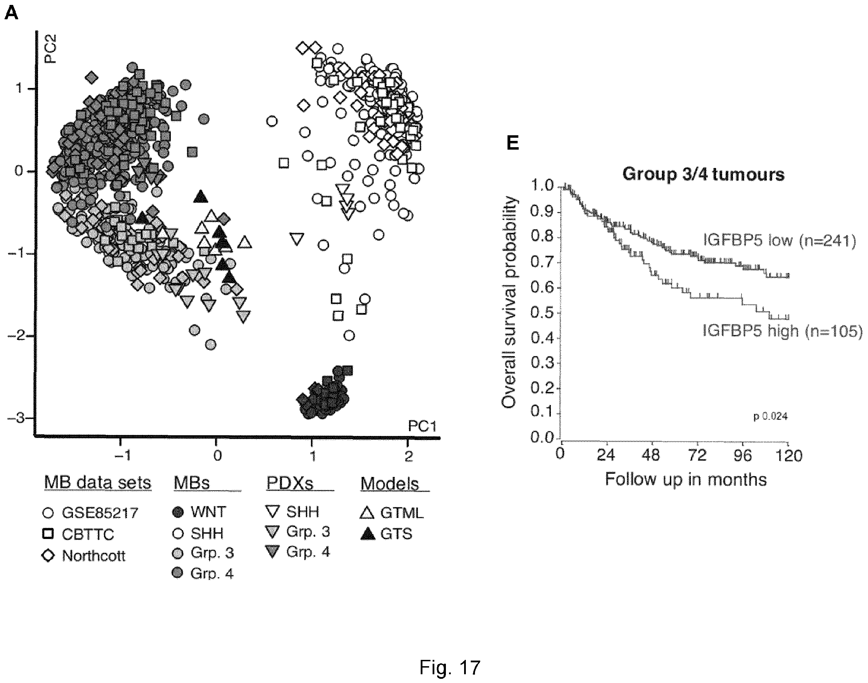

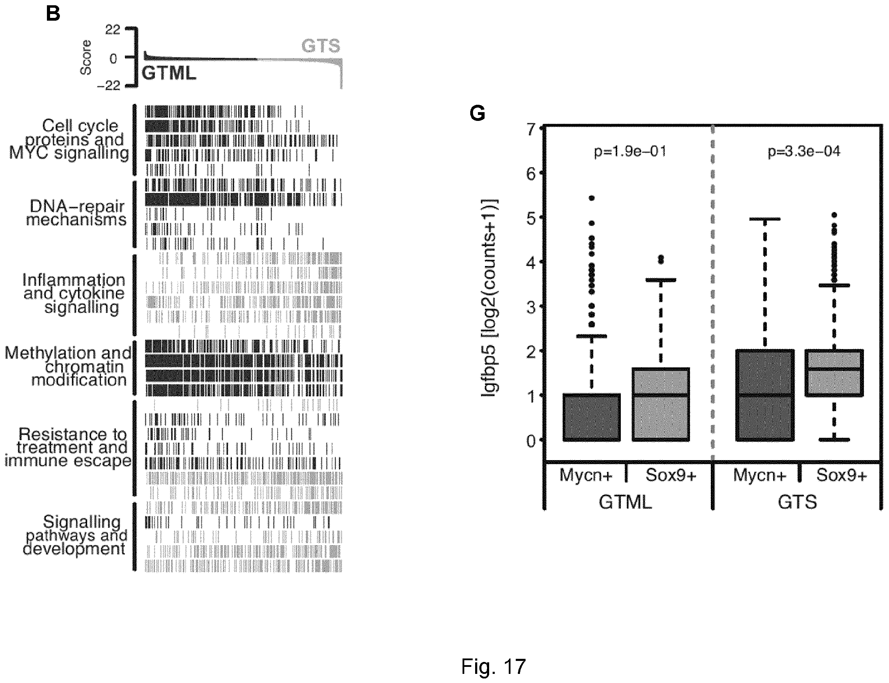

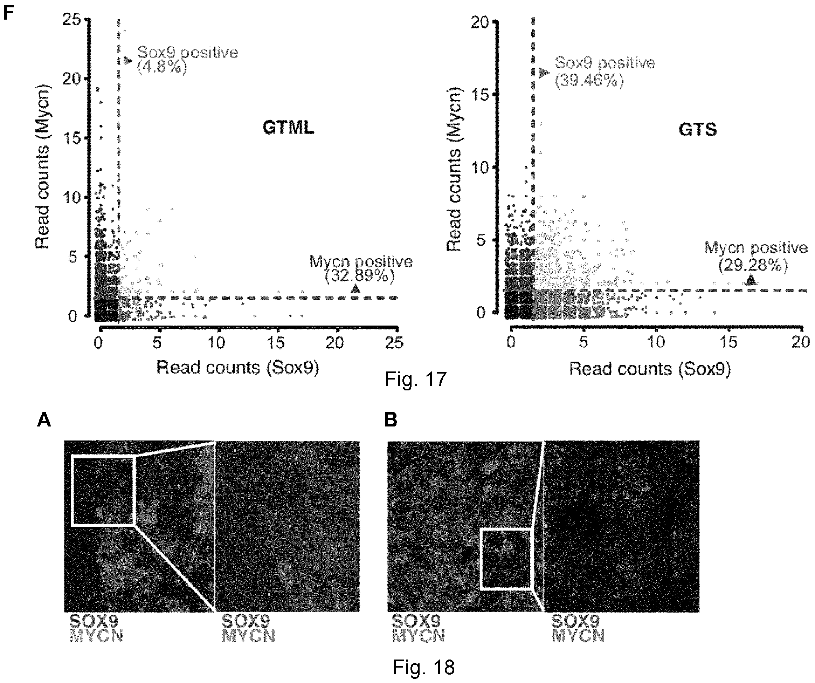

Fig. 17 Recurrent MBs molecularly resemble primary MBs but are more inflammatory. (A) PCA plot showing the affiliation of primary GTML tumors and recurrent GTS tumors with expression profiles of MB subgroups (data from Cancer Cell 2017, 31: 737-754.e736, CBTTC: Nature 2017, 547: 311-317 and MB PDXs (data from PMID: Cell Stem Cell 2019, 25: 855-870 e811), following cross-platform projection via the Metagene (Proc Natl Acad Sci U S A 2007, 104: 5959-5964) method. (B) GSEA results for 32 selected key gene sets from 6 pathway categories significantly enriched in either the GTS or GTML tumors. (C) GO_LOCOMOTION was one of the top significant gene sets overlapping the genes with significantly higher ATAC-Seq peaks and upregulated RNA-Seq expression in GTS cell lines. (D) Recurrent GTS tumor cells show elevated intrinsic as well as secreted levels of IGFBP5 compared to primary tumor cells when comparing lysates of cells or supernatants (supnt) in cytokine arrays. (E) High IGFBP5 levels correlate with a poor 10-year overall survival in Group 3 and Group 4 MB from Cancer Cell 2017, 31: 737-754.e736 at last quartile cut off. (F) Single cell sequencing read counts for MYCN and SOX9 in GTML (left) and GTS (right), indicating that the percentage of MYCN-positive (reads≥2) cells remains largely unchanged between the two tumor models, while the GTS tumor reveals an increased percentage of SOX9-positive cells. (G) Boxplot comparing the IGFBP5 expression between MYCN-positive and SOX9-positive cells from (F), demonstrating that in the GTS tumor IGFBP5 expression is significantly higher (p<0.05; Welch's t-test) in SOX9-positive cells as compared to MYCN-positive cells.

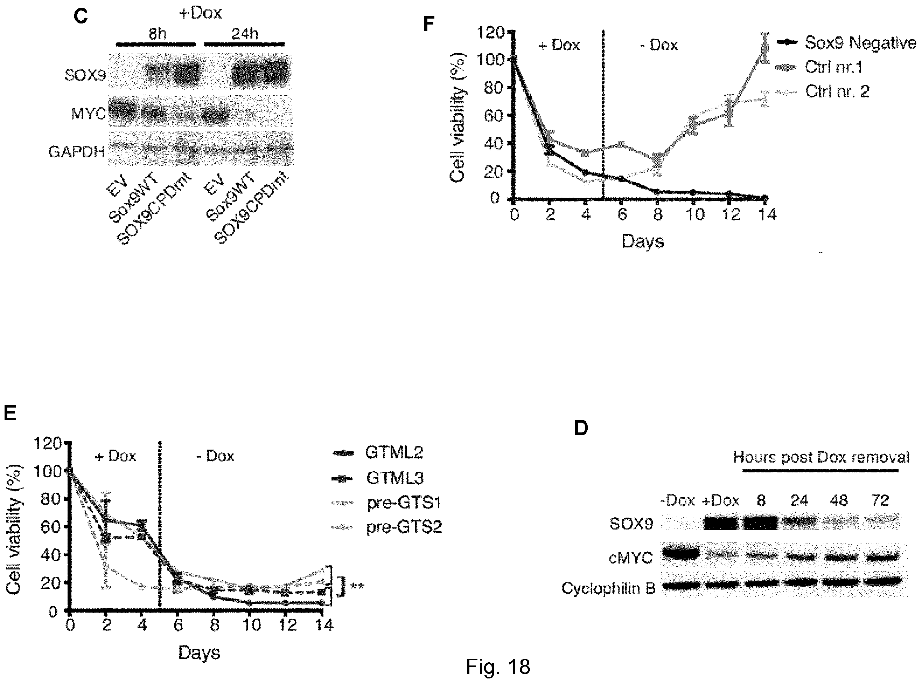

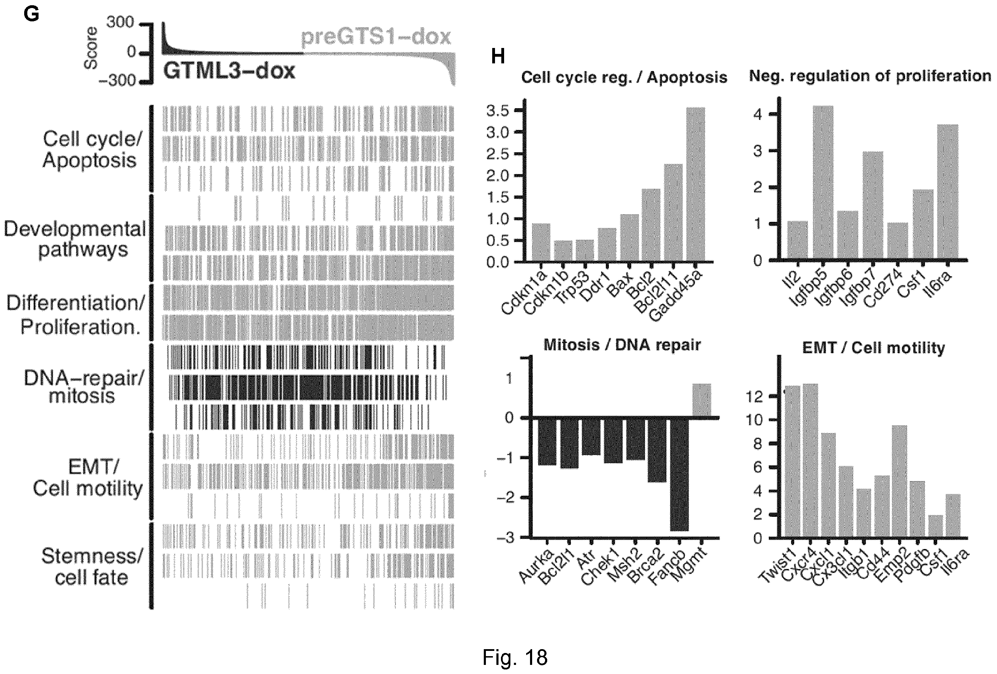

Fig. 18 SOX9 inhibits MYC in therapy-resistant cells and is essential for driving recurrence. (A) RNAscope staining in a representative primary GTML tumor (×40 magnification). The MYCN oncogene is expressed in the whole tumor while the SOX9 RNA can be seen only in few cells (×63 magnification). (B) RNAscope staining of a representative recurrent GTS tumor (×40 magnification). The recurrent tumor is similar for MYCN and SOX9 RNA expression. The SOX9 expressing cells are scarce in the tumor (×63 magnification). (C) MB002 cells were transduced with lentiviral vectors encoding inducible EV, SOX9WT or SOX9CPDmt. The transduced cell lines were treated with dox for 8 or 24 hours and stained for expression of SOX9, cMYC and GAPDH. After induction of SOX9, cMYC levels are decreasing. The decrease is more pronounced when the stabilized SOX9CPDmt is induced. (D) MB002-SOX9CPDmt treated with dox for 24 h (for SOX9 induction) after which they were resuspended in fresh media. SOX9, cMYC and cyclophilin B protein was analyzed with WB at 8, 24, 48 and 72 h after Dox removal. After 72 h, protein levels decreased but neither SOX9 nor cMYC was restored to normal levels. (E) Proliferation (Alamar blue assay) of GTML vs pre-GTS cells under long term dox treatment. pre-GTS cells start to recover after dox treatment, GTML cells do not. Mann-Whitney ** p<0.01. (F) Proliferation (Alamar blue assay) of CRISPR-Cas9 edited and control pre-GT20 cells under long term dox treatment. Control cells start to recover after dox treatment, SOX9 edited pre-GT20 cells (sgSox9) do not. (G) Enrichment plots of pathways significantly altered in 48 h dox-treated pre-GTS tumor cells compared to 48 h dox-treated GTML tumor cells. (H) Bar plots showing the logFC for selected genes from the gene sets depicted in (G) as calculated by edgeR between GTML and pre-GTS cells under dox treatment.

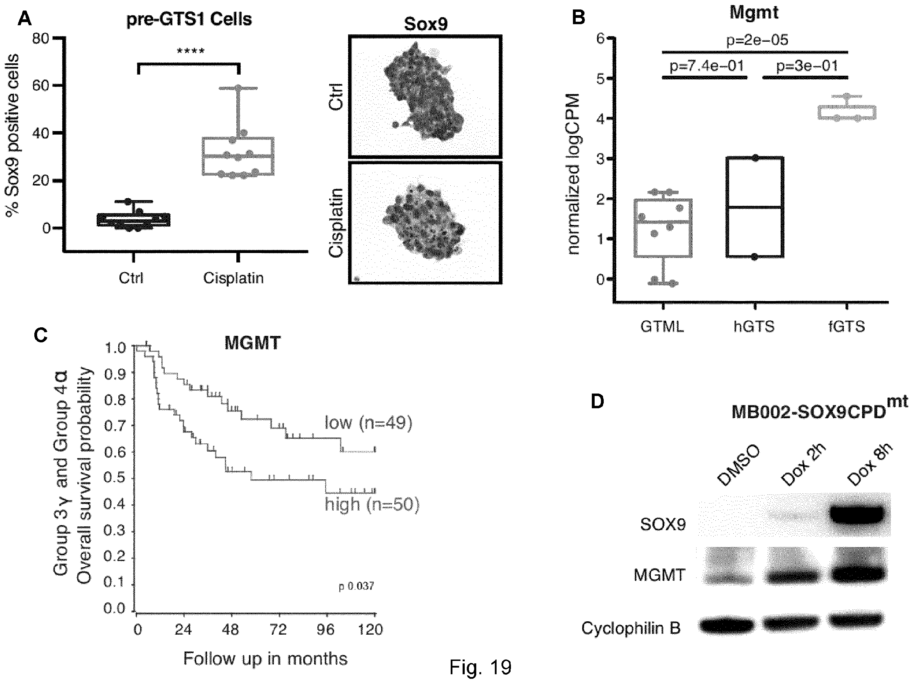

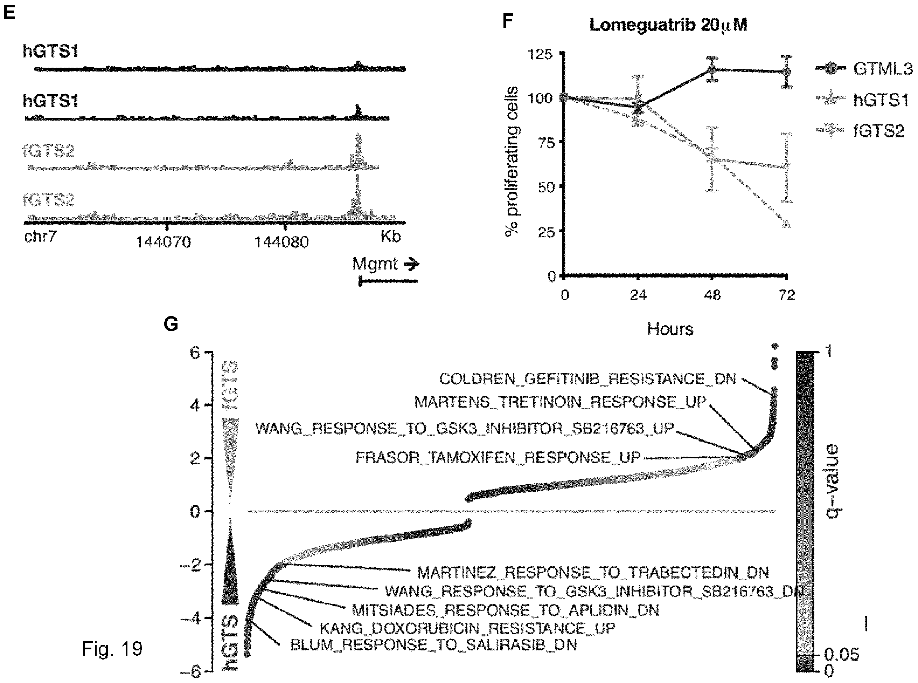

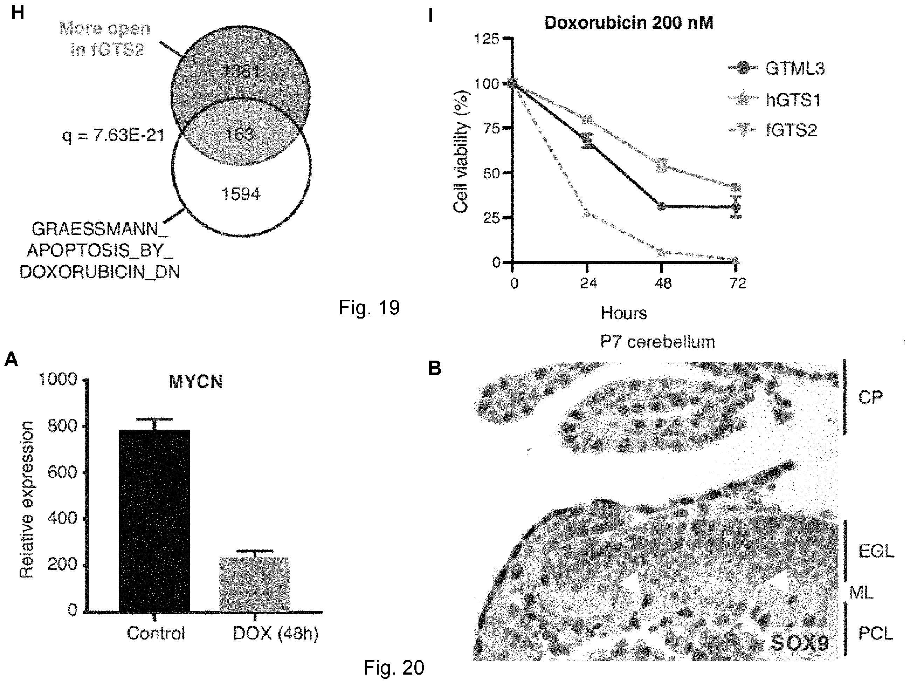

Fig. 19 MB recurrences respond to MGMT inhibition and to doxorubicin therapy. (A) Cell counts and representative pictures of pre-GT20 cells positively stained for SOX9 in sphere cultures after treatment with Cisplatin (10 µM) or DMSO for 72 h *in vitro.* (B) Expression levels of Mgmt in primary GTML tumors (n=8) and recurrent GTS tumors (n=7) from RNA-Seq. (C) High MGMT levels significantly correlate with a poor 10-year overall survival in Group 3y and Group 4α MYC and MYCN high MB subgroups from Cancer Cell 2017, 31: 737-754.e736 at median cut off. (D) Induction of SOX9 in MB002-SOX9CPDmt using doxycycline also leads to increased MGMT protein already after 2 and 8 h. (E) ATACseq of one GTS hindbrain cell line (GT20) and one GTS forebrain cell line (GT21) show open chromatin at the MGMT promoter in GT21 suggesting that MGMT expression is induced in distant recurrences. (F) Proliferation (Alamar blue) after 24, 48 and 72 h of Lomeguatrib (20 µM) treatment of two different GTML (GTML3 and GTML13) vs two different GTS (GT20=hindbrain and GT21=forebrain) cell lines. (G) Normalized enrichment scores from a GSEA comparing GTS-1 (hindbrain) and GTS-2 (forebrain) expression profiles against the CGP (chemical and genetic perturbation) database of gene sets; significantly enriched drug treatment gene sets are highlighted. (H-I) Genes with significantly higher ATAC-seq peaks in the GTS-2 (forebrain) cell line as compared to the hindbrain GTS-1 line overlapped significantly (p<0.05) with genes downregulated by doxorubicin treatment (H) and genes found downregulated in Gefitinib-resistant non-small cell lung carcinoma cell lines (I). Unpaired non-parametric Mann-Whitney test in (A) and Student's t-test in (E, F) * p<0,05, *** p<0,0005, *** p<0,0001.

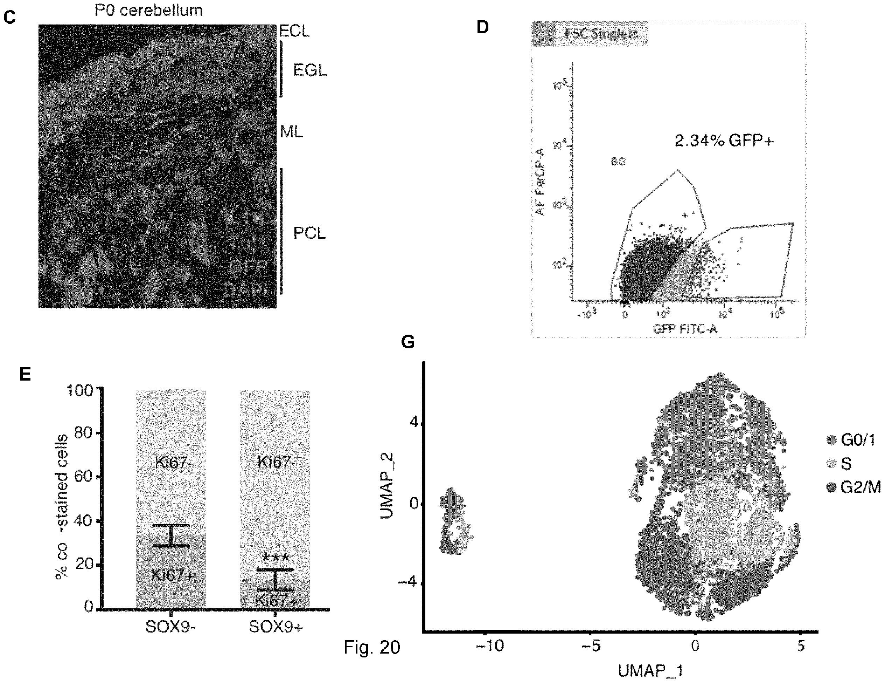

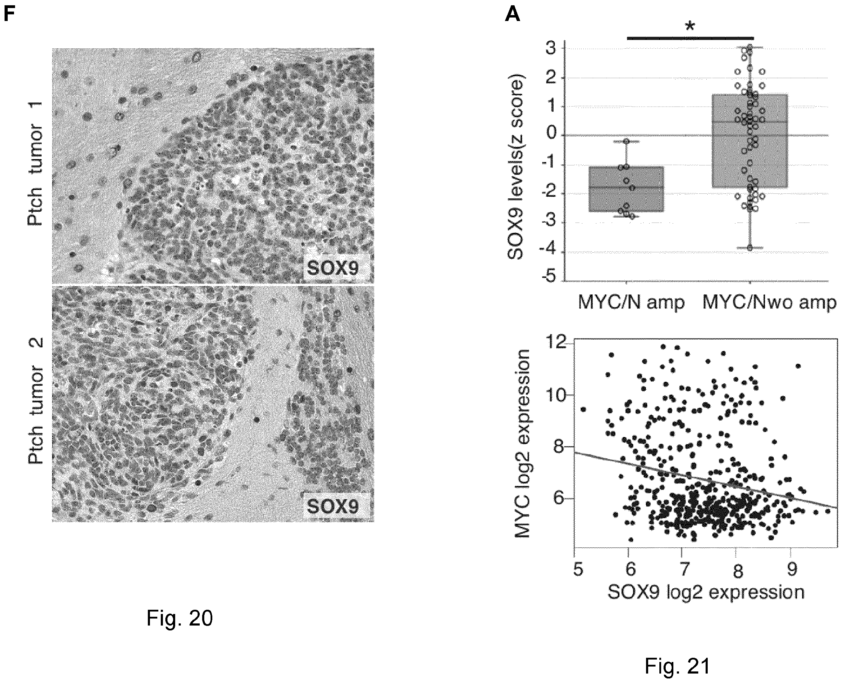

Fig. 20 SOX9 is upregulated by MYCN loss and is induced in therapy-resistant dormant cells. (A) RT-PCR of 48 h *in vitro* dox treated GTML cells. MYCN levels decrease in the surviving cell population of GTML cells. (B) IHC of SOX9 protein expression in the developing cerebellum (P7). (C) IF of SOX9 protein in the P0 brain of a transgenic mouse where the SOX9 promoter is driving GFP (pSOX9-GFP). (D) FACS quantification of rare GFP-expressing cells in GTML-pSOX9-GFP tumor cells. (E) Quantification of Ki67-positive and Ki67-negative cells in SOX9-positive and SOX9-negative GTML cell populations (***p=0.0008). (F) IHC of SOX9 expression in two different Ptch+/-/Math1-SB11/T20nc animals carrying SHH tumors in the cerebellum (Nature 2012, 482: 529-533). (G) Umap of cell cycle analysis shows distribution of G0/1, S and G2M phase in GTML cells.

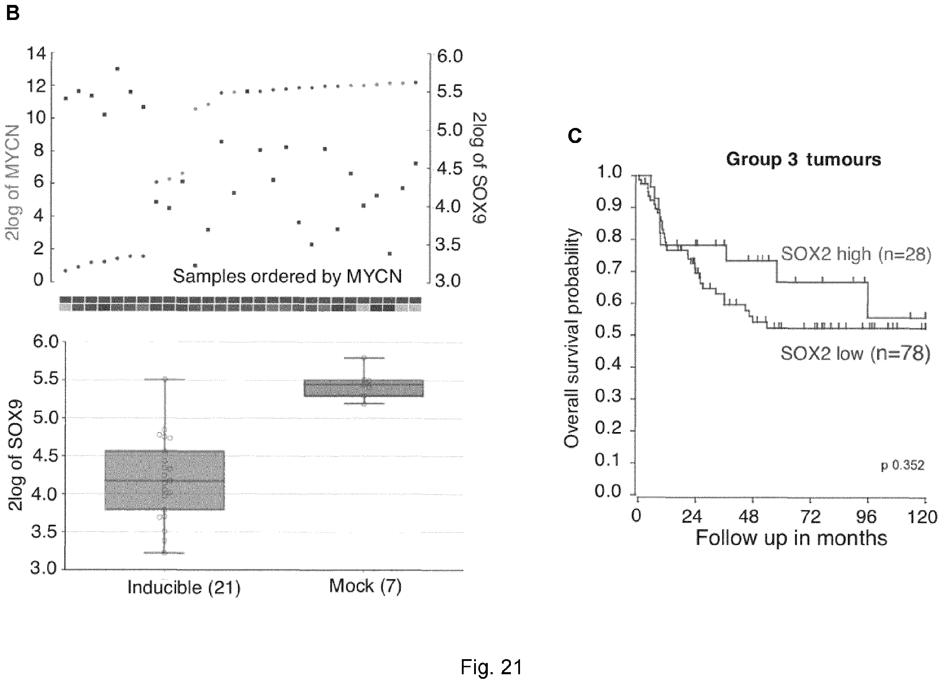

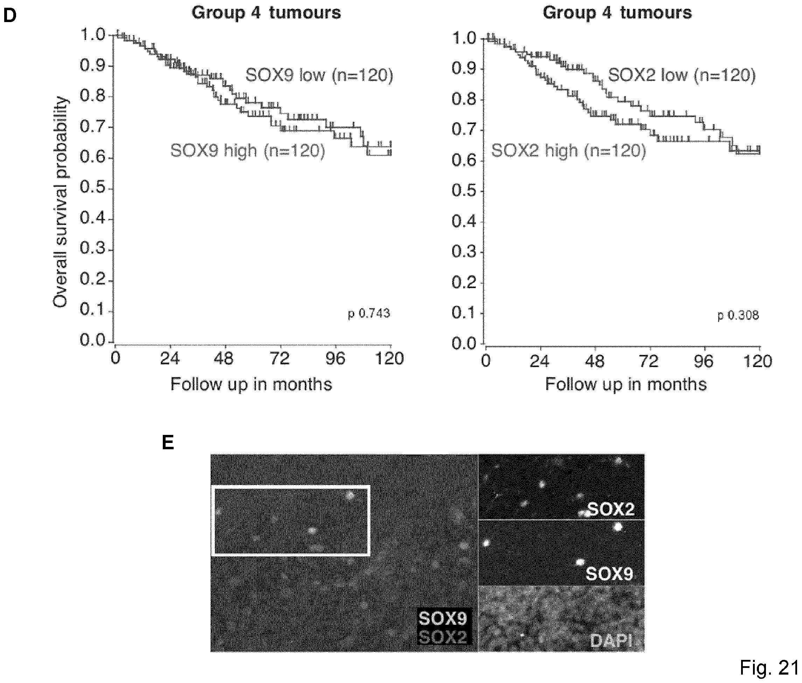

Fig. 21 SOX9 increases in residual disease models and marks quiescent cells in Group 3 MB patients. (A) (top) In a cohort of 64 MB patients, the levels of SOX9 expression and MYC/MYCN amplification were found to anti correlate. In a larger cohort (bottom) of Group 3/Group 4 patients (n=470) MYC also showed a significant negative correlation with SOX9 expression. (B) Dox-induced MYCN induction in MYCN2 neuroblastoma cells (Cell Oncol (Dordr) 2014, 37: 387-398) analyzed using the R2 Genomics Analysis and Visualization Platform. (top) Gene expression in the MYCN2 cell line correlation of SOX9 and MYCN levels in individual samples: 7 Mock-treated followed by 21 dox-treated samples at varying time points of MYCN induction (from left to right) and (bottom). (C) Kaplan Meier plots (10-year overall survival) of SOX2 levels correlating with a good overall survival in Group 3 patients from Cancer Cell 2017, 31: 737-754.e736 at last quartile cut off as similarly presented in Fig. 14F. (D). High SOX9 (left) and high SOX2 expression levels (right) do not correlate with a significant poor 10-year overall survival in Group 4 patients from Cancer Cell 2017, 31: 737-754.e736 at median cut off. (E) ICC of a GTML tumor section showing SOX9 and SOX2. Some overlap of SOX9 and SOX2 expression is found, SOX9 expression seemingly more restricted than SOX2.

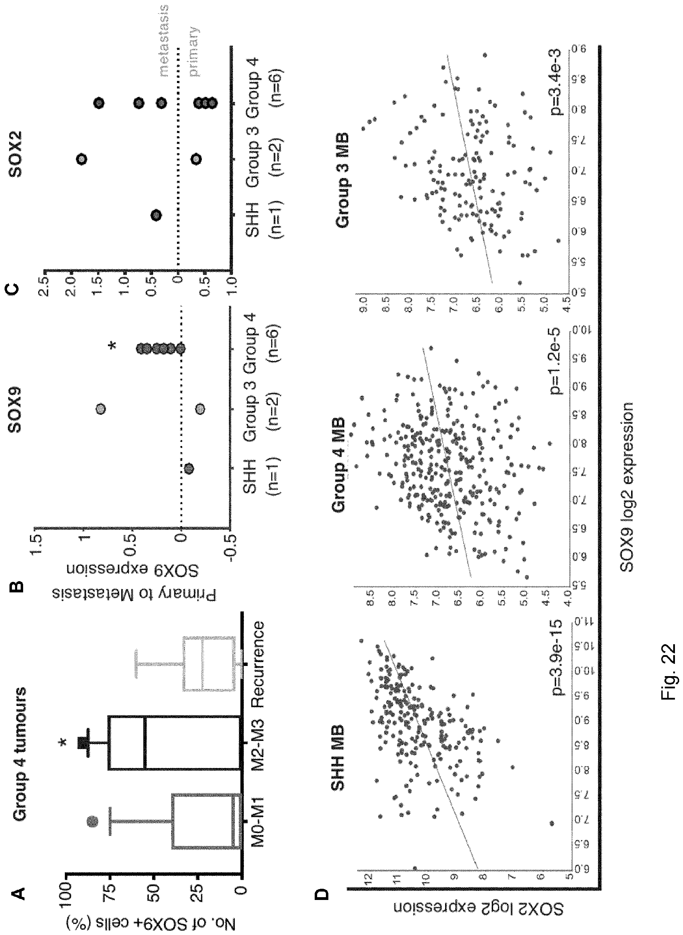

Fig. 22 SOX9 accumulates in metastatic disease and in relapses of paired primary-recurrent MB. (A) Percentage of SOX9 levels in M0-M1 and M2-M3 stage metastatic Group 4 MB as well as recurrent tumors of the same subgroup. High metastatic status of Group 4 MB has high SOX9 protein levels. (B) Paired primary/metastatic MB samples of SHH, Group 3 and 4 subgroups show elevated SOX9 levels in metastases from Group 3 and 4 MB. (C) Paired primary/metastatic MB samples show no difference in SOX2 levels. (D) Correlation of expression of SOX9 and SOX2 in SHH, Group 3 and Group 4 MB patients from Cancer Cell 2017, 31: 737-754.e736.

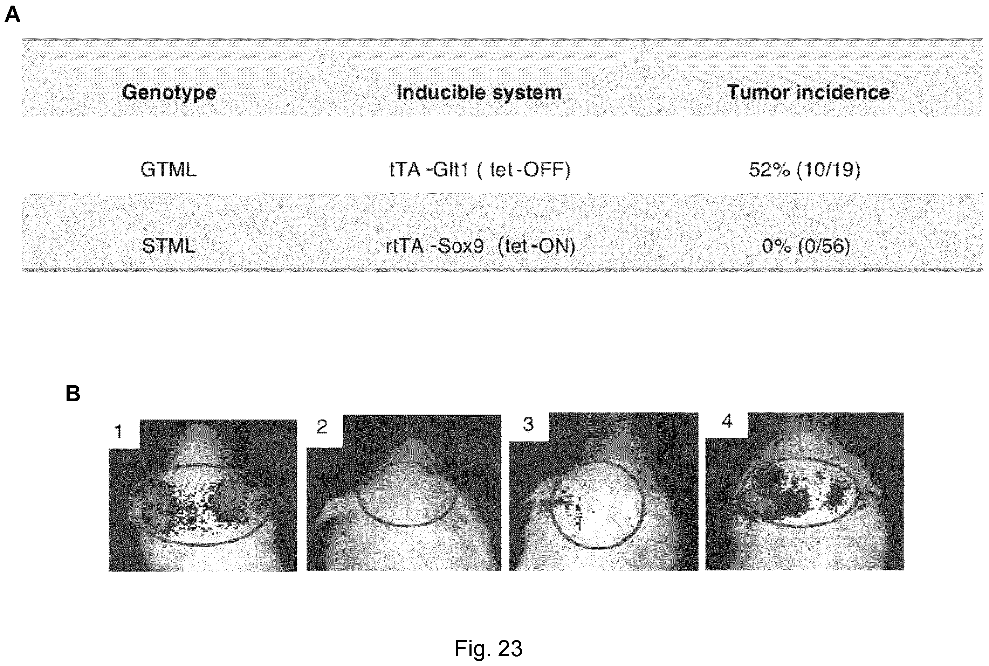

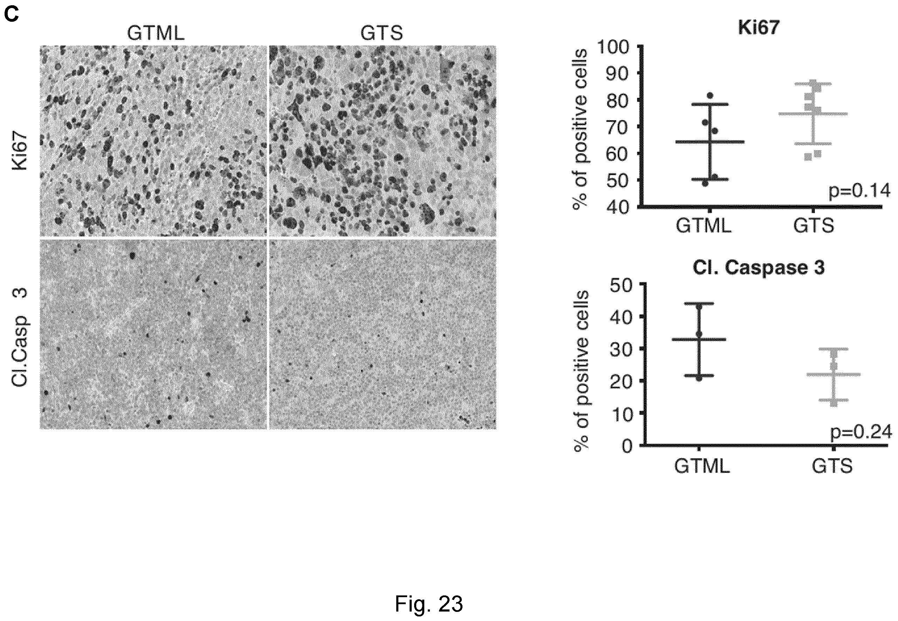

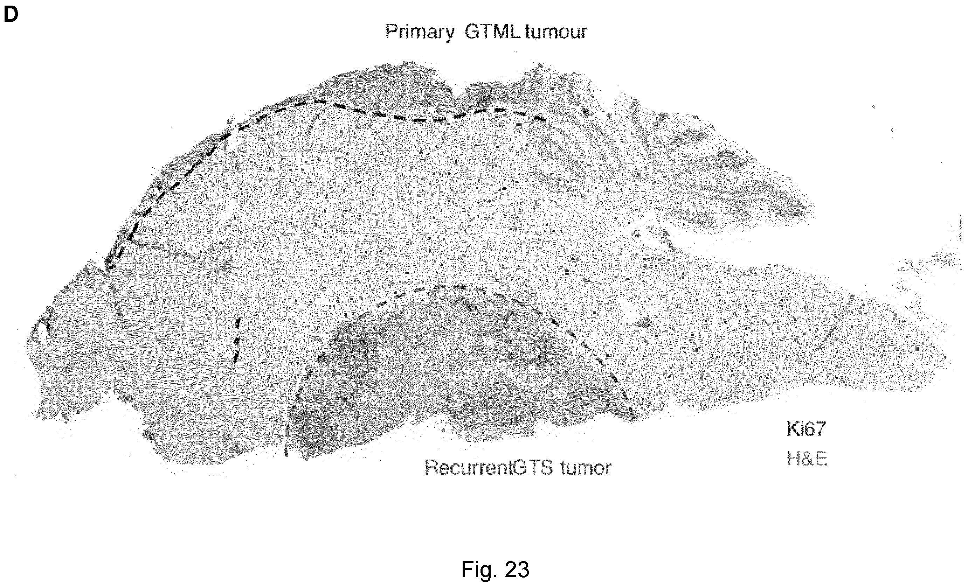

Fig. 23 The recurrence process is reproduced in a novel TetON/TetOFF transgenic GTS model (A) Tumor incidence in GTML and STML transgenic models. Roughly 50 % of GTML mice develop MB, whilst MYCN overexpression in the SOX9-positive cell population (STML) does not lead to primary tumor formation. (B) Luciferase imaging of a GTS mouse with primary tumor undergoing dox treatment, dox removal and sequential tumor relapse. 1) A large primary (GTML) tumor has developed. 2) 30 days of dox treatment lead to tumor recovery. 3) Approx. 30 days after dox removal the tumor starts to recur. 4) A large recurrent GTS tumor has developed 50 days after dox removal and the mouse start showing symptoms. (C) (left) Representative images from IHC of Ki67 and cleaved caspase 3 in GTML vs GTS mouse tumors. (right) Quantification of Ki67 and Cleaved caspase 3 in GTML vs GTS mouse tumors. (D) Representative IHC (Ki67) of whole mouse brain showing non-proliferating primary tumor cells after dox treatment together with a highly proliferative recurrent GTS tumor.

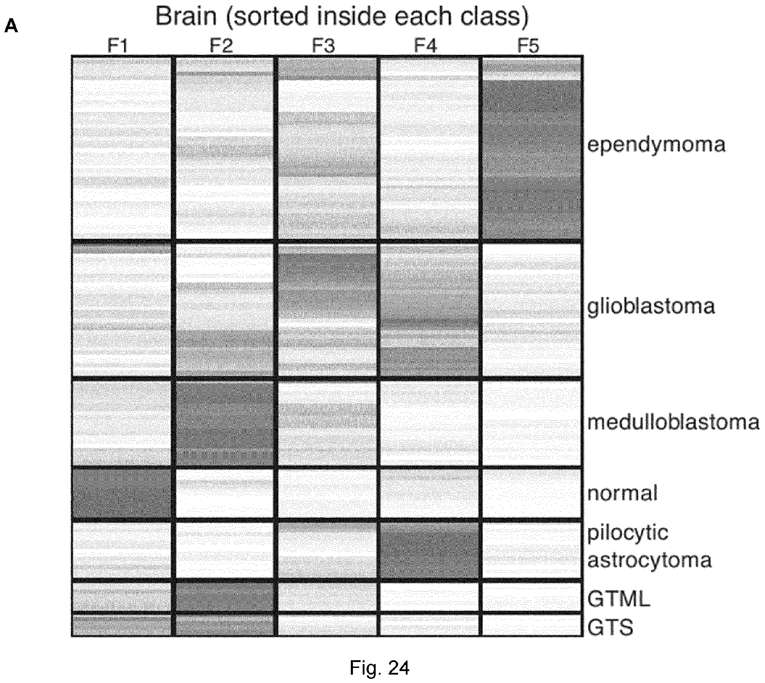

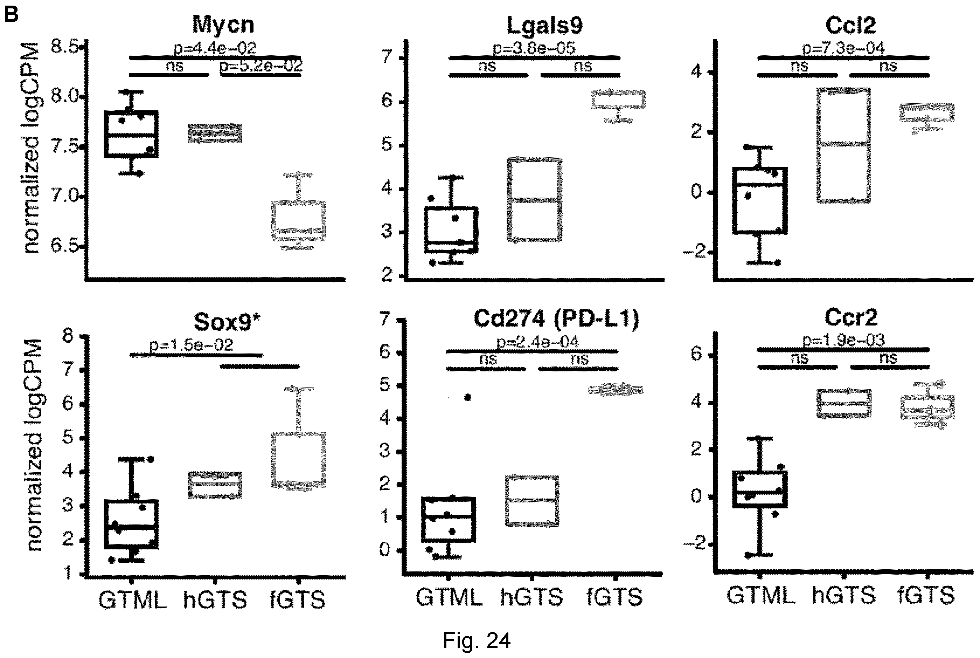

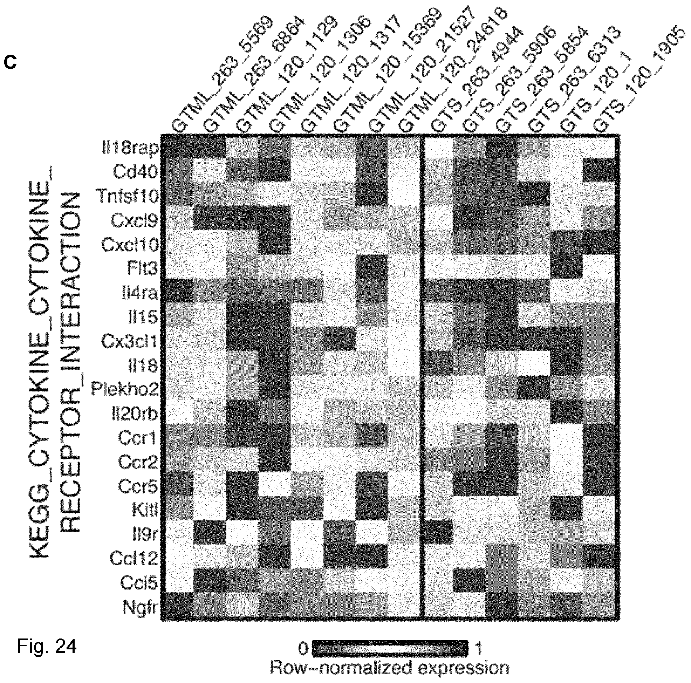

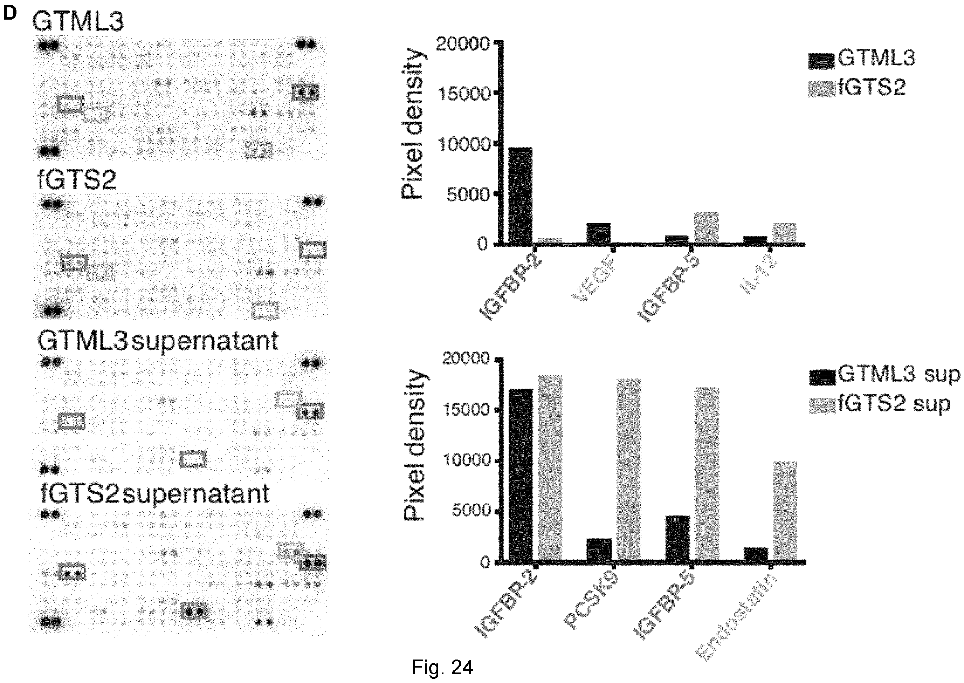

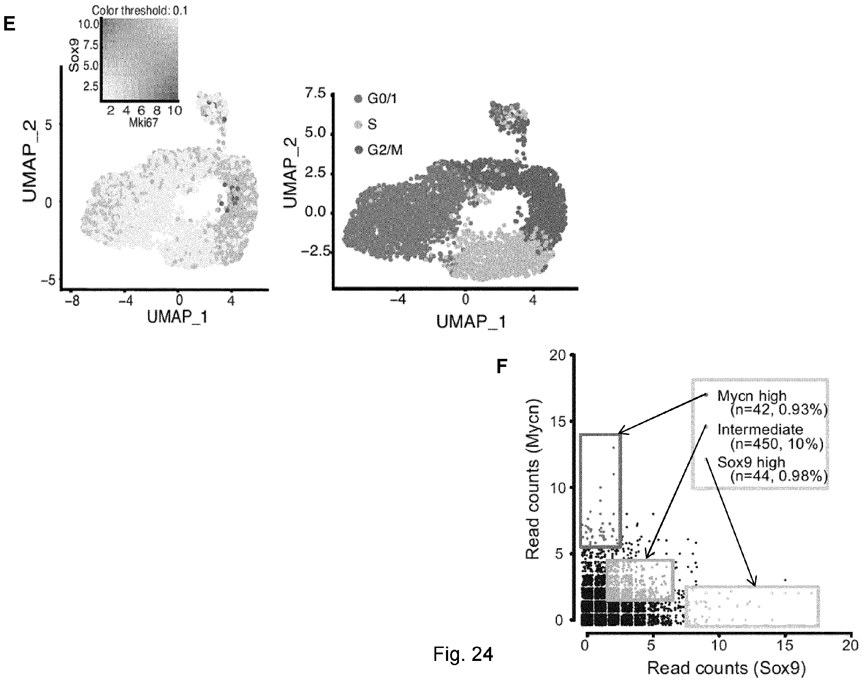

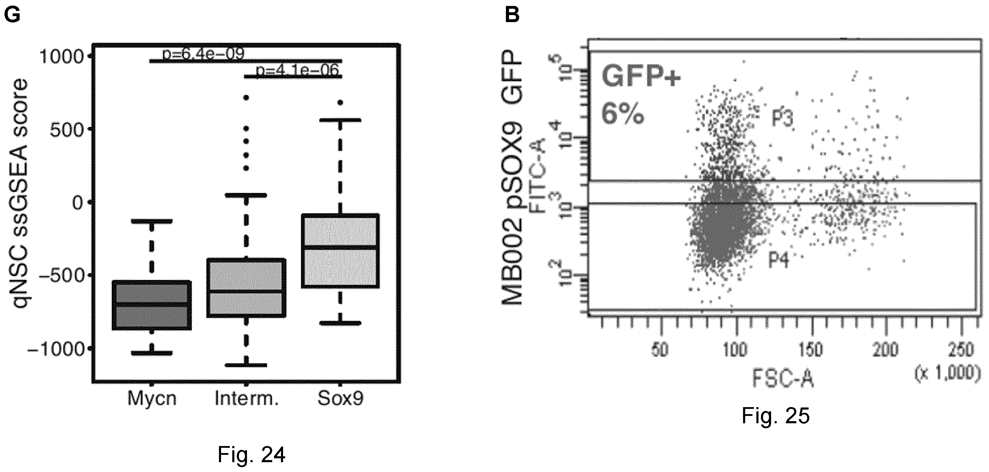

Fig. 24 Recurrent MBs molecularly resemble primary MBs but are more inflammatory. (A) Heatmap produced by the metagene script, which shows cross-platform projection of murine GTS and GTML expression profiles onto a dataset comprising various human brain tumor types and normal brain samples. Both GTML and GTS are modeling medulloblastoma. (B) Box-plots showing differential expression of the Cd247, Ccl2, Ezh2, Lgals9, Dnmt3b, and Kmt2d (MII2) genes between GTML and GTS tumors. (C) Heatmap showing expression of 20 genes from the KEGG_CYTOKINE_CYTOKINE_RECEPTOR_INTERACTION gene set, which were upregulated (FDR corrected p-value < 0.2) in the GTS tumors as compared to the GTML. (D) Comparative cytokine profiles for GTML and GTS cells and supernatant. (E) Umap plot of SOX9 and KI67 in GTS cells shows SOX9-positive cell and KI67-positive cell are exclusive each other. Umap of cell cycle analysis shows distribution of G0/1, S and G2M phase in GTS cells. (F) Single cell sequencing of GTS cells indicates that high MYCN and SOX9 expression is largely mutually exclusive. The top ~1 % of cells with highest MYCN and SOX9 expression, respectively, and ~10 % of cells with intermediary expression were selected for downstream analyses. (G) Boxplot comparing the ssGSEA scores for a quiescent NSC signature between the groups from (F), indicating that SOX9 high GTS cells appear significantly (p<0.05, Welch's t-test) more quiescent than MYCN-high and intermediate cells.

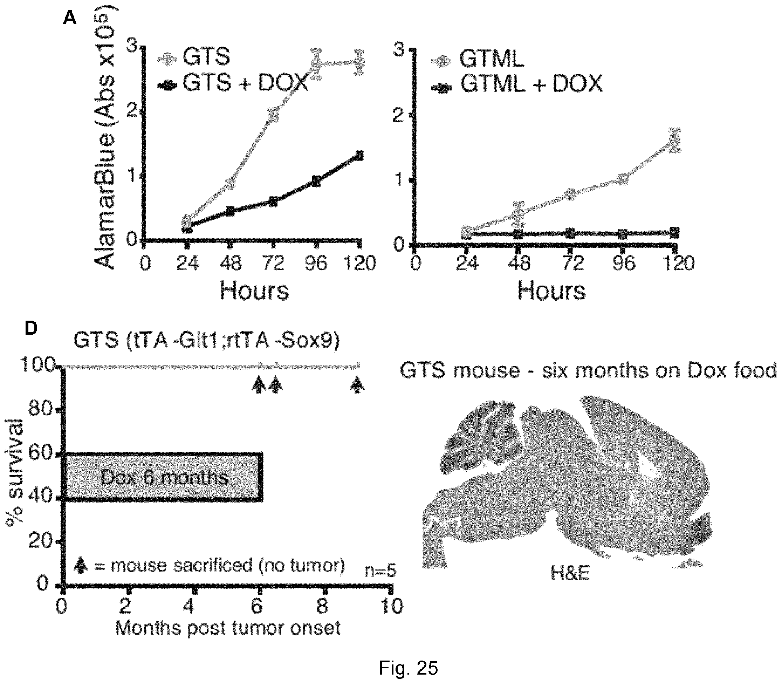

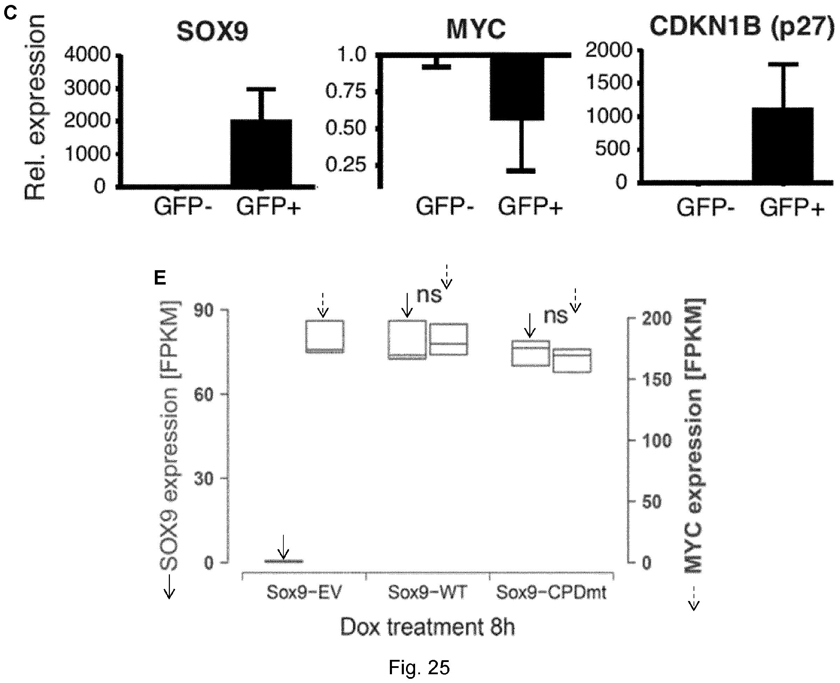

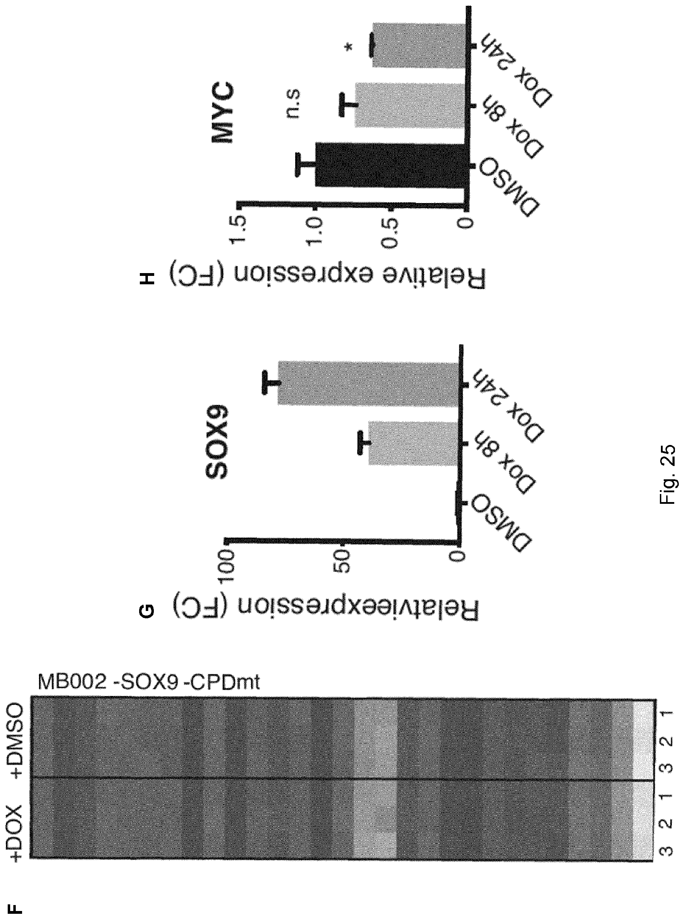

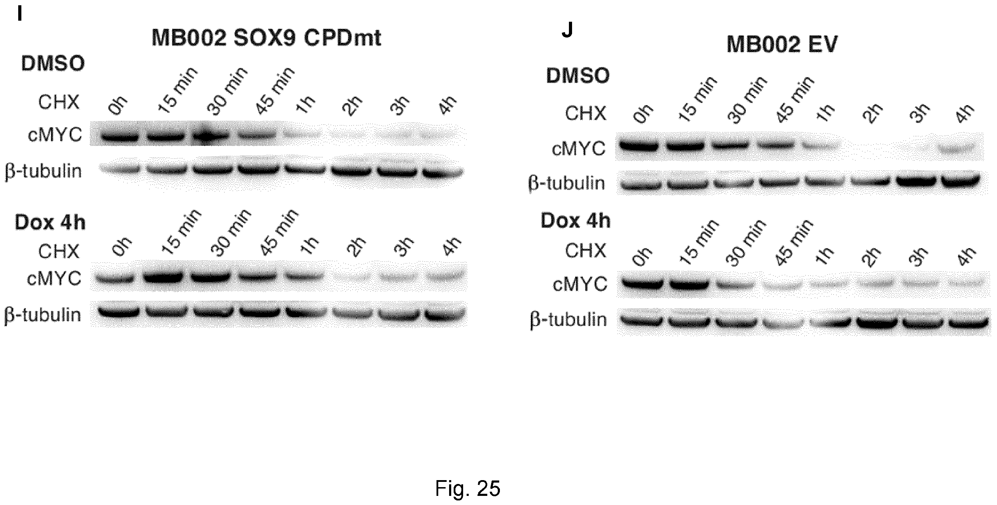

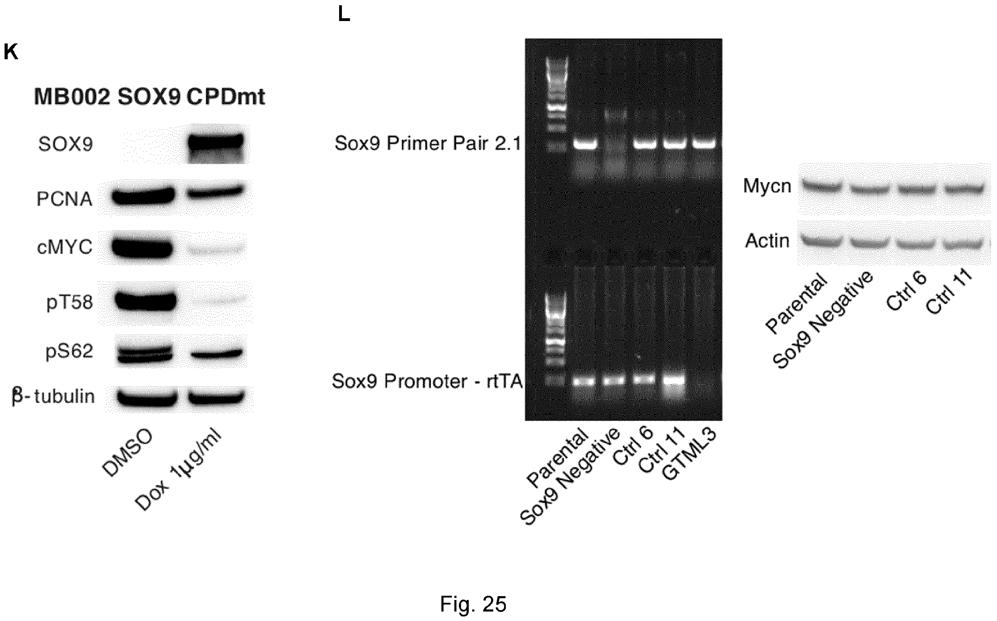

Fig. 25 SOX9 inhibits MYC in therapy-resistant cells and is essential for driving recurrence. (A) Recurrent tumor cells (GTS) can proliferate in dox-containing media while primary tumor cells (GTML) do not survive the treatment. (B) FACS quantification of GFP-positive cells in MB002 cells transfected with lentiviral pSOX9-GFP. Only a small percentage of cells (6 %) express SOX9. (C) Relative expression from qPCR analysis of SOX9, MYC and the CDK2/Cyclin E and CDK4/Cyclin D inhibitor p27 (CDKN1B) in the GFP-positive cell population as well as the GFP-negative cell population from. SOX9 expression is high in GFP-positive cells while MYC expression goes down. Cell cycle inhibitor p27 is high indicating low proliferation rate in the SOX9-positive GFP-positive cell population. (D) GTS mice treated for 6 months with dox do not develop relapses. A representative H&E staining of a whole brain of a GTS mouse treated with dox for 6 months, showing no tumor or any tumor remnants. (E) Gene expression levels of MYC and SOX9 in MB002-SOX9EV, MB002-SOX9WT, and MB002-SOX9CPDmut. (F) Methylation analysis of CpG sites in the promoter and gene body of MYC in MB002-SOX9CPDmut shows no differential methylation between cells treated for 8 h with DMSO or dox. (G) Upon 8 h treatment with dox, MB002 cells transduced with lentiviral vectors encoding inducible SOX9WT or SOX9CPDmut showed increased expression of SOX9 as compared to MB002 cells transduced with EV. (H) Whereas neither MB002-SOX9WT nor SOX9CPDmut showed an immediate decrease of MYC gene expression levels as compared to MB002-EV. (I-J) Induction of stabilized SOX9 depletes MYC expression in MB002CPDmut cells. MYC protein stability in (I) MB002 EV and (J) MB002-SOX9CPDmt after 4 h dox-treatment. (K) Induction of stabilized SOX9 leads to lower protein levels of PCNA (proliferation marker), total MYC and less phosphorylation of the MYC activating residue 252 as well as residue T58. (L) Sox9 CRISPR-Cas9 editing was confirmed using PCR on DNA isolated from parental pre-GT20, SOX9 edited (sgSox9) and control (sgCtrl1/2) cell lines. Western blot shows unchanged MYCN protein levels in sgSox9 cells as compared to controls.

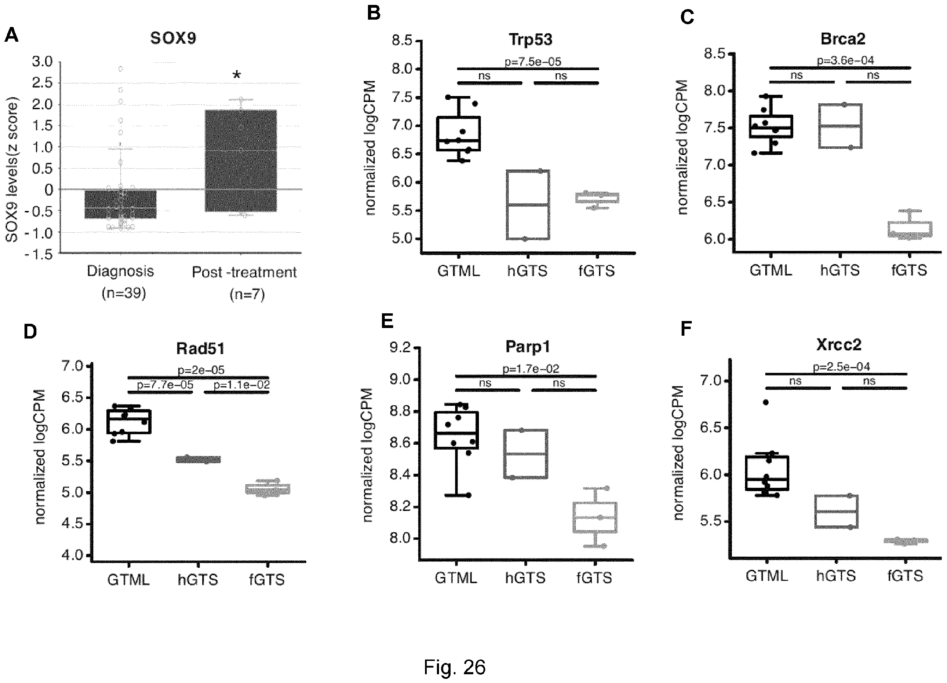

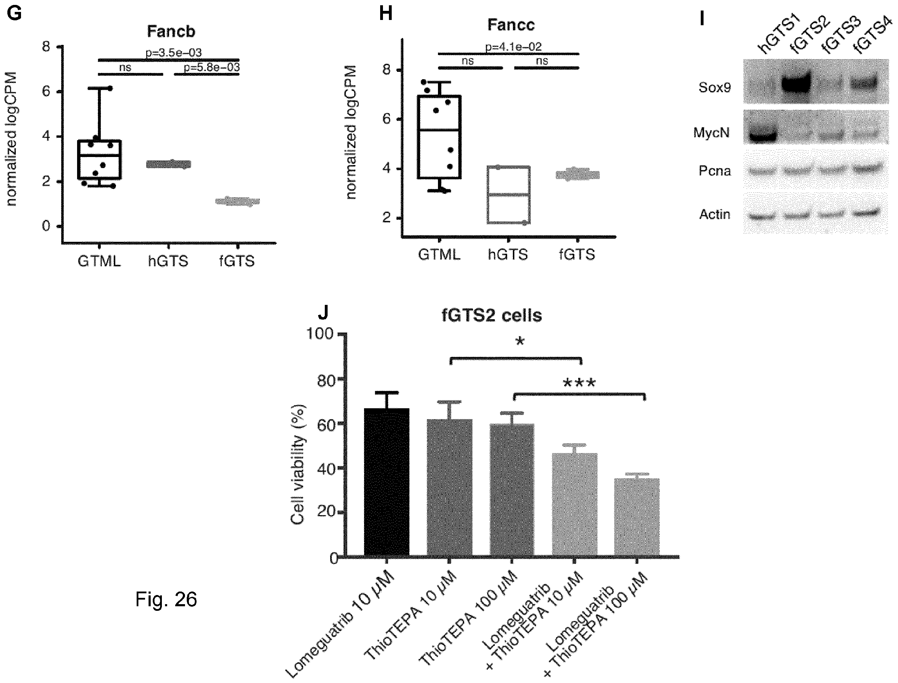

Fig. 26 MB recurrences respond to MGMT inhibition and to doxorubicin therapy. (A) SOX9 levels significantly increase in patient tumors post-treatment, as compared to primary diagnosis. (B-H) Typical DNA repair genes (Trp53, Brca2, Rad51, Parp1, Msh6, Xrcc2, Fancb and Fancc) are significantly downregulated in GTS tumors. (I) WB depicting SOX9, MYCN, PCNA and Actin in four different GTS recurrence cell lines (hGTS1 and fGTS22). (J) Proliferation of GTS cells after 72 h of Lomeguatrib (10 µM) or ThioTEPA (10, 100 µM) treatment alone or in combination. Adding 10 µM Lomeguatrib to ThioTEPA treatment significantly decreases proliferation of GTS recurrent tumor cells.

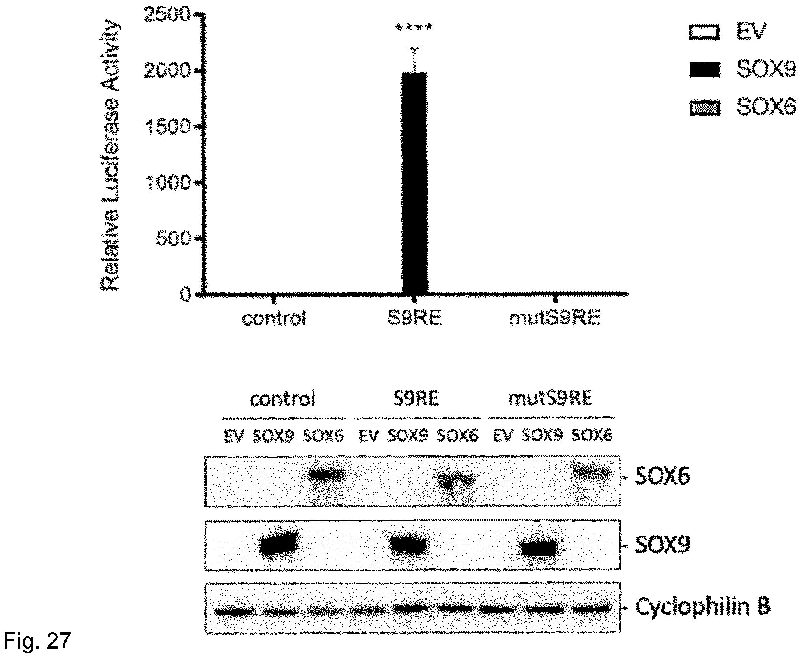

Fig. 27 The S9RE reporter is strongly activated by SOX9 in HEK-293T cells. No significant activation was observed with SOX6. Transfection efficiency was normalized on the basis of the activity of the co-transfected Renilla luciferase. All values are presented as the fold induction over the control luciferase construct (minimal promoter alone). Representative data from three independent experiments are shown. ****p < 0.0001. One way ANOVA followed by Tukey's multiple comparisons test. Western blot analysis to confirm expression of transfected plasmids (bottom). EV = Empty vector control.

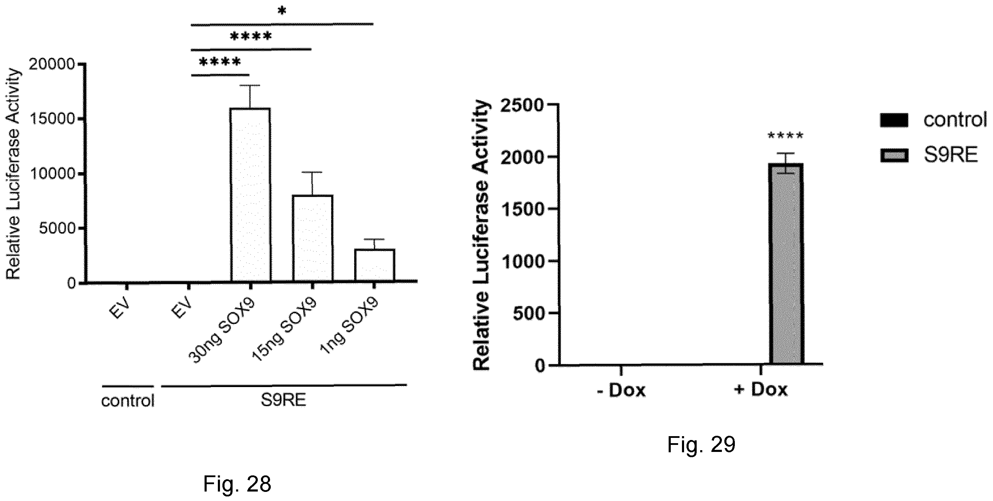

Fig. 28 Effects of decreasing concentration of SOX9 plasmid on the activation of the S9RE luciferase reporter in HEK-293T cells. Transfection efficiency was normalized on the basis of the activity of the co-transfected Renilla luciferase. The activity for each luciferase construct was further normalized for activity observed with the minimal promoter alone (control). Representative data from two independent experiments are shown. ****p < 0.0001, *p < 0.05. One way ANOVA followed by Tukey's multiple comparisons test. EV = empty vector control.
Fig. 29 Luciferase assay of MB002 cells in the absence or presence of SOX9. Cells were treated with doxycycline (dox) to induce expression of SOX9-CPDmt (T236/240A) 24 hours before luciferase assay. Transfection efficiency was normalized on the basis of the activity of the co-transfected Renilla luciferase. ****p < 0.0001. One way ANOVA followed by Tukey's multiple comparisons test.

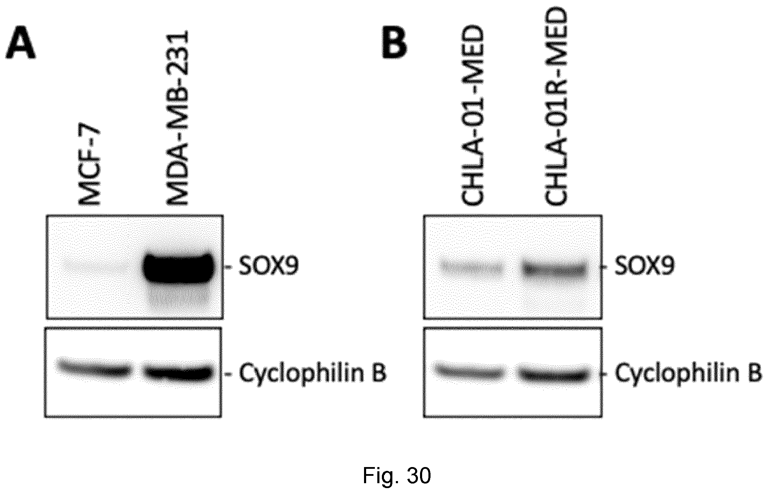

Fig. 30 SOX9 expression in matched cancer cell lines. (A) SOX9 protein expression in breast cancer cell lines MCF-7 (hormone dependent) and MDA-MB-231 (hormone independent). (B) Western blot analysis showing SOX9 protein expression and loading control Cyclophilin B in matched primary CHLA-01-MED and recurrent CHLA-01R-MED cells generated from different biopsies from the same patient operated twice.

### DETAILED DESCRIPTION

The present invention generally relates to cancer diseases, and in particular to methods of estimating the likelihood of cancer relapse and predicting a response to cancer treatment, and viral vectors and medicaments useful in treatment of cancers.

Recurrence and metastases are the major barriers to therapeutic success, and relapse is responsible for a significant portion of the cancer associated deaths. Given the near-universal treatment failure encountered in the setting of relapsed cancer disease it is important to have new diagnostic and prognostic tools to identify patients that are at higher risk of developing relapses.

Hence, there is a general need for identifying subjects having a comparatively high likelihood of cancer relapse to thereby, at as early stage as possible, take countermeasures and start or continue cancer treatment.

Cancer relapses may be caused by clonal selection and expansion of a therapy-resistant clone. The inventors have observed rare populations of cancer cells forming quiescent subclones in recurrent tumors. This rare population of quiescent and dormant cancer cells included SOX9 positive (SOX9+) cancer cells. The SOX9+ cancer cells have enhanced migratory capacity, become reprogrammed to escape the immune system, are more resistant to standard anti-cancer therapy and are able to form distant metastases.

The transcription factor SOX-9 (SOX9) is a protein that is encoded by the SOX9 gene. SOX9 recognizes the sequence CCTTGAG or similar along with other members of the high mobility group (HMG) box class DNA-binding proteins. It is expressed by proliferating but not hypertrophic chondrocytes that is essential for differentiation of precursor cells into chondrocytes and, with steroidogenic factor 1 (SF-1), regulates transcription of the anti-Müllerian hormone (AMH) gene. SOX9 also plays a pivotal role in male sexual development; by working with SF-1, SOX9 can produce AMH in Sertoli cells to inhibit the creation of a female reproductive system. It also interacts with other genes to promote the development of male sexual organs.

SOX9 has been implicated in both initiation and progression of multiple solid tumors. SOX9 appears to induce invasiveness and therapy-resistance in prostate, renal, thyroid, gastrointestinal, lung, skin, pancreatic, liver, colorectal, breast, bladder, gastric, ovarian and other cancers *(*Seminars in Cancer Biology 2020, 67(Part 1): 122-153; Journal of Oncology 2019, 6754040; Cell 2016, 165(1): 45-60; Cell 2012, 148(5): 1015-1028; Nat Med 2020, 26(2): 259-269; Clin Cancer Res 2020, 26(7): 1678-1689; Mol Cancer Ther 2019, 18(8): 1386-1395; Cell Stem Cell 2015, 17(1):60-73; Cancer Cell 2012, 22(6): 737-750*;* Oncogene 2021, 40(4): 848-862; Cell Rep 2019, 27(3): 971-986.e9; Mol Cell 2015, 60(2): 307-318; Hum Pathol 2016, 52: 38-46; Br J Cancer 2018, 119(11): 1358-1366; Nucleic Acids Res 2013, 41(8): 4459-4469; Oncogene 2009, 28(35): 3121-3131; Int J Oncol 2018, 53(1): 189-202), and therefore promotes lethal metastasis.

An aspect of the invention relates to a method of estimating a likelihood of cancer relapse for a subject following cancer treatment, see Figs. 1 and 10. The method comprises introducing, in step S1, ex *vivo* a nucleic acid molecule comprising a reporter gene under transcriptional control of a SOX9 promoter (SOX9p) or of a promoter operatively linked to an enhancer element comprising at least one SOX9 binding site into cancer cells obtained from a tumor sample from a subject. The method also comprises exposing, in step S2, ex *vivo* the cancer cells to a cancer treatment. The method further comprises determining, in step S3, the amount of SOX9+ cancer cells following cancer treatment based on expression of the reporter gene. A likelihood of cancer relapse following cancer treatment is then estimated in step S4 for the subject based on the determined amount of SOX9+ cancer cells.

This aspect of the invention thereby determines a likelihood that a subject having undergone cancer treatment will suffer from cancer relapse. The estimation is based on identifying any population of SOX9+ cancer cells among cancer cells as taken from a tumor sample from the subject. Such SOX9+ cancer cells constitute a population of quiescent and dormant cancer cells but with enhanced migratory capacity, can escape the immune system, are more resistant to standard anti-cancer therapy and are able to form distant metastases. The identification of such SOX9+ cancer cells is, in an embodiment, performed using a nucleic acid molecule comprising a reporter gene under transcriptional control of SOX9p. Hence, cancer cells, in which SOX9p is active, will transcribe the reporter gene and can thereby be identified and differentiated from SOX9 negative (SOX9-) cancer cells by the expression of the reporter gene. Alternatively, the identification of SOX9+ cancer cells is performed using a nucleic acid molecule comprising a reporter gene under transcriptional control of a promoter operatively linked to an enhancer element comprising at least one SOX9 binding site. Hence, SOX9+cancer cells express SOX9 that binds to the at least one SOX9 binding site in the enhancer element, which enhances transcription of the reporter gene. These SOX9+ cancer cells will thereby transcribe the reporter gene and can be identified and differentiated from SOX9- cancer cells by the expression of the reporter gene.

In a general embodiment, the nucleic acid molecule comprises a reporter gene under transcriptional control of a SOX9 response element (SOX9re). In an embodiment, this SOX9re comprises the SOX9p. In another embodiment, the SOX9re comprises the SOX9p operatively linked to the enhancer element comprising the at least one SOX9 binding site. In a further embodiment, the SOX9re comprises a promoter other than the SOX9p operatively linked to enhancer element comprising the at least one SOX9 binding site.

Hence, in an embodiment, the method of estimating a likelihood of cancer relapse for a subject following cancer treatment comprises introducing, in step S1, *ex vivo* a nucleic acid molecule comprising a reporter gene under transcriptional control of a SOX9 promoter (SOX9p) into cancer cells obtained from a tumor sample from a subject. The method also comprises exposing, in step S2, *ex vivo* the cancer cells to a cancer treatment. The method further comprises determining, in step S3, the amount of SOX9 positive (SOX9+) cancer cells following cancer treatment based on expression of the reporter gene and estimating, in step S4, a likelihood of cancer relapse following cancer treatment for the subject based on the determined amount of SOX9+ cancer cells.

Generally, a high amount of SOX9+ cancer cells is associated with a high likelihood of cancer relapse, whereas a comparatively low amount of SOX9+ cancer cells is associated with a comparatively lower likelihood of cancer relapse. A high amount of SOX9+ cancer cells represent a population of cancer cells having escaped the cancer treatment as applied *ex vivo* in step S2. Hence, this population of cancer cells is then more resistant to the applied cancer treatment as compared to other cancer populations in the tumor sample. Furthermore, the population is SOX9+cancer cells have enhanced migratory capacity and are able to form distant metastases. This means that a high amount of SOX9+ cancer cells following cancer treatment indicates that the tumor in the subject comprises SOX9+ cancer cells that are resistant to standard anti-cancer therapy, have enhanced migratory capacity and are able to form distant metastases in the subject. Hence, the subject is likely to suffer from cancer relapse if treated with a standard cancer treatment.

A reporter gene as used herein refers to a gene that, when expressed in a cancer cell, confers a characteristic to the cancer cells that is easily identified and optionally measurable, or is a selectable marker. Illustrative examples of reporter genes are genes that induce visually identifiable characteristics, such as encoding for fluorescent or luminescent proteins. Examples of such reporter genes are *gfp* encoding green fluorescent protein (GFP), *rfp* encoding red fluorescent protein (RFP), *yfp* encoding yellow fluorescent protein, *cfp* encoding cyan fluorescent protein (CFP), and *luc* encoding luciferase enzyme (LUC).

The full length human SOX9p is present within the nucleotide sequence of from -2500 to +1500, wherein +1 indicates the start site of transcription of the SOX9 gene, see Fig. 12 and SEQ ID NO: 1. The full length human promoter comprises a Sry site, two δEF1 sites, a NFAT site, a NFκB site, a REL cites, two SP1 sites, a one-half CREB site, two CCAAT sites in addition to the TATA box.

The SOX9p as used according to the embodiments could be the full length SOX9p (SEQ ID NO: 1), or a transcriptionally active portion thereof. Transcriptional active portion of the SOX9p refers to a portion of the SOX9p that is transcriptionally active, i.e., can initiate transcription of a gene under transcriptional control of the portion of the SOX9p. Examples of such transcriptional active portions of the SOX9p include from -1350 to +250 (SEQ ID NO: 2) or from -1034 to +67 (SEQ ID NO: 4). Experimental data indicates that the core SOX9 promoter, i.e., the minimal portion of the SOX9 promoter required to initiate transcription, corresponds to nucleotide positions -256 to +67 (SEQ ID NO: 3), or a portion thereof (Matrix Biology 2005, 24: 185-197).

The SOX9p is preferably from the same species as the source of the cancer cells, i.e., the same species as the subject. In a particular embodiment, the subject is a human subject. In such a particular embodiment, the SOX9p is the human SOX9p (hSOX9p). The present invention is, however, not limited to human subjects and hSOX9p but could also be used for other animal species, in particular mammal species. Non-limiting, but illustrative, examples of such species include dog, cat, sheep, goat, cow, horse, rabbit, mouse, rat, non-human primates, such as monkeys. In such a case, the SOX9p could be a canine SOX9p, a feline SOX9p, a hircine SOX9p, a bovine SOX9p, an equine SOX9p, a leporine SOX9p, or a murine SOX9p as illustrative examples.

The enhancer element of the embodiments comprises at least one SOX9 binding site. As mentioned above, SOX9 recognizes the sequence CCTTGAG. In an embodiment, the SOX9 binding site is CCTTGAG. In another embodiment, the SOX9 binding site has the following nucleotide sequence CATTCAT.

In an embodiment, the enhancer element comprises at least one copy of a 49-bp chondrocyte specific intronic enhancer segment from type II collagen alpha 1 (*Col2a1*) gene comprising the SOX9 binding site. This 49-bp enhancer segment is shown below and in SEQ ID NO: 12:
CTGTGAATCGGGCTCTGTGTGCGCTCGAGAAAAGCCCCATTCATGAGAG

In an embodiment, the enhancer element comprises one copy of the SOX9 binding site, such as CATTCAT, for instance one copy of SEQ ID NO: 12. However, it is generally preferred if the enhancer element comprises multiple, i.e., at least two, copies of the SOX9 binding site, such as multiple copies of CATTCAT, for instance multiple copies of SEQ ID NO: 12. In a currently, preferred embodiment, the enhancer element comprises at least three, and more preferably at least four copies of the SOX9 binding site, such as at least three or at least four copies of CATTCAT, for instance at least three or at least four copies of SEQ ID NO: 12. An advantage of using multiple copies of the SOX9 binding site is to amplify the overall output of the binding of SOX9 to the enhancer element.

A currently preferred SOX9 response element (S9RE) that could be used as enhancer element according to the embodiments comprises four copies of SEQ ID NO: 12 and is presented below and in SEQ ID NO: 13:

CTGTGAATCGGGCTCTGTGTGCGCTCGAGAAAAGCCCCATTCATGAGAGCTGTGAATCGGGCTCT
GTGTGCGCTCGAGAAAAGCCCCATTCATGAGAGCTGTGAATCGGGCTCTGTGTGCGCTCGAGAAA
AGCCCCATTCATGAGAGCTGTGAATCGGGCTCTGTGTGCGCTCGAGAAAAGCCCCATTCATGAGA
G

An alternative S9RE comprises two additional nucleotides CT at the 3'-terminal and is presented here below and in SEQ ID NO: 14:

CTGTGAATCGGGCTCTGTGTGCGCTCGAGAAAAGCCCCATTCATGAGAGCTGTGAATCGGGCTCT
GTGTGCGCTCGAGAAAAGCCCCATTCATGAGAGCTGTGAATCGGGCTCTGTGTGCGCTCGAGAAA
AGCCCCATTCATGAGAGCTGTGAATCGGGCTCTGTGTGCGCTCGAGAAAAGCCCCATTCATGAGA
GCT

The enhancer element comprising the at least one SOX9 binding site, such as shown in SEQ ID NO: 12 to 14, is operatively linked to a promoter.

"Enhancer element" as used herein refers to a regulatory sequence that increases expression of an operatively linked gene or coding sequence but does not have promoter activity. An enhancer element can generally be provided upstream, downstream and to the other side of a promoter without significant loss of activity. Furthermore, an enhancer element may be positioned within the coding sequence of the gene.

The term "operatively linked" refers to a functional linkage between the regulatory sequence (enhancer element or promoter) and a coding sequence (gene) or a functional linkage between two regulatory sequences (enhancer element and promoter). The components so described are, thus, in a relationship permitting them to function in their intended manner. By placing a coding sequence under regulatory control of a promoter means positioning the coding sequence such that the expression of the coding sequence is controlled by the promoter. Correspondingly, by placing a promoter under regulatory control of an enhancer element means positioning the promoter such that the activity of the promoter in expressing a coding sequence is enhanced by the presence of the enhancer element.

The enhancer element comprising at least one SOX9 binding site could be operatively linked to any promoter that is transcriptionally active in the cancer cells and where the activity of the promoter is enhanced by the enhancer element. In an embodiment, the promoter is SOX9p. In another embodiment, the promoter is a promoter that is substantially inactive in the cancer cells or is transcriptionally active at a low level in the cancer cells when not operatively linked to the enhancer element. In such an embodiment, operatively linking the enhancer element to the promoter significantly enhances the activity of the promoter and expression of the reporter gene in the presence of SOX9, i.e., in SOX9+cancer cells, but not in the absence of SOX9, i.e., in the SOX9- cancer cells. Hence, the construct of the enhancer element operatively linked to the promoter preferably leads to a significant increase in expression of the reporter gene in the presence of SOX9 as compared to in the absence of SOX9. This means that the enhancer element makes the operatively linked promoter respond to SOX9 and thereby becomes a SOX9-inducible promoter. Illustrative, but non-limiting, examples of promoters that could be operatively linked to the enhancer element include human elongation factor 1α (hEF1α) promoter, cytomegalovirus (CMV) promoter, simian virus 40 early (SV40) promoter, human Ubiquitin C (UBC) promoter, phosphoglycerate kinase 1 (PGK) promoter, tetracycline-responsive element (TRE) promoter and CMV early enhancer/chicken β-Actin (CAG) promoter.

The nucleic acid molecule is preferably a deoxyribonucleic acid (DNA) molecule but could be a ribonucleic acid (RNA) molecule. In the latter case, the RNA molecule, or at least the portion thereof, comprising SOX9p or the promoter operatively linked to the enhancer element comprising at least one SOX9 binding site and the reporter gene, is reverse transcribed in the cancer cells into a complementary DNA (cDNA) molecule comprising the SOX9p and the reporter gene.

The reporter gene is under transcriptional control of SOX9p or of the promoter operatively linked to the enhancer element comprising at least one SOX9 binding site. This means that the reporter gene will be expressed in cancer cells and under conditions, under which SOX9p is active and the SOX9 gene would be expressed. Hence, cancer cells comprising the nucleic acid molecule comprising the reporter gene under transcription control of SOX9p or of the promoter operatively linked to the enhancer element comprising at least one SOX9 binding site and expressing the reporter gene can be detected as SOX9+ cancer cells due the characteristic conferred by the reporter gene on the cancer cells. This means that the determination of whether a cancer cell is SOX9+ or not in the method in Fig. 1 can be determined or decided based on whether the cancer cell expresses the reporter gene or not.

The determination of the amount of SOX9+ cancer cells in step S3 can be based on measuring or counting the number of cancer cells expressing the reporter gene, by quantifying the characteristic conferred by the reporter gene, such as by fluorescent, spectrophotometry or bioluminescence measurements or in any other way detecting or quantifying the expression of the reporter gene or its gene product.

In an embodiment, the method also comprises comparing the determined amount of SOX9+ cancer cells with at least one threshold value. In such an embodiment, step S4 preferably comprises estimating the likelihood of cancer relapse following cancer treatment for the subject based on the comparison. For instance, the subject could be estimated to have a high likelihood of cancer relapse if the determined amount of SOX9+ cancer cells exceeds a threshold value or is equal to or exceeds the threshold value.

Correspondingly, the subject is estimated to have a low likelihood of cancer relapse if the determined amount of SOX9+ cancer cells is equal to or below the threshold value or below the threshold value.

A single threshold value could be used in the comparison. Alternatively, multiple, i.e., at least two, threshold values could be used in the comparison. In an example, two threshold values could be used to differentiate between subjects having low likelihood of cancer relapse from subjects having medium likelihood of cancer relapse and subjects having high likelihood of cancer relapse.

The actual values of the at least one threshold is at least partly dependent on the particular cancer disease and can be determined experimentally by determining the amount of the SOX9+ cancer cells in a plurality of subjects following cancer treatments and where diagnostic data regarding recurrence of any cancer disease is available. The at least one threshold value could then be determined based on the determined amounts of SOX9+ cancer cells in subjects having no cancer relapse and the determined amounts of SOX9+ cancer cells in subjects having cancer relapse.

Fig. 2 is a flow chart illustrating an additional step of the method in Fig. 1 according to an embodiment. In this embodiment, the method continues from step S3 in Fig. 1. A next step S10 comprises determining a proportion of the cancer cells following cancer treatment that are SOX9+cancer cells. The method then continues to step S4 in Fig. 1, which, in this embodiment, comprises estimating the likelihood of cancer relapse based on the determined proportion.

Hence, in this embodiment, the proportion of the cancer cells following the cancer treatment in step S2 that are SOX9+ cancer cells is determined in step S10. Such a proportion may, for instance, be a ratio between the number of SOX9+ cancer cells and the total number of cancer cells in the sample or a ratio between the number of SOX9+ cancer cells and the number of SOX9- cancer cells. The proportion may be expressed as a percentage of the cancer cells that are SOX9+ cancer cells or indeed any other quantity representing the proportion of cancer cells that are SOX9+.

Generally, a high proportion of SOX9+ cancer cells is associated with a high likelihood of cancer relapse and a comparatively low proportion of SOX9+ cancer cells is correspondingly associated with a lower likelihood of cancer relapse.

This embodiment of the method could also comprise a comparison between the proportion of SOX9+ cancer cells and at least one threshold value as discussed in the foregoing.

In an embodiment, the method also comprises determining the amount of SOX9+ cancer cells prior to cancer treatment based on expression of the reporter gene as shown in step S20 of Fig. 3. The determination in step S20 in Fig. 3 is typically conducted in a similar way as the determination in step S3 in Fig. 1. However, in step S20 the amount of SOX9+ cancer cells is determined prior to exposing ex *vivo* the cancer cells to the cancer treatment, whereas step S3 determines the amount of SOX9+ cancer cells following the cancer treatment. The method continues from step S20 in Fig. 3 to step S2 in Fig. 1. In an embodiment, step S4 comprises estimating the likelihood of cancer relapse based on the determined amounts of SOX9+ cancer cells prior to and following the cancer treatment.

In a particular embodiment, the method comprises step S21 as shown in Fig. 3. This step S21 comprises determining a first proportion of cancer cells prior to cancer treatment that are SOX9+ cancer cells. The method then continues to step S2 in Fig. 1. In this particular embodiment, a second proportion of cancer cells following cancer treatment that are SOX9+ cancer cells is determined in step S10 in Fig. 2. In such a particular embodiment, step S4 comprises estimating the likelihood of cancer relapse based on the determined first and second proportions.

The first and second proportions of SOX9+cancer cells can be determined as previously described herein in connection with Fig. 2. These two proportions can then be used as a basis to estimate whether the subject is likely to suffer from cancer relapse or not. Generally, the subject is estimated to have a high likelihood of cancer relapse if the second proportion is significantly higher than the first proportion. This would correspond to a situation where there is a large population of SOX9+ cancer cells that have escaped the cancer treatment applied in step S2 and are thereby generally resistant to the cancer treatment. Such a resistant population of SOX9+ cancer cells may cause cancer relapse in the subject by, additionally, having enhanced migratory capacity and being to form distant metastases. Hence, in an embodiment, step S4 comprises estimating a likelihood of cancer relapse based on a comparison of the first and second proportions.

The nucleic acid molecule comprising the reporter gene under transcriptional control of the SOX9p or of the promoter operatively linked to the enhancer element comprising at least one SOX9 binding site could be introduced into the cancer cells in step S1 using any nucleic acid delivery system or nucleic acid construct, typically a DNA or RNA delivery system or DNA or RNA construct, comprising the nucleic acid molecule.

In an embodiment, step S1 of Fig. 1 comprises transfecting ex *vivo* the cancer cells obtained from the tumor sample with nucleic acid molecules.

Non-limiting, but illustrative, examples of such nucleic acid delivery systems or nucleic acid constructs include vectors, such as plasmids or plasmid vectors, viral or virus vectors. In a particular embodiment, the vector is an expression vector capable of producing a protein product of the reporter gene through transcription of the reporter gene followed by translation of the messenger RNA (mRNA) produced.

In an embodiment, the expression vector comprises sequences that encode for a polyadenylation tail, which creates a polyadenylation tail at the end of the transcribed pre-mRNA that protects the mRNA from exonucleases and ensures transcriptional and translational termination and stabilizes mRNA production in the cancer calls; and Kozak sequence, which assembles the ribosome for translation of the mRNA. The expression vector may have minimal untranslated region (UTR) length for expression of the reporter gene in the cancer cells. UTRs contain specific characteristics that may impede transcription or translation and, thus, the shortest UTRs or none at all are encoded for in optimal expression vectors.

A currently preferred vector is a viral vector. In such an embodiment, step S1 in Fig. 1 comprises transducing ex *vivo* the cancer cells with a viral vector comprising the nucleic acid molecule.

In a particular embodiment, the viral vector is selected from a group consisting of a lentiviral (LV) vector, an adenoviral vector, an adeno-associated viral (AAV) vector, a rertroviral vector and a hybrid vector.

Lentiviruses are a subclass of retroviruses. They are adapted as gene delivery vehicles (vectors) thanks to their ability to integrate into the genome of non-dividing cells, which is the unique feature of lentiviruses as most other retroviruses can infect only dividing cells. The viral genome in the form of RNA is reverse-transcribed when the virus enters a cell to produce DNA, which is then inserted into the genome at a position by the viral integrase enzyme. The vector, now called a provirus, remains in the genome and is passed on to the progeny of the cell if it divides. For safety reasons lentiviral vectors typically never carry the genes required for their replication. To produce a lentivirus, several plasmids are transfected into a so-called packaging cell line, commonly HEK 293. One or more plasmids, generally referred to as packaging plasmids, encode the virion proteins, such as the capsid and the reverse transcriptase. Another plasmid contains the genetic material to be delivered by the vector. It is transcribed to produce the single-stranded RNA viral genome and is marked by the presence of the Ψ (psi) sequence. This sequence is used to package the genome into the virion.

Retroviruses are one of the mainstays of current gene therapy approaches. The recombinant retroviruses, such as the Moloney murine leukemia virus, have the ability to integrate into the host genome in a stable fashion. They contain a reverse transcriptase that allows integration into the host genome. Retroviral vectors can either be replication-competent or replication-defective. Replication-defective vectors are the most common choice because the viruses have had the coding regions for the genes necessary for additional rounds of virion replication and packaging replaced with other genes, or deleted. These viruses are capable of infecting various cells and delivering their viral payload, but then fail to continue the typical lytic pathway that leads to cell lysis and death.

If the expression vector is a lentiviral or retroviral vector then the nucleic acid molecule is preferably a RNA molecule.

Adenoviral vector as used herein include adenovirus vectors and adenovirus-derived virus vectors. Adenoviral DNA does not integrate into the genome and is not replicated during cell division. An adeno-derived virus vector is based on an adenovirus but in which various modifications have been done, such as relating to the nucleotide sequences coding replication proteins, regulation protein, viral surface proteins, etc. Adeno-associated virus (AAV) is a small virus that infects humans and some other primate species. AAV can infect both dividing and non-dividing cells and may incorporate its genome into that of the host cell. Moreover, AAV mostly stays as episomal, performing long and stable expression. Whereas AAV packages a single strand of DNA and requires the process of second-strand synthesis, self-complementary adeno-associated virus (scAAV) packages both strands which anneal together to form double stranded DNA. By skipping second strand synthesis, scAAV allows for rapid expression in the cell.

If the expression vector is an adenoviral vector then the nucleic acid molecule is preferably a DNA molecule.

A hybrid vector is a vector virus that is genetically engineered to have qualities of more than one vector. For instance, a hybrid vector may be a combination of an adenovirus and a lentivirus.

Currently preferred viral vectors include LV and AAV vectors. LV and AAV vectors are capable of efficiently transducing non-dividing cells, making them promising tools for delivering genes to quiescent and dormant cancer cells.

Although a vector is currently preferred for introducing ex *vivo* the nucleic acid molecule into the cancer cells in step S1 in Fig. 1 the embodiments are not limited thereto. In fact, naked DNA or a non-vector DNA construct could be introduced into the cancer cells using various methods including, but not-limited to, chemical based transfection, such as using calcium phosphate, cationic polymers, lipofection, fugene or dendrimer; electroporation; cell squeezing; sonoporation; optical transfection; protoplast fusion; and particle-based methods, such as magnetofection or impalefection.

The cancer treatment applied to the cancer cells ex *vivo* could be any cancer treatment that mimics a cancer treatment that can be applied *in vivo* to the subject. For instance, the cancer treatment could be selected from the group consisting of chemotherapy, radiation therapy, immunotherapy, and any combination thereof.

Chemotherapy is a type of cancer treatment that uses one or more anti-cancer drugs, i.e., chemotherapeutic agents, including, but not limited to, alkylating agents, anthracyclines, cytoskeletal disruptors (taxanes), epothilones, histone deacetylase inhibitors, inhibitors of topoisomerase I, inhibitors of topoisomerase II, kinase inhibitors, nucleotide analogs and precursors thereof, peptide antibiotics, platinum-based agents, retinoids and vinca alkaloids and derivatives thereif. In a particular embodiment, the at least one chemotherapeutic agent added to or contacting the cancer cells *ex vivo* in step S2 is preferably the same or comprises the same active agent as the at least one chemotherapeutic agent selected for the cancer treatment of the subject.

Radiation therapy, also referred to as radiotherapy, is a cancer treatment comprising irradiation cancer cells using ionizing radiation, such as proton therapy. Any type of radiation therapy that is traditionally used during cancer treatment could be used in step S2 including external beam radiation therapy, brachytherapy, and radioisotope therapy.

Immunotherapy is a cancer treatment that is based on activating the immune system. Cell-based immunotherapies are effective for some cancers using immune effector cells, such as lymphocytes, macrophages, dendritic cells, natural killer cells, and cytotoxic T lymphocytes to defend the body against cancer by targeting abnormal antigens expressed on the surface of cancer cells. Such immunotherapies also include therapies involving engineered immune cells, such as chimeric antigen receptor (CAR) T cell therapy. In such a case, step S2 comprises adding immune effector cells *ex vivo* to the cancer cells.

The cancer treatment applied in step S2 could also be a combination of two or more of chemotherapy, radiation therapy and immunotherapy.

In an embodiment, the method also comprises selecting a cancer treatment to use in step S2 based on the type of cancer disease the subject is suffering from. Hence, in an embodiment, the particular cancer treatment to expose the cancer cells to *ex vivo* is preferably dependent on the particular cancer disease the subject is suffering from and preferably corresponds to the cancer treatment selected for the subject. Hence, the *ex vivo* cancer treatment in step S2 should preferably represent or mimic the planned or, indeed ongoing, cancer treatment for the subject.

In an embodiment, the subject is suffering from a solid tumor cancer disease. Illustrative, but non-limiting, examples of such solid tumor cancer diseases include a brain cancer, a lung cancer, a colon cancer, a skin cancer, a breast cancer, a renal cancer, a thyroid cancer, a gastrointestinal cancer, a pancreatic cancer, a liver cancer, a colorectal cancer, a bladder cancer, a gastric cancer, an ovarian cancer, and a prostate cancer, preferably a brain cancer. In a particular embodiment, the subject is suffering from a solid tumor cancer disease, preferably selected from the group consisting of a brain cancer, a lung cancer, a colon cancer, a skin cancer, a breast cancer and a prostate cancer, and more preferably a brain cancer.

In a preferred embodiment, the subject is suffering from a malignant brain cancer and more preferably such a malignant brain cancer disease selected from the group consisting of a malignant brain cancer, preferably MB and GBM.

The tumor sample taken from the subject to obtain the cancer cells could be any sample comprising cancer cells from the tumor. A typical example is tumor biopsy.

In an embodiment, the method comprises an additional step S30 as shown in Fig. 4. This step S30 comprises determining the subject to be likely to suffer from cancer relapse based on the likelihood estimated in step S4 in Fig. 1.

Hence, this embodiment identifies or selects the subject to be a subject that is likely to suffer from cancer relapse, i.e., has an estimated high likelihood of cancer relapse as determined based on the determined amount of SOX9+ cancer cells in the tumor sample following ex *vivo* exposure to the cancer treatment.

In a particular embodiment, the method of estimating likelihood is a decision support method, i.e., a non-diagnostic method. This means that the decision support method will result in decision support information, which includes merely interim results. Additional data and the competence of a physician are typically required for providing a final diagnosis or prognosis. Furthermore, other parameters than the amount of SOX9+ cancer cells may influence any cancer relapse, such as the case history, age and sex of the subject, the type of tumor or cancer, genetic factors, etc. Thus, the method gives, in an embodiment, a decision support upon which a physician can base his or her decision about which measures that should be taken.

The present invention not only enables predicting or estimating the likelihood of cancer relapse for subjects suffering from a cancer disease. The invention can also be used to predict or estimate a response to a cancer treatment for a subject.

Hence, another aspect of the invention relates to an *in vitro* method of predicting a response to a cancer treatment, see Fig. 5. The method comprises introducing, in step S40, *ex vivo* a nucleic acid molecule comprising a reporter gene under transcriptional control of a SOX9p or of a promoter operatively linked to an enhancer element comprising at least one SOX9 binding site and a suicidal gene under transcriptional control of a SOX9p or of a promoter operatively linked to an enhancer element comprising at least one SOX9 binding site into cancer cells obtained from a tumor sample from a subject. Alternatively, or in addition, step S40 comprises introducing *ex vivo* a first nucleic acid molecule comprising the reporter gene under transcription control of a SOX9p or of a promoter operatively linked to an enhancer element comprising at least one SOX9 binding site and a second nucleic acid molecule comprising the suicidal gene under transcriptional control of a SOX9p or of a promoter operatively linked to an enhancer element comprising at least one SOX9 binding site into the cancer cells. The suicidal gene encodes a prodrug activating protein capable of converting a prodrug or a metabolite thereof into a cytotoxic agent.

The method also comprises exposing, in step S41, *ex vivo* the cancer cells to a cancer treatment. A next step S42 comprises determining a first amount of SOX9+ cancer cells following cancer treatment based on expression of the reporter gene. The method further comprises adding *ex vivo* the prodrug to the cancer cells in step S43 and determining a second amount of SOX9+ cancer cells following addition of the prodrug based on expression of the reporter gene in step S44. The method additionally comprises predicting, in step S45, a response of the subject to a cancer treatment based on the determined first and second amounts.

In an embodiment, the *in vitro* method of predicting a response to a cancer treatment comprises introducing, in step S40, *ex vivo* i) a nucleic acid molecule comprising a reporter gene under transcriptional control of a SOX9p and a suicidal gene under transcriptional control of a SOX9p or ii) a first nucleic acid molecule comprising the reporter gene under transcriptional control of a SOX9p and a second nucleic acid molecule comprising the suicidal gene under transcriptional control of a SOX9p into cancer cells obtained from a tumor sample from a subject. The suicidal gene encodes a prodrug activating protein capable of converting a prodrug or a metabolite thereof into a cytotoxic agent. The method also comprises exposing, in step S41, *ex vivo* the cancer cells to a cancer treatment and determining, in step S42, a first amount of SOX9+cancer cells following cancer treatment based on expression of the reporter gene. The method further comprises adding, in step S43, *ex vivo* the prodrug to the cancer cells and determining, in step S44, a second amount of SOX9+cancer cells following addition of the prodrug based on expression of the reporter gene. The method additionally comprises predicting, in step S45, a response of the subject to a cancer treatment based on the determined first and second amounts.

The suicidal gene, also referred to as suicide gene, will cause the cancer cells to kill themselves through apoptosis. Suicidal genes are an efficient strategy for making cancer cells more vulnerable and sensitive to cytotoxic agents. As an example, a suicidal gene can mediate this sensitivity by coding for a prodrug activating protein, such as an enzyme, which converts an inactive drug, i.e., a prodrug or a metabolite thereof, into a cytotoxic agent, such as a toxic antimetabolite, for instance, inhibiting the synthesis of nucleic acid.

There are different methods of suicidal gene therapy that can be used. For instance, gene-directed enzyme-producing therapy (GDEPT) uses a gene taken from the cancer cell and then modified with other genes to form enzymes that are harmless to healthy cells. This foreign enzyme is inserted into the cancer cells where it releases a prodrug, which is harmless to healthy cells, but destructive to the cancer cells. The modified suicidal gene converts the non-toxic prodrug into a cytotoxic substance. Another example of suicide gene therapy is called virus-directed enzyme-prodrug therapy. This method uses a virus as the carrier, or vector, to deliver the modified gene(s) to the cancer cells.

Various combinations of suicidal genes and prodrugs could be used according to the embodiments. For instance, herpes simplex virus-1 thymidine kinase (HSV-TK) (EC 2.7.1.21) could be used together with ganciclovir (GCV) or valganciclovir (ValGCV). HSV-TK converts GCV into ganciclovir triphosphate (GCV-TP), which blocks DNA synthesis, causing S and G2 phase arrest and mitochondrial damage, and ultimately apoptosis. The cytotoxic agent is active in proliferating cells. ValGCV is the L-valyl ester of GCV and is broken down into GCV by the intestine and liver when administered to a subject. Hence, GCV is a metabolite of ValGCV.

Another example of suicidal gene is cytosine deaminase (EC 3.5.4.1), which converts the prodrug 5-fluorocytosine (5-FC) into 5-fluorouracil (5-FU). 5-FU blocks DNA and RNA synthesis and is active mostly in proliferating cells, but can, in high concentrations, inhibits growth of both proliferating and non-proliferating cells.

A further example of suicidal gene is nitroreductase (NTR), such as *Escherichia coli* NTR (EC 1.6.99.7), which converts the prodrug CB1954, i.e., 5-(aziridin-1-yl)-2,4-dinitrobenzamide, and analogues thereof, into 2-hydroxylamine and 4-hydroxylamine derivatives. The resulting cytotoxic agents cause cross linking of DNA interstrands and are active in both proliferating and non-proliferating cells.

Carboxypeptidase G2 (3.4.17.11) is another suicidal gene that can convert the prodrugs CMDA, i.e., N-[4-[(2-chloroethyl)(2-mesyloxyethyl)amino]benzoyl]-I-glutamic acid, compound 39b, i.e., 4-[N-[4'-bis(2' '-iodoethyl)aminophenyl]-N'-methylcarbamoyloxymethyl]phenylcarbamoyl-I-glutamic acid, and ZD-2767P, i.e., bis-iodo phenol mustard, into cytotoxic agents, such as N-4-[(2-chloroethyl)(2-mesyloxyethyl)amino]benzoic acid (CMBA) and bis-iodophenol mustard. These cytotoxic agents cause cross linking of DNA interstrands and are active in both proliferating and non-proliferating cells.

Yet another suicidal gene that could be used is cytochrome P450 (EC 1.14.-.-), which converts the prodrugs cyclophosphamide and ifosfamide into phosphoramide mustard and acrolein, respectively. The resulting cytotoxic agents cause cross linking of DNA interstrand and are active mostly in proliferating cells.

Purine nucleoside phosphorylase (EC 2.4.2.1) catalyzes the glycosidic cleavage of purine ribonucleoside prodrugs, such as 6-methylpurine 2-deoxyriboside (MEP-dr) and fludarabine, into 2-deoxyribose-1-phosphate (or arabinose-1-phosphate) and free base compounds, such as 6-methylpurine and 2-fluoroadenine. The resulting cytotoxic agents inhibit DNA, RNA and protein synthesis and are active in both proliferating and non-proliferating cells.

In this aspect, quiescent and dormant SOX9+ cancer cells surviving the cancer treatment applied in step S41 is targeted using another type of cancer treatment in terms of gene therapy or suicidal or suicide gene therapy. In an embodiment, the effectiveness of this type of cancer treatment, i.e., the gene therapy, can then be assessed for the subject based on the determined amounts of SOX9+ cancer cells prior to and following addition of the prodrug in step S43.

Generally, if the second amount of SOX9+ cancer cells determined in step S44 is comparatively lower than the first amount of SOX9+ cancer cells determined in step S42 the cancer cells respond well, i.e., are effectively killed by the gene therapy, and the subject's response to such a cancer treatment is thereby predicted to be good. However, if the second amount of SOX9+ cancer cells determined in step S44 is equal to or even higher than the first amount of SOX9+ cancer cells determined in step S42, the subject is predicted to not respond well to any SOX9-based gene therapy.

Hence, in an embodiment, step S45 comprises predicting a response of the subject to a suicidal gene therapy based on the determined first and second amounts. The suicidal gene therapy comprises administration of a medicament according to the invention to the subject, i.e., administration of a viral vector comprising the suicidal gene under transcriptional control of the SOX9p or of a promoter operatively linked to an enhancer element comprising at least one SOX9 binding site and administration of a prodrug. The suicidal gene encodes a prodrug activating protein capable of converting the prodrug or a metabolite thereof into a cytotoxic agent.

The *in vitro* method in Fig. 5 can also provide valuable information of the effectiveness of the cancer treatment applied in step S41 and can thereby be used to predict a response of the subject to a corresponding cancer treatment as applied to the subject. For instance, if the amount of SOX9+ cancer cells determined in step S42 is low the subject is predicted to respond well to the cancer treatment, whereas if the amount of SOX9+ cancer cells determined in step S42 is high the subject is predicted to not respond well to the cancer treatment.

The determination of the amount of SOX9+ cancer cells in step S42 and S44 can be performed as previously described herein in connection with step S3 in Fig. 1.

Furthermore, a respective proportion of cancer cells that are SOX9+ cancer cells could be determined in step S42 and S44 as previously described in connection with steps S10 in Fig. 2 and S21 in Fig. 3. Hence, in an embodiment, a first proportion of cancer cells following cancer treatment that are SOX9+ cancer cells is determined in step S42 and a second proportion of cancer cells following addition of the prodrug that are SOX9+ cancer cells is determined in step S44. In such an embodiment, step S45 comprises predicting the response of the subject to the cancer treatment based on the first and second proportions.

The prediction in step S45 is based on the determined amounts of SOX9+ cancer cells or the first and second proportions. For instance, a ratio could be calculated between the determined amounts of SOX9+ cancer cells or the first and second proportions and used as a basis for the prediction. In another embodiment, the prediction is based on a difference between the amounts of SOX9+ cancer cells or the first and second proportions.

Step S41 in Fig. 5 can be performed as previously described herein in connection with step S2 of Fig. 1 and is not further described herein.

In an embodiment, a single nucleic acid molecule is introduced ex *vivo* in step S40 into the cancer cells. This nucleic acid molecule then comprises both the reporter gene and the suicidal gene both under transcriptional control of the SOX9p or of the promoter operatively linked to the enhancer element comprising at least one SOX9 binding site. In an embodiment, both the reporter gene and the suicidal gene could be under transcriptional control of a single, common SOX9p or of a single, common promoter operatively linked to the enhancer element comprising at least one SOX9 binding site. In another embodiment, the nucleic acid molecule comprises the reporter gene under transcriptional control of a first SOX9p or of a first promoter operatively linked to the enhancer element comprising at least one SOX9 binding site and the suicidal gene under transcriptional control of a second SOX9p or of a second promoter operatively linked to the enhancer element comprising at least one SOX9 binding site.

The discussion in the foregoing regarding the nucleic acid molecule introduced ex *vivo* in step S1 in Fig. 1, including type of nucleotides, nucleic acid delivery system or construct and usage of vector, also applies to the single nucleic acid molecule introduced *ex vivo* in step S40.

In another embodiment, two nucleic acid molecules are introduced *ex vivo* into the cancer cells in step S40. The first nucleic acid molecule then comprises the reporter gene under transcriptional control of a (first) SOX9p or of a (first) promoter operatively linked to the enhancer element comprising at least one SOX9 binding site and the second nucleic acid molecule comprises the suicidal gene under transcriptional control of a (second) SOX9p or of a (second) promoter operatively linked to the enhancer element comprising at least one SOX9 binding site. The discussion in the foregoing regarding the nucleic acid molecule introduced *ex vivo* in step S1 in Fig. 1, including type of nucleotides, nucleic acid delivery system or construct and usage of vector, also applies to the first and second nucleic acid molecules introduced *ex vivo* in step S40.

The single nucleic acid molecule or the second nucleic acid molecule could comprise a single suicidal gene or multiple suicidal genes. Hence, it is possible to use a combination of different suicidal genes. In such a case, step S43 in Fig. 5 comprises adding *ex vivo* multiple prodrugs to the cancer cells, i.e., at least one prodrug per suicidal gene in the single nucleic acid molecule or the second nucleic acid molecule. If the single or second nucleic acid molecule comprises multiple suicidal genes the genes could be under transcriptional control of a single, common SOX9p or of a single, common promoter operatively linked to the enhancer element comprising at least one SOX9 binding site or be under transcriptional control of a respective SOX9p or of a promoter operatively linked to the enhancer element comprising at least one SOX9 binding site.

The first nucleic acid molecule comprising the reporter gene under transcriptional control of the SOX9p or of the promoter operatively linked to the enhancer element comprising at least one SOX9 binding site is introduced *ex vivo* into the cancer cells preferably prior to exposing *ex vivo* the cancer cells to the cancer treatment in step S41. The second nucleic acid molecule comprising the suicidal gene under transcriptional control of the SOX9p or of the promoter operatively linked to the enhancer element comprising at least one SOX9 binding site is introduced *ex vivo* into the cancer cells at any point in time prior to adding *ex vivo* the prodrug to the cancer cells in step S43. Hence, the second nucleic acid molecule could be introduced *ex vivo* into the cancer cells prior to step S41, following step S41 but prior to step S42 or following step S42 but prior to step S43. The two nucleic acid molecules could be introduced *ex vivo* to the cancer cells simultaneously or serially, in any order.

In an embodiment, step S40 is implemented as shown in Fig. 6. This embodiment comprises transducing *ex vivo* the cancer cells with a first viral vector comprising the first nucleic acid molecule in step S50 and transducing *ex vivo* the cancer cells with a second viral vector comprising the second nucleic acid molecule in step S51. The method then continues to step S41 in Fig. 5.

A related aspect of the invention defines a kit for use in the *in vitro* method according to above. The kit comprises i) a nucleic acid molecule comprising a reporter gene under transcriptional control of a *SOX9* promoter or of a promoter operatively linked to an enhancer element comprising at least one SOX9 binding site and a suicidal gene under transcriptional control of a *SOX9* promoter or of a promoter operatively linked to an enhancer element comprising at least one SOX9 binding site, or ii) a first nucleic acid molecule comprising the reporter gene under transcriptional control of a *SOX9* promoter or of a promoter operatively linked to an enhancer element comprising at least one SOX9 binding site and a second nucleic acid molecule comprising the suicidal gene under transcriptional control of a *SOX9* promoter or of a promoter operatively linked to an enhancer element comprising at least one SOX9 binding site. The kit also comprises a prodrug. The suicidal gene encodes a prodrug activating protein capable of converting the prodrug or a metabolite thereof into a cytotoxic agent.

A further aspect of the invention relates to a viral vector comprising a suicidal gene under transcriptional control of a SOX9p or of a promoter operatively linked to an enhancer element comprising at least one SOX9 binding site. This viral vector may be used to transduce *ex vivo* the cancer cells in step S51 in Fig. 6. This viral vector may also be used to transduce *in vivo* the cancer cells in step S60 in Fig. 7.

In an embodiment, the viral vector comprises a suicidal gene under transcriptional control of a SOX9p.

The suicidal gene comprised in the viral vector can be selected among the previously disclosed suicidal genes. Hence, in an embodiment, the suicidal gene encodes a protein selected from the group consisting of herpes simplex virus-1 thymidine kinase (HSV-TK), cytosine deaminase, nitroreductase, caboxypeptidase G2, cytochrome P450, purine nucleoside phosphorylase, and a combination thereof.

In a particular embodiment, the suicidal gene encodes HSV-TK.

The viral vector may comprise a single suicidal gene or multiple different suicidal genes as mentioned in the foregoing. In the latter case, the multiple different suicidal genes could be under transcriptional control of a single, common SOX9p or under transcriptional control of a respective SOX9p.

The viral vector may be selected among the previously described examples of viral vectors. Hence, in an embodiment, the viral vector is selected from the group consisting of a LV vector, an adenoviral vector, an AAV vector, a retroviral vector and a hybrid vector. In a particular embodiment, the viral vector is selected from the group consisting of a LV vector and an AAV vector. An illustrative example of viral vector that could be used according to the invention is an AAV serotype 9 (AAV9) vector.

LV vectors have become particularly attractive for clinical applications due to their ability to more efficiently transduce non-proliferating or slow-proliferating cells. A phase 1/2 study of ProSavin, a LV vector gene therapy for Parkinson's disease, demonstrated the safety of local lentiviral gene delivery in the CNS (Human Gene Therapy Clinical Development 2018, 29: 148-155). *In vivo* gene delivery using lentiviral vector has also been applied clinically (Human Gene Therapy 2017, 28: 99-111). Disadvantages of LV vectors include relatively moderate titers (≤ 10⁹ infectious particles/mL) and the potential of insertional mutagenesis. AAV vectors have revealed their strong potential for *in vivo* gene delivery. AAV vectors can transduce a wide range of tissue and lead to long-term transgene expression both dividing and non-dividing cells (Cancer Gene Therapy 2005, 12: 913-925). Secondly, AAV vectors are very safe since wild-type AAV has never been shown to cause any human disease (Journal of Virology 2000, 74: 11456-11463), seldom cause immune reactions and have low pathogenicity (Neurobiology of Disease 2012, 48: 179-188). AAV persist as stable episomes and remain transcriptionally active in multi-year transgene expression after gene transfer to the liver has been documented in dogs, primates and humans (Blood 2009, 113: 797-806; The New England Journal of Medicine 2014, 371: 1994-2004). The limitations of AAV vectors include the difficulty in producing large quantities of infectious particles and their small transgene packaging capacity (≤4.5 kb). To date, there are 12 naturally occurring serotypes that differ somewhat in their gene delivery properties. AAV9 vectors are very efficient in crossing the blood brain barrier (BBB) and are able to deliver transgenes to brain and spinal cord after intravenous vector injection *(*Human Gene Therapy 2008, 19: 61-70).

In an embodiment, the viral vector is capable of transducing non-dividing cells. As mentioned in the foregoing, many tumors comprise a population of quiescent and dormant, i.e., non-dividing, SOX9+ cancer cells. The viral vector is thereby preferably capable of transducing such SOX9+ cancer cells.

Generally, viral vectors capable of transducing non-dividing cells can cross the nuclear membrane of the non-dividing cells. The genome of these viral vectors can enter the nucleus through nuclear pores by having a sequence generally denoted flap sequence or triplex sequence, which allows the nucleotide molecule to adopt a confirmation allowing passage in a nuclear pore in the nuclear membrane (Somatic Cell and Molecular Genetics 2001, 26: 35-49).

Generally, successful gene therapy depends on the selective introduction of therapeutic genes into the appropriate target cancer cells. Enzyme prodrug therapy is a suicidal gene therapy approach that relies on the use of enzymes, which convert normally non-toxic prodrugs into a cytotoxic agent, leading to the selective killing of target cancer cells. There are several suicide genes encoding prodrug-activating enzymes, among them, the well characterized HSV-TK (Proceedings of the National Academy of Sciences of the United States of America 1996, 93: 3525-3529) that has been studied extensively in preclinical and clinical studies to treat a wide range of solid tumors including brain tumors (Nature medicine 1999, 5: 1256-1263). HSV-TK phosphorylates the prodrug GCV into a toxic metabolite that kills the target cancer cells. Additionally, the toxic metabolite can be efficiently transferred to adjacent cells resulting in elimination of unmodified cells in so-called 'bystander effect'. The strategy can be so efficient that as few as 10 % of the cancer cells expressing the HSV-TK gene in a population is sufficient to eliminate all tumor cells and it has been used in several clinical trials of various cancer types (Annals of Neurology 2003, 54: 479-487; Cancer Gene Therapy 2009, 16: 723-730). ValGCV is an orally administered prodrug that gets rapidly metabolized to active GCV in intestine and liver and is suitable for long-term treatment. ValGCV is a standard drug in clinical practice for cytomegalovirus infections in e.g., the brain and the spinal cord (Antiviral Research 2006, 71: 154-163). In children and in adults, it penetrates the BBB well and reaches high concentrations in the cerebrospinal fluid (CSF) (Italian Journal of Pediatrics 2015, 41: 26).

To specifically target SOX9+ cancer cells with a suicidal gene therapy approach, the viral prodrug enzyme, such as HSV-TK, may be incorporated into a self-inactivating viral vector controlled by SOX9p, preferably human SOX9 promoter (hSOX9p). LV and AAV vectors are capable of efficiently transducing non-dividing cells (Science 1996, 272: 263-267), making them promising tools for delivering genes to quiescent and dormant cancer cells that escape treatment. The hSOX9p is silent in the normal brain cells, except for certain stem cells, mature glial cells and some cells in the choroid plexus. Most importantly, even if the viral vector is targeting SOX9+ non-dividing cells when applied, the activated prodrug is specifically cytotoxic only to actively dividing cells. The approach, thus, avoids killing of normal non-dividing brain cells, therefore providing an additional level of tumor selectivity.

Emergence of therapy resistance is a major cause of treatment failure with conventional chemotherapy and radiation. It is therefore imperative to find novel options to target therapy-resistant tumor populations. Previous gene therapies have only employed viruses hitting all dividing tumor cells and not given any targeted approach also to non-dividing tumor cells. The gene-directed enzyme prodrug therapy disclosed herein provides targeted drug activation also in radiation- and chemo-resistant non-dividing cancer cells. After eradication of the bulk tumor using standard cancer treatment, the viral vector of the invention can be used for targeting of remaining quiescent cancer cells, which very likely escape the standard cancer treatment. Prodrug administration at time of tumor recurrence will eliminate the treatment-resistant cancer clones once they became dangerous and reveal themselves.

This approach is outlined in Fig. 11. Hence, a viral vector of the embodiments comprising a suicidal gene, such as HSV-TK, under transcriptional control of a SOX9p, such as hSOX9p, is used to infect tumor cells, such as brain tumor cells, in a subject. The subject then undergoes standard cancer treatment to eradicate the bulk of the tumor. Administration of a prodrug for the suicidal gene, such as GCV or ValGCV for HSV-TK, will then induce death of SOX9+ cancer cells that may otherwise cause cancer relapse in the subject.

Hence, yet another aspect of the invention relates to a medicament comprising a viral vector according to the invention and a prodrug for use in treatment of a cancer disease in a subject. The suicidal gene comprised in the viral vector under transcription control of SOX9p or of the promoter operatively linked to the enhancer element comprising at least one SOX9 binding site encodes a prodrug activating protein capable of converting the prodrug or a metabolite thereof into a cytotoxic agent.

In an embodiment, the viral vector is formulated for administration to the subject prior to, during and/or following exposing the subject to a cancer treatment. In this embodiment, the prodrug is formulated for administration to the subject following administration of the viral vector to the subject.

Thus, the viral vector could be administered to the subject before the cancer treatment, as a part of the cancer treatment and/or indeed after the cancer treatment. The prodrug is then administered to the subject once the viral vector has been administered to the subject. In a particular embodiment, the prodrug is formulated for administration to the subject following administration of the viral vector to the subject and following exposing the subject to the cancer treatment. For instance, the prodrug is administered to the subject in response to detection of cancer relapse in the subject. Alternatively, the prodrug may be administered as prophylaxis if the subject is determined or identified as being likely to suffer from cancer relapse as previously disclosed herein in connection with Figs. 1 to 4.

In an embodiment, the suicidal gene encodes HSV-TK. In such an embodiment, the prodrug is preferably selected from the group consisting of GCV, a prodrug of GCV, preferably ValGCV, and a combination thereof.

The cancer treatment applied to the subject to eradicate the bulk of the tumor could be any standard cancer treatment, such as selected, among others, based on the type of tumor. In an embodiment, the cancer treatment is selected from the group consisting of surgery, chemotherapy, radiation therapy, immunotherapy, hormonal therapy, and a combination thereof. In a particular embodiment, the cancer treatment is selected from the group consisting of surgery, chemotherapy, radiation therapy, and a combination thereof.

In an embodiment, the subject is suffering from a solid tumor cancer disease. In a particular embodiment, the subject is suffering from a solid tumor cancer disease selected from the group consisting of a brain cancer, a lung cancer, a colon cancer, a skin cancer, a breast cancer, a renal cancer, a thyroid cancer, a gastrointestinal cancer, a pancreatic cancer, a liver cancer, a colorectal cancer, a bladder cancer, a gastric cancer, an ovarian cancer, and a prostate cancer, preferably a brain cancer. In a particular embodiment, the subject is suffering from a solid tumor cancer disease, preferably selected from the group consisting of a brain cancer, a lung cancer, a colon cancer, a skin cancer, a breast cancer and a prostate cancer, and more preferably a brain cancer.

In a preferred embodiment, the subject is suffering from a malignant brain cancer and more preferably such a malignant brain cancer disease selected from the group consisting of a malignant brain cancer, preferably MB and GBM.

In a particular embodiment, the subject is suffering from a cancer disease selected from the group consisting of MB and GBM.

The medicament of the invention is useful in treatment, including inhibition or prevention, of a cancer disease, and in particular for treatment, including inhibition or prevention, of a cancer relapse.

The present invention also relates to a method of treating a subject suffering from a cancer disease, see Fig. 7. The method comprises administering, in step S60, a viral vector according to the invention to the subject prior to, during and/or following exposing the subject to a cancer treatment. The method also comprises administering, in step S61, a prodrug to the subject. The suicidal gene encodes a prodrug activating protein capable of converting the prodrug or a metabolite thereof into a cytotoxic agent.

The viral vector is preferably administered to the subject in step S60 by intravenous or subcutaneous administration. Alternatively, or in addition, the viral vector could be administered into the tumor, e.g., in the form of an intratumoral injection. Also other administration routes could be used depending on the type of cancer disease the subject is suffering from, such as intracisternal administration for brain tumors, such as in MB or GBM subjects.

The prodrug could be administered to the subject in step S61 according to various administration routes including, but not limited to, oral administration, intravenous administration, subcutaneous administration, intratumoral administration, nasal administration, buccal administration, rectal administration, dermal administration, tracheal administration, bronchial administration, or topical administration.

The viral vector and/or the prodrug may be formulated with any suitable pharmaceutically acceptable excipient or carrier that may be selected based on the particular viral vector or prodrug and/or based on the particular administration route.

In an embodiment, the method comprises an additional step S70 as shown in Fig. 8. The method starts in this step S70 or continues from step S60 in Fig. 7. Step S70 comprises exposing the subject to a cancer treatment. In an embodiment, the cancer treatment is selected from the group consisting of surgery, chemotherapy, radiation therapy, immunotherapy, hormonal therapy, and a combination thereof. In a particular embodiment, the cancer treatment is selected from the group consisting of surgery, chemotherapy, radiation therapy, and a combination thereof.

In an embodiment, the method comprises an additional step S80 as shown in Fig. 9. The method then comprises selecting, in step S80, a subject to be likely to suffer from cancer relapse based on a likelihood of cancer relapse as estimated according to the invention as disclosed herein in connection with Figs. 1 to 4. The method then continues to step S60 in Fig. 7, where the viral vector is administered to the selected subject.

### EXAMPLES

### EXAMPLE 1

Tumor recurrence is a slow biological process involving therapy resistance, immune escape and metastatic spread. It is also the leading cause of death in medulloblastoma (MB), the most frequent malignant pediatric brain tumor. By following cellular changes in paired primary-recurrent patient samples and patient-derived xenografts significant accumulation of SOX9-positive cells were identified in relapses and metastases. Such cells exist as rare, quiescent cells in biopsies of Group 3 and Group 4 patients that constitute two thirds of MB. To follow tumor relapse mechanisms at the single cell level an inducible dual Tet Off animal model of MYC-driven MB was developed, where MYC can be directed from treatment-sensitive bulk cells to resistant, dormant SOX9-positive cells. SOX9 promotes stemness, immune escape and DNA repair suppression and is essential for brain tumor relapses. Tumor cell dormancy was non-hierarchical, migratory and depended on MYC suppression by SOX9. By using computational modeling and targeted treatment we further identified and showed how doxorubicin and MGMT inhibitors are specifically targeting these dormant and therapy-evading cells.

### Material and Methods

### Patient samples

Paraffin-embedded tissue sections from paired primary and recurrent human MB samples were obtained from Dr. Ulrich Schüller and the tissue archive of the Center for Neuropathology, Hospital of University of Munich, after patients had given their informed consent. Approval was obtained from the institutional review boards of the hospital. Data on the DNA methylation levels of two CpGs (cg12434587 and cg12981137) in the MGMT promoter was derived from 450K methylation array (Illumina) analysis of Genomic DNA from 11 Group 3 and 19 Group 4 matched primary-recurrent MB pairs from patients treated at UK CCLG institutions and collaborating centers and generated as previously described (Cancer Cell 2015, 27: 72-84). Informed consent was obtained from all subjects and human tumor investigations were conducted with approval from Newcastle/North Tyneside Research Ethics Committee. We also used transcriptome and DNA copy-number data for SOX9, MYC and MYCN genes in 64 primary human MB specimens that were obtained and analyzed as previously described (J Clin Oncol 2011, 29: 3852-3861). We used transcriptome data from 763 primary human. Their expression profiling with the classification of the four different subgroups have been described before (Cancer Cell 2017, 31: 737-754.e736).

### Animal experiments

Several different transgenic mouse lines were used in this study. The Glt1-tTA; TRE-MYCN:Luc (GTML) model and the bioluminescence imaging using Luciferase has been previously described (Genes & development 2010, 24: 1059-1072; Cancer Cell 2012, 21: 601-613). Mice carrying SOX9-rtTA transgene were kindly provided by Dr. Michael German (UCSF) and crossed with TRE-MYCN:Luc to generate the STML mice in an FVB/N background. Tg(SOX9-EGFP)EB209Gsat (pSOX9-GFP) animals were obtained from Mutant Mouse Resource & Research Centers (MMRRC) facility at UC Davies, California, USA. Glt1-tTA was crossed with pSOX9-GFP to generate the GTML; pSOX9-GFP model and Glt1-tTA was crossed with SOX9-rtTA strains in the FVB/N background and were both kept as doubly heterozygouts. Athymic Nude mice were obtained from Envigo and NOD Scid gamma (NSG) mice from Jackson Laboratory. Animals were euthanized upon development of tumor symptoms (hunched back, paralysis, reduction of fur quality, weight loss and tilted head) or one year post transplantation. Sections from tumors from Ptch+/-/Math1-SB11/T2Onc animals were obtained from Dr. Michael Taylor (Toronto) and generated as previously described (Nature 2012, 482: 529-533). All animal experiments were performed in accordance with local animal ethical committees.

### Orthotopic transplantation

Injections of tumor cells into mouse brains were performed as thoroughly described elsewhere (Cancer Cell 2016, 29: 311-323; Cancer Cell 2012, 21: 601-613) and as follows. For allograft and *in vivo* transplantation studies, adult Nude mice were anesthetized and cerebellar allografts generated by injection of 100,000 cells into the cerebellum. Mice were monitored until phenotypic presentation of a brain tumor, at which point they were euthanized. At sacrifice, whole brains were excised and biopsies taken for experimental procedures. PDX models used included BT084 (metastatic SHH MB established in Dr. Till Milde's lab, Heidelberg, Germany), Med-211FH, Med-411FH (metastatic Group 3 MBs established in Dr. Jim Olson's lab, Seattle, USA) and ICb-1572MB (metastatic Group 3 MB established in Dr. Xiaonan Li's lab, Houston, USA) all obtained from Dr. Robert Wechsler-Reya's lab and used as described elsewhere (Cancer Cell 2016, 29: 311-323). Orthotopic PDX growth was studied in brain and spinal cord in NSG mice that were maintained in the animal facilities at Sanford Burnham Prebys Medical Discovery Institute (SBP) and at the Sanford Consortium for Regenerative Medicine. All experiments were performed in accordance with national guidelines and regulations, and with the approval of the animal care and use committees at Uppsala University, SBP and at the University of California San Diego (UCSD).

### Doxycycline treatments and irradiation experiments

Doxycycline (dox) was delivered ad libitum to the mice *in vivo* via a dox rich (625 mg/kg) diet (Harlan Laboratories/Envigo). For irradiation in vivo we treated mice 7 days after injection of 100,000 GTML brain tumor cells transplanted into the cerebellum with 36 Gy irradiation over 3.5 weeks for a total of 16 days (excluding weekends). Irradiation was performed with the Small Animal Radiation Research Platform (SARRP) by Xstrahl (Surrey, UK) at the Preclinical Cancer Treatment Center, SciLifeLab, Uppsala University. We irradiated two thirds of the brain covering the entire hindbrain region (transplantation site) and posterior forebrain region but not the anterior forebrain with the olfactory bulb. Cone beam computer tomography performed by the SARRP was used to set the isocenter for dose delivery to the brain. A total dose of 2 Gy per day was delivered with 2 parallel opposed beams of 1 Gy each (10x10 mm collimator) by the SARRP using 220 kVp, 13 mA photons with a broad focal spot. Mice were sacrificed when they reached the humane endpoint (score ≥0.3 according to the Uppsala University form "Extended assessment health conditions of small rodents and rabbits".

### Cell Lines

Tumor cells (GTML, GTS and preGTS) were isolated as previously described from tumor bearing mice (Cancer Cell 2012, 21: 601-613) and cultured as neurospheres in serum-free conditions with Neurobasal (NB) media, additional B27 supplement, 100 units Penicillin and 0.1 mg/ml Streptomycin, 2 mM L-glutamine, 1:500 Antibiotic-Antimycotic, 20 ng/ml EGF and 20 ng/ml FGF-2. Human MB002 primary cell culture was obtained from Dr. Yoon Jae-Cho and cultured in serum-free conditions, 1:1 of Neurobasal Media (-vitamin A) and DMEM/F12 with Sodium Pyruvate, NEAA, HEPES, GlutaMAX, 100 units Penicillin and 0.1 mg/ml Streptomycin, B27 supplement, 20 ng/ml EGF and 20 ng/ml FGF-2, 1X LIF and 1X Heparin as previously described Clin Cancer Res 2014, 20: 912-925. Stable doxycycline-inducible SOX9wt and SOX9CPD mutant cell lines were generated as described (EMBO J 2016, 35: 2192-2212), lentiviruses were first packed in HEK293T cells by the specified pTRIPZ lentivector with the packaging plasmids, psPAX2 and pMD2.G. Supernatants containing viral particles (48 - 96 hours post transfection) were used to infect the target cells in the presence of freshly added hexadimethrine bromide (8 mg/mL). Seventy-two hours post-transfection, the cells were selected and maintained in appropriate culture medium containing puromycin (0.125 - 0.5 µg/mL).

### Flow cytometry and cell sorting

Tumor burdened GTML;SOX9-eGFP mice were sacrificed and tumors dissociated, cells cultured as we described above. Cells were harvested, accutased and filtered with 30 µm cell strainer, then sorted for GFP-positive (SOX9) cells (488_FITC-A) and GFP-negative cell population. For cisplatin treatment experiment, cells were treated with cisplatin 24 h before cell sorting. The BD FACSMelody was used for sorting and quantifying in this experiment. MB002-pSOX9-GFP cells were dissociated using accutase and sorted for GFP expression (488_FITC-A). The BD FACSarialll was used for this experiment and it was analyzed using BD FACSDiVa version 8.0.

### Immunohistochemistry

Brains were fixed in 4 % PFA and embedded in paraffin block. preGT20 sphere cultures were fixed in 4 % PFA for a minimum of 12 hours at 4°C and then embedded in HistoGel followed by paraffin embedding. 6-7 µm sections of paraffin-embedded tissue were used for all staining. The complete protocol for the IHC was described previously (Genes & development 2010, 24: 1059-1072).

### Immunofluorescence

Mouse brains were isolated, dehydrated and embedded in paraffin. 6 µm brain sections were deparaffinized, hydrated and antigen retrieved. Sections were blocked in 10 % donkey serum with 1 % BSA for 1 hour and then incubated with primary antibodies overnight at 4°C, followed by washing and secondary antibody for 1 hour protected from light. After washing nuclei were visualized with DAPI (5 mg/ml) for 5 min. Finally, sections were washed and mounted with Fluoromount. Images were taken using Leica DMi8 fluorescent microscope.

### Lentiviral constructs

pSOX9-Luc/GFP was generated by replacing the CMV promoter of pBMN(CMV-copGFP-Luc2-Puro) with the human SOX9 promoter.

### RNAscope^{®}

RNAscope^{®} Technology is a cutting-edge *in situ* hybridization (ISH) technology based on Advanced Cell Diagnostics (ACD) unique, patented probe design strategy that enables simultaneous signal amplification and background noise suppression (www.acdbio.com). RNA scope was performed according to manufacturer's instructions (Advanced Cell Diagnostics, CA). We used specific probes detecting mouse SOX9 (ACD-401051-C2) and human MYCN (ACD-417501). The detection was performed on 6 µm sections from paraffin embedded brain tissues. Sections were deparaffinized and processed according to manufacturer's instructions. For our staining of MYCN and SOX9 we used RNAscope-Fluorescent Multiplex Detection Reagents (ACD-320821), Pre-treatment Kit-FLFF (ACD-320842), RNA Scope Wash Buffer (ACD-310091) and 10× Pre-treat (ACD-320043). We used recommended HybEZ oven to incubate the samples in the humidity control tray at 40°C.

### Western Blot

20 µg protein was loaded in 4-12 % Bis-Tris gels and transferred to iBlot nitrocellulose membrane. ECL secondary antibodies (1:5 000) were detected using Supersignal West Pico Chemiluminescent substrate.

### Doxycycline treatments

For *in vitro* experiments, doxycycline hyclate (dox) (Sigma) was dissolved in PBS and added to culturing medium (1 µg/ml).

### Doxycycline induction or SOX9 in MB002-SOX9CPDmt

1 µg/ml of Doxycyline was added to MB002-SOX9CPDmt in culture media. For stable SOX9 overexpression, new dox was added every 48 h. For recovery experiments, cells were cultured in dox conditioned media for 24 h after which they were spun down and resuspended in fresh media for continued cell culturing in normal conditions and harvested 8, 24, 48 and 72 h after dox removal. Cell pellet was lysed and analyzed for protein content using WB.

### Drug treatment and Proliferation assay

To assess cell proliferation, 10 000 cells were plated in triplicates in 96-well plate. Proliferation was measured every 24 h for five days using Resazurin colorimetric assay. The MGMT inhibitor Lomeguatrib was dissolved in DMSO and added to cells 24 h post cell plating to a final concentration of 20 µM. ThioTEPA was dissolved in DMSO and added similarly to a final concentration of 10-100 µM. Doxorubicin was dissolved in H₂O and added to a final concentration of 100-120 nM. Spheres from pre-GT20 cells were treated with Cisplatin at a final concentration of 10 µM. Number of SOX9-positive cells per spheres was counted in 10 spheres in treated (Cisplatin) and untreated (DMSO) conditions. Statistical significance was calculated using a unpaired non-parametric Mann-Whitney test.

### Long term proliferation with dox treatment

GTML and preGTS cells were seeded in triplicates of 10 000 cells per well in polyornithine and laminin coated 96 well plates. 24 h post seeding (0 h), dox was added to cells (final concentration 1 µg/ml). 72 h after first dose of dox, a second dose was added in fresh media. DMSO was used as control. Fresh media was given to cells at day 7 and 10 of the experiment. Every 48 h proliferation was measured using a resazurin based assay.

### CRISPR-CAS9 targeting SOX9

CRISPR-Cas9 gene editing of SOX9 was performed using Integrated DNA Technologies (IDTs) Alt-R^{®} CRISPR-Cas9 system and protocol (Alt-R^{®} CRISPR-Cas9 System: Delivery of ribonucleoprotein complexes into Jurkat T cells using the Neon^{®} Transfection System). Three individual crRNAs targeting SOX9 were selected:
- GUUCACCGAUGUCCACGUCGGUUUUAGAGCUAUGCU (SEQ ID NO: 5);
- ACCAUGUCGGAGGACUCGGCGUUUUAGAGCUAUGCU (SEQ ID NO: 6);
- GAAGGGCUACGACUGGACGCGUUUUAGAGCUAUGCU (SEQ ID NO: 7)

These were combined with ITDs tracrRNA ATTO^{™} 550. pre-GT20 cells (5×10⁵) were electroporated using the Neon^{®} Transfection System 10 µL kit (setting 7; pulse voltage: 1200, pulse width: 30, number of pulses: 1), with a combination of the three SOX9 targeting crRNAs, all at a concentration of 1.8 µM, to excise a segment of the SOX9 gene, seeded as single cells in 96 well plates and visually inspected for fluorescent labeling. Single positive cells were expanded to form monoclonal cell lines. pre-GT20 cells were electroporated resuspended in Buffer R only and then expanded from single cells to create control cell lines. SOXP9 gene editing was validated using PCR (forward primer CCGGCCCCAGGAGAACACCTT (SEQ ID NO: 8), revers primer GTGAGTGCAGCCCGCGTCCC (SEQ ID NO: 9)) and protein levels were investigated using western blot (ab185966). Cell line identity was confirmed using PCR targeting the SOX9-rtTA construct specific to the GTS mouse strain.

### RT-qPCR

RNA was isolated using Trizol and RNeasy Mini Kit. RNA concentration was measured with Qubit RNA BR Assay Kit. cDNA synthesis was done using SuperScript VILO cDNA Synthesis Kit. qPCR experiments were run in triplicates with 2.5 ng RNA using SYBR Green PCR Master Mix or PrimePCR (Bio-Rad Laboratories) SYBR Green Assay on StepOnePlus Real-Time PCR System or CFX96 TouchTM Real-Time PCR Detection System. Primer sequences for mouse GTML cells for SOX9, MYCN and Gapdh have been used previously (Cancer Cell 2012, 21: 601-613). Human primers can be found in Table 1. The data was analyzed with ΔΔCt method *(*Methods 2001, 25: 402-408).

**Table 1 - Primers**

| **Primer name** | **Primer sequence (5' → 3')** |
|---|---|
| SOX9 (human) | TCAACGGCTCCAGCAAGAACAAG (SEQ ID NO: 10) |
| | ACTTGTAATCCGGGTGGTCCTTCT (SEQ ID NO: 11) |

| **PrimePCR primer** | **Bio-Rad** |
|---|---|
| cMYC (human) | Seq N/A cat# qHsaCID0012921 |
| GAPDH (human) | Seq N/A cat# qHsaCED0038674 |

### Cytokine array

Cell lysates (200 µg) and supernatants (2×10⁶ cells 48 h in culture) were analyzed using Proteome Profiler Mouse XL Cytokine Array and instructions were followed accordingly. ECL secondary antibodies (1:5000) (GE healthcare) were detected using SuperSignal^{™} West Femto Maximum Sensitivity Substrate. Results were analyzed with ImageJ software and negative controls were used as background. The experiment was repeated once and figures show representative images.

### Chase assay

MB002-SOX9CPDmt and EV cells were treated with Doxycycline (1 µg/ml) or DMSO for 4 hours after which Cycloheximide (100 µg/ml) was added. Cells were harvested at the indicated time points and analyzed with WB for cMYC.

### Single cell RNA-Seq experiments

For single-cell library preparation on the 10× Genomics platform, we used: the Chromium Single Cell 3' Library & Gel Bead Kit v3 (PN-1000128), Chromium Single Cell 3' Chip kit v2 (PN-1000127) and Single Index Kit T Set A (PN-1000213), according to the manufacturer's instructions in the Chromium Single Cell 3' Reagents Kits V3.1 User Guide. Just prior to cell capture, GTML;SOX9-eGFP and fGT21 cell lines were harvested, accutased and filtered with 30 µm cell strainer, then spun at 300×g for 5 minutes followed by resuspension in PBS with 0.04 % BSA on ice. 17 000 cells were loaded per lane of the chip, aiming for capture of 10 000 single-cell transcriptomes. All samples were processed in parallel, on the same day. Resulting cDNA libraries were quantified on an Agilent TapeStation and sequenced on an Illumina NovoSeq 6000.

### ATAC-seq experiments

ATAC-seq with in-houseTn5 in GFP+ cells and GFP- cells from GTML;SOX9-eGFP and GTS cells was performed as described (Nat Methods 2016, 13: 1013-1020). Briefly, 50 000 cells were centrifuged 500×g 5 min at room temperature. The cell pellet was resuspended in 50 µl lysis buffer (10 mM Tris-Cl, pH 7.4, 10 mM NaCl, 3 mM MgCl₂, 0.01 % Igepal CA-630) and centrifuged immediately 500×g for 10 min at 4°C. The cell pellet was resuspended in 50 µl transposase mixture (25 µl 2X TD buffer (20 mM Tris, 10 mM MgCl₂, 20 % dimethylformamide), 22.5 µl dH₂O and 100 nM in-houseTn5) and incubated at 37°C 30 min. After transposition, the mixture was purified with Qiagen Mini-purification kit and eluted in 10 µl Qiagen EB elution buffer. Sequencing libraries were prepared following the original ATAC-seq protocol. The sequencing was performed on Illumina NovaSeq at the Novogene Europe.

### ATAC-seq data analysis

ATAC-seq paired-end reads were trimmed for Illumina adapter sequences and transposase sequences using an in-house script and mapped to hg19 using Bowtie2 (Genome Biol 2009, 10: R25) v2.1.0 with parameters -very sensitive. Over ~11 million mapped reads were generated in each sequencing library and used for downstream data mining. Duplicate reads were removed with Picard (http://picard.sourceforge.net) v1.79. Peak calling was performed by MAC21 (Genome Biol 2008, 9: R137) narrow peak mode with parameters -q 0.01 -nomodel -shift 0. Overlapping peaks from all samples were merged together to a consensus peak list, and number of unique and properly paired reads mapped to each peak for each individual samples was quantified to calculate the Pearson Correlation. The insert size of fragments was estimated from the distance between the pair-ended reads, and plotted against the frequency in a histogram. In order to identify genes with regions of differentially opened or closed chromatin, respectively, we performed differential analyses (via the edgeR package) of ATAC-seq peaks between conditions. Subsequently, peaks were mapped to the closest gene within 20 kB and up to 2 000 genes with the closest associated peaks were selected for downstream gene-set overlap analyses.

### Single cell RNA-seq data analysis

The Cell Ranger v3.3.0 pipeline was used to process data generated using the 10× Chromium platform. This aligns sequencing reads to the mouse reference genome mm10 v3.0.0. Following alignment, gene expression matrices were generated. Using Seurat v3.0 package in R, We first removed cells with a low number (<200) of unique detected genes and genes (<3) with low number of cells, then we removed the cells unique detected genes less than 500 or great than 7 000, finally, we removed cells in which the proportion of the UMI count attributable to mitochondrial genes was greater than 20 %. Umap of SOX9/KI67 and cycle cell analysis in GTML,SOX9-GFP and GTS cells were generated using Seurat package.

### DNA methylation profiling

DNA methylation profiling was conducted on the Illumina MethylationEPIC platform and was performed by the Uppsala SNP&SEQ Technology Platform in Uppsala (www.genotyping.se). Preprocessing of raw IDAT files was conducted in R using the MethPed (1.6.0), IlluminaHumanMethylationEPICanno.ilm10b2.hg19 (0.6.0), and IlluminaHumanMethylationEPICmanifest (0.3.0) packages. Probes were removed, if any sample had an associated p-value p>0.01. Average beta values were extracted after normalization on red-green channels using the preprocessNoob scheme. Average Beta after normalization with the preprocessNoob function from the minfi (version 1.24.0).

### Sequencing data preparation

Total RNA was sequenced using the IonProton^{™} System in the Uppsala Genome Center, SciLifeLab, Uppsala University. For the dox treated GTML and preGTS cells, raw sequencing reads were mapped to the mm9 genome assembly according to facility standards, and the according gene specific read counts were used directly. For GTS and GTML tumors, we mapped raw sequencing reads to the mm9 mouse genome using a two-round approach of the STAR alignment algorithm, followed by a subsequent alignment of unmapped reads using BOWTIE2 in local mode. Gene specific read counts were then obtained via the featureCounts function from the Subread package.

### Differential Expression Analysis

Differential gene expression analyses were conducted in R using the edgeR package, including a normalisation for differences between raw library sizes via the calcNormFactors function in conjunction with the TMM method. GTS and GTML tumors samples were processed in two mixed batches and displayed batch-related biases. To account for those batch-effects, differential expression analyses between GTS and GTML tumors were conducted by including the batch as a factor in the edgeR design matrix. Genes were considered significantly differentially expressed, if their FDR corrected p-value q<0.05.

### Removal of batch effects

While differential expression analyses were conducted by including batches as a factor in the design matrix, various types of data visualization required a removal of batch effects among the GTML and GTS dataset prior to figure plotting. For this purpose, we applied the removeBatchEffect function from the R package limma.

### Mapping of orthologs and translation of human gene symbols

All translation between different identifiers within the same species or between ortholog genes in mouse and human were conducted via the biomaRt package in R. Specifically, where necessary, Ensembl gene ids obtained after mapping to the Gencode mm9 annotation track were mapped to official mouse gene symbols. Mouse gene symbols (GTML and preGTS cells) and Ensembl gene ids (GTML and GTS tumors) were further mapped to their human orthologs and translated to HGNC symbols in order to facilitate cross-species analyses and GSEA.

### Gene set enrichment analyses

All gene set enrichment analyses (GSEAs) were performed in the GenePattern (https://genepattern.broadinstitute.org/) platform and using the GSEApreranked method. Specifically, for every comparison, genes were scored as S=-sign(logFC)*log10(p-value), where the logFC and p-value were obtained from the respective edgeR differential expression analysis, and the score was used as the gene ranks. Subsequently, the ranked gene lists were analyzed using the GSEApreranked framework on seven different gene set databases (H: hallmark gene sets, CGP: chemical and genetic perturbations, CP:KEGG: KEGG gene sets, CP:REACTOME: Reactome gene sets, TFT: transcription factor targets, BP: GO biological process, C6: oncogenic signatures) and using the classical scoring scheme. Gene sets were considered to be significantly enriched, if their associated FDR corrected p-value q<0.05.

### MB Subtype classification

The possible MB subtype affiliation of the mouse samples was estimated using the Metagene code for cross-platform, cross-species projection of transcription profiles (Proc Natl Acad Sci U S A 2007, 104: 5959-5964). As a MB reference set, we merged three gene expression data sets, i.e., 763 samples from Cancer Cell 2017, 31, 737-754.e736, 123 from CBTTC: BMC Genomics 2016, 17 Suppl 4: 434, and 170 from Nature 2017, 547: 311-317. The merged data was batch-normalized using ComBat (Biostatistics 2007, 8: 118-127) and samples that could not be robustly classified into their annotated subgroup were discarded, resulting in a total of 997 MB samples. Using the metagene software, GTML and GTS expression profiles were then projected onto the MB specific metagene space, and subsequently classified via a support vector machine and processed via a principle component analysis. To confirm the resulting classification, we simultaneously also projected a set of 20 MB PDX samples (Cell Stem Cell 2019, 25: 855-870 e811) onto the same MB data set and processed them alongside the GTML and GTS samples.

### Differential expression and survival analysis in MB samples

In order to compare the expression of SOX9 and SOX2 between primary and metastatic MB tissues, we used expression data of 22 samples from paired primary and metastatic compartments comprising six patients classified as Group 4, two patients classified as Group 3 and one patient classified as SHH (Acta Neuropathol 2015, 129: 449-457). The processed expression of both the SOX9 and SOX2 genes were downloaded from the R2: Genomics Analysis and Visualization Platform (http://r2.amc.nl). For patients with multiple metastatic compartments linked to the same primary tumor, the mean value over all metastatic compartments was used to represent the corresponding metastatic expression. The significance of differences between primary and metastatic expression means was calculated using the paired Student's t-test. We also used the Delattre set in R2 (https://r2.amc.nl) containing 57 samples analyzed using the Human Genome U133 Plus 2.0 Array (u133p2) arrays, but excluded typical nodular/desmoplastic SHH tumors as well as apparent Beta-catenin mutated WNT tumors from the analysis when comparing primary to post-treated samples. When calculating expression differences between different groups they were considered significantly different when p < 0.05, as calculated using the one way analysis of variance (ANOVA) test included in the R2 online software.

For survival analyses in Kaplan Meier plots we used a 10 year/120 month time point as a cut off for survival due to the potential risk to include secondary tumors and further excluded nodular/desmoplastic MB samples from the Group 3 and Group 4 analysis from the Cavalli set in order to not risk including tumors from typically desmoplastic SHH MBs (Cancer Cell 2017, 31: 737-754.e736). The p-value for survival curves is calculated using a LogRank test of reported patient survival data as described in R2 (https://r2.amc.nl). Only a-priori fixed cut offs (median, average, first or last quartiles) were used to calculated p-values in the Kaplan-Meyer survival analysis.

### Animal Survival Statistics

All animal experiments were conducted once. Animal survival was graphically shown as a Kaplan Meier curve, made and assessed using GraphPad Prism 7 software. The p-value for survival curves is calculated using a LogRank test.

### Immunohistochemistry and Immunofluorescence Quantification

All micrographs shown are representative images of the respective mouse strain tumors. Where appropriate for statistical analysis and immunohistochemical quantification, at least three representative micrographs from at least three individual tumors were analyzed in Fiji ImageJ. Positivity of staining was expressed as a % of total cell population.

### Quantification of Metastatic Dissemination

Quantification of metastatic dissemination was assessed via histological examination of H&E-stained transverse sections of spinal cord. Each spinal cord was divided into several smaller, transverse regions prior to being processed, and multiple sections were taken per spinal cord. Metastatic dissemination was considered either positive or negative - where positivity was determined as a clear aggregate of 10 or more tumor cells. Numbers of analyzed animals are shown in the respective figures.

### Reporting of p Values, Sample Size and Statistical Significance

Significant p values are reported for appropriate figures either in the figure legend or at the respective figure panel. Sample size (n) can be found in the figure legend or in the respective figure panel. Error bars represent the standard error of mean.

### Randomization and Inclusion/Exclusion Criteria

Animals included in the allograft studies had no inclusion/exclusion criteria prior to being stratified into treatment or vehicle groups. After sacrifice, presence of physical tumor was confirmed by pathological examination and H&E staining in brain or spinal cord. Animals that were found dead without obvious signs of tumor (primary tumor penetrance studies) were marked as censored. No other data was excluded from analysis and reporting in this study. No blinding was carried out at any stage of the study.

### Results

### SOX9 is upregulated by MYCN loss and is induced in therapy-resistant dormant cells

We first characterized how SOX9 mRNA levels changed following MYCN depletion, using dox treatment of tumor cells derived from our TetOFF-inducible Group 3 MB model (GTML) where the Glt1 promoter drives MYCN and Luciferase via tTA binding to their Tet response element (TRE). MYCN expression levels were suppressed by the TetOFF system and SOX9 expression levels increased more than fourfold after 48 h of dox treatment of GTML cells *in vitro* (Figs. 13A and 20A). We next explored the SOX9 protein levels in the GTML model *in vivo.* SOX9 is normally expressed in glial cells, NSCs, ependymal cells and also show scattered expression in rare cells within the external granular layer (EGL) in the developing cerebellum. Using a transgene where the SOX9 promoter drives GFP expression (pSOX9-GFP), corresponding to SOX9 protein expression in the developing cerebellum (Figs. 20B and 20C), we found 2-5 % of GFP-positive tumor cells in established tumor lines isolated from crosses of GTML and pSOX9-GFP mice (GTML-pSOX9-GFP) (Fig. 20D). Consistently, 2-3 % of cells in tumor biopsies of GTML tumors stained positive for SOX9 (Fig. 13B, upper panel). To see if SOX9-positive tumor cells survive standard therapy better than SOX9-negative cells, we treated GTML-pSOX9-GFP cells with cisplatin and found a significant increase from 2.4% to 8.6% in SOX9-positive cells after 24 h treatment (Fig. 13C).

Dox treatment is known to kill MYCN-driven cells in the GTML model, and long-term dox treatment (30 days) can cure GTML mice with brain tumors. We observed an increased number of SOX9-positive cells in spontaneously developed GTML tumors already after 6 hours of dox treatment (Fig. 13B). Regardless of dox treatment, SOX9 and the proliferation marker Ki67 rarely co-stained in GTML tumors, whereas SOX9-negative tumor cells were significantly more proliferative, suggesting that most (about 90 %) SOX9-positive cells are quiescent or slow-cycling cells in these tumors (Figs. 13B and 20E). In order to study if SOX9-positive cells are more quiescent we employed ATAC-Seq and single cell (sc)RNA-Seq analysis of GTML-pSOX9-GFP tumor cells sorted for GFP. GFP-positive cells showed a significantly open chromatin at the SOX9 promoter region as compared to GFP-negative cells (Fig. 13D). GFP-positive cells correlated with a quiescent molecular signature of neural stem cells as compared to GFP-positive cells that had a more proliferative profile as shown using single samples GSEA against quiescent and actively dividing neural stem cells (Fig. 13E). Further, scRNA-Seq data of GTML tumor cells suggested that high expression of SOX9 or MYCN was almost entirely mutually exclusive in these cells (Figs. 13F and 13G), with the SOX9 positive cells displaying a significantly more quiescent signature (Fig. 13H) correlating with non-dividing cells residing in the G0/G1 cell cycle phase (Fig. 20G).

As quiescent SOX2 positive cells are known to drive hierarchical growth and relapse in Ptch-deficient mouse SHH tumors, we studied SOX9 levels in a number of these tumors. In contrast to our Group 3 MB mouse model, almost all Ptch-deficient tumor cells were strongly positive for SOX9 (Fig. 20F).

### SOX9 increases in residual disease models and marks quiescent cells in Group 3 MB patients

To see if SOX9-positive cells are increased following dox treatment and might be responsible for regrowth of suppressed tumor we treated one GTML line (GTML2) with very low SOX9 levels and one (GTML3) with higher SOX9 levels *in vivo* (Fig. 14A). Following 5 days of dox treatment *in vitro* very few cells survived in the GTML2 line but in GTML3, a small number of non-proliferative viable tumor cells were found that we previously showed had higher levels of SOX9 (data not shown). Still, cells surviving 5 days of dox therapy did not recover and could not recur following 2 weeks *in vitro* (data not shown). We instead tested if transplanted GTML tumor cells could mimic disease recurrence *in vivo.* MYCN depletion with 7 days of dox treatment was not enough to cure the mice, but it significantly extended the overall survival to 16.5 weeks for GTML2 but only to 9 weeks for GTML3 cells (Fig. 14B). In both lines the number of SOX9-positive tumor cells increased after treatment (at minimal residual disease). Intriguingly, in regrown GTML2 tumors the population of SOX9-positive cells contributed to a significantly larger fraction of SOX9-positive cells as compared to primary tumors (Fig. 14C). The data suggest that SOX9-positive cells that survived the MYCN-suppressive therapy accumulate *in vivo.*

We next checked the plasticity and potential hierarchy of SOX9-positive tumor cells. Single cell sorting of SOX9-positive (GFP+) cells followed by propagation and expansion for two weeks gave rise to SOX9-negative (GFP-) cells and vice versa, that SOX9-positive cells gave rise to cells lacking GFP (data not shown). In order to follow how fast SOX9-positive cells give rise to SOX9-negative cells we sorted cells for GFP and cultured them in normal stem cell conditions. Already two days after cell sorting the number of SOX9-positive cells dropped to 70 %, and a week later to 50 % (Fig. 14D). Some cells (Intermediate) were still SOX9-positive but showed less GFP intensity than SOX9-positive cells that were clearly positive. This suggests that SOX9-positive cells indeed give rise to SOX9-negative cells. SOX9-negative cells were further shown to generate 2 % SOX9-positive cells after a week in culture, highlighting the plasticity of the tumor cell population. In summary, our data suggests that SOX9-positive tumor cells are less proliferative, more treatment resistant, better survive MYCN-suppressing therapies and that they accumulate during *in vivo* relapse.

In order to study if irradiation causes brain tumor escape and relapse after long-term irradiation in vivo we treated mice with GTML2 brain tumor cells transplanted into the hindbrain with 36 Gy irradiation over 3.5 weeks. We intentionally irradiated two thirds of the brain covering the transplantation site in the hindbrain region but not the anterior forebrain. Scars from irradiated tumors at injection site showed efficient tumor targeting from radiation (not shown), but tumor cells that migrated to the forebrain and quickly relapsed in the olfactory bulb 5 days after treatment had low SOX9 levels (like untreated controls), while the tumor cells that more slowly returned after almost 2 weeks of treatment had higher SOX9 levels (Fig. 14E). The latter scenario with SOX9 accumulation suggest the presence of temporarily quiescent and therapy resistant tumor cells, which more closely correlate to a true relapse seen in pediatric brain tumor patients as compared to the former fast "relapse" including cells that just expanded their growth in the mouse brain. This also confirms that the radiotherapy used in the clinic is indeed efficient but that tumor cells that relapse found ways to escape treatment.

To bridge our preclinical findings, we next wanted to check if we could validate our findings in human MB patient materials. We first compared the expression of SOX9 in MYC/MYCN amplified cases as compared to MYC/MYCN balanced cases in a cohort of 64 human MB samples (Fig. 21A, top). The expression of SOX9 was significantly lower when MYC genes were amplified as compared to when they were balanced. This correlation was also significant when comparing SOX9 to MYC expression in a cohort of 470 MB Group 3 and Group 4 samples (Fig. 21A, bottom). We previously examined the connection between SOX9 and MYCN in a mouse model of retrovirally-induced MB and found that MYCN transduction suppresses SOX9 protein levels in NSCs. SOX9 expression is also significantly suppressed in neuroblastoma, childhood peripheral nervous system tumors, when MYCN is transiently induced (Fig. 21B). The analysis suggests that the negative correlation between MYCN and SOX9, which we have described in the mouse model, is also true in patients. When using a larger set of patient samples of Group 3 tumors, elevated SOX9 mRNA levels further correlated with poor survival as compared to elevated SOX2 levels that did not correlate with poor survival in this subgroup (Figs. 14F and 21C). Neither SOX9 nor SOX2 levels correlated significantly with poor survival in Group 4 samples (Fig. 21D). When analyzing the overlap of SOX9 and SOX2 expression in GTML tumor sections by ICC co-staining, SOX9 is found in SOX2 positive cells (Fig. 21E). However, SOX9 expression is restricted to fewer cells as compared to SOX2 staining that is active in relatively more cells in the tumor area. We finally tested if we could follow the ratio of SOX9 and MYC also in scRNA-Seq data of human MB samples. We found that the number of SOX9-positive cells were rare in human Group 3 MB biopsies as compared to MYC-positive cells that constituted a large portion of these tumors (Fig. 14G). When performing a single sample GSEA of the SOX9-positive and MYC-positive populations it was further clear that SOX9-positive cells in patient samples presented a significant more quiescent signature than the MYC-positive cells (Fig. 14H).

### SOX9 accumulates in metastatic disease and in relapses of paired primary-recurrent MB

To see if SOX9 contributes to recurrence in individual cases, we matched primary-recurrent patient biopsies from MBs of all four subgroups. SOX9-positive cells were significantly enriched in recurrences of both Group 3 and Group 4 samples (Figs. 15A and 15B). However, there was no significant difference in SOX9 levels in SHH tumor pairs and too few matched WNT samples with recurrences for us to draw any conclusions (Fig. 15B). The data suggests that the number of SOX9-positive cells is increasing in Group 3 and Group 4 tumors from primary diagnosis to acquired recurrences.

SOX9 is regulated post-translationally by FBXW7, can promote metastatic spread, and SOX9 protein levels are significantly upregulated in M3 MB samples (i.e., macroscopic metastatic disease with spinal cord dissemination). Group 3 MBs with M0-M1 status has a significantly better prognosis than Group 3 patients with M2-M3 status (macroscopic metastatic disease). When comparing quantified SOX9-positive cells in our previous dataset of primary Group 3 biopsies (with M0-M1 or M3 status) and recurrent Group 3 biopsies, there is a significant increase in SOX9-positive cells in both primary Group 3 tumors with M3 status and recurrent Group 3 tumors, as compared to primary M0-M1 cases (Fig. 15C). There is a positive trend but no significant accumulation of SOX9-positive cells in recurrent samples of Group 4 patients when comparing primary and recurrent groups. However, M status is a strong predictor of poor prognosis for Group 4 tumors, and we found a significant increase of SOX9-positive cells in M2-M3 Group 4 tumors as compared to M0-M1 Group 4 tumors (Fig. 22A).

Additionally, we checked for SOX9 mRNA levels in a set of paired primary/metastatic human MB samples. We found that SOX9 was elevated in some metastases from Group 3 pairs and was significantly higher in the metastasis of Group 4 pairs (Fig. 22B). In comparison, SOX2 levels were not elevated in metastasis of these subtypes (Fig. 22C). Although SOX9 and SOX2 are in the same family of SOX transcription factors, their expression levels correlate much better in SHH tumors as compared to Group 3 and Group 4 tumors (Fig. 22D) suggesting that these related factors have different roles in various contexts. The results albeit suggest that SOX9 is a good marker for poor prognosis and for recurrent tumors in Group 3 patients and for metastatic cells and recurrence in Group 4 patients.

### The recurrence process is reproduced in a novel TetON/TetOFF transgenic GTS model

MYCN-induced tumors that develop in the GTML model completely regress *in vivo* when dox is injected or if dox is included in the mouse diet for 30 days. In order to specifically study and target SOX9-specific cell populations within the tumor we employed a Sox9-rtTA transgene driving reverse tTA (TetON) from the SOX9 promoter (Fig. 16A). To clarify the role of SOX9 in MB initiation, we first studied if MYCN can induce tumors from SOX9-positive cells. We crossed mice carrying Sox9-rtTA with mice carrying TML (STML) and thus created a TetON system where expression of MYCN and Luc is activated in SOX9 expressing cells upon dox administration. We followed 56 mice over 6 months with different starting points of dox administration. 40 mice were given dox chow starting from embryonal day 0 (dox diet given during pregnancy), 10 mice from P0 (dox transferred to pups during breastfeeding) and 6 mice from P21 and were followed for 6 months or longer. None of these STML mice that were put on dox diet developed any tumors (Fig. 23A), suggesting that SOX9-positive cells are likely not the cells of origin for these MYCN-driven tumors.

We then crossed the GTML (TetOFF) model with the Sox9 (TetON) model and generated a dual Tet system in a triple transgenic Glt1-tTA; TML; Sox9-rtTA (GTS) model. In the absence of dox, GTS mice are identical to GTML mice as the rtTA molecules produced under the control of SOX9 promotor are inactive (Fig. 16A). When 25 GTML mice that developed large tumors were given a dox diet for 30 days, these tumors were completely eradicated (Fig. 16B). The 24 GTS mice that developed similarly large primary tumors were put on a 30 day dox diet following the same procedure that successfully cured GTML mice. At first, these GTS mice were healthy and lacked or presented very low levels of luciferase signal (Fig. 23B). However, 45 up to 120 days after dox removal, all of the mice (n=24) developed recurrences (Fig. 16B). Surprisingly, tumors in GTS mice were mainly localized to the forebrain, whereas tumors in GTML mice displayed a dominant localization to the hindbrain region (Figs. 16C and 16D), as we previously observed. Recurrent tumors showed no significant differences in apoptosis or cell proliferation (Fig. 23C). In almost half of the GTS mice tumor spread to the spinal cord was observed (Figs. 16D and 16E). The metastatic rate in GTS mice was significantly increased, consistent with what is observed in patients during recurrences, compared to primary GTML tumor development where dissemination to the spinal cord rarely occurred. When we investigated this further, all GTS forebrain recurrences (fGTS) had spinal metastases while recurrences located in the hindbrain (hGTS) had none (Fig. 16F). GTML and GTS metastases had unarguably more SOX9-positive cells as compared to their corresponding relapses (Fig. 16G).

We wanted to further validate these finding in human MB. Both SHH and Group 3 MB PDXs that were stereotactically injected into the cerebellum had significantly elevated SOX9 levels in spinal cord metastases as compared to corresponding intracranial primary tumors (Fig. 16H). Interestingly, cells in spinal metastases from Group 3 MB were significantly less proliferative than cells in the respective primary tumor, again suggesting increased SOX9 levels might suppress proliferation in these cells as well (Fig. 16I). In SHH MB, there was no significant difference (Fig. 16H). The same pattern was observed in our GTS model. Typically, recurrent tumors stained strongly for Ki67 compared to remnant primary tumors that silently remained in the hindbrain regions in animals months after sufficiently curative 30-day dox treatment, as previously also described in the GTML model (Fig. 23D). The histopathology of the recurrent tumors was still not different from the primary GTML tumors and was similar to the large cell anaplastic or classic MB (data not shown). Taken together, the pathology, location and dissemination of recurrent tumors, induced using the GTS system, resemble that of relapsed human MB.

### Recurrent MBs molecularly resemble primary MBs but are more inflammatory

To analyze the molecular differences between primary and recurrent tumors we performed RNA-Seq analysis. Using the transcriptional profiles, we started with a cross species comparison against various types of brain tumors, demonstrating a clear affiliation of both GTML and GTS tumors with MB (Fig. 24A). Subsequently, we employed a cross-species analysis against MB patient data in order to further establish subgroup affiliations for the tumor groups. The recurrent GTS tumors (n=6) clustered in close proximity to the primary GTML tumors (n=8), and both were clearly aligned with Group 3 MB (Fig. 17A). Consistent with these observations, a one-to-all support vector machine (SVM) classification and heat map inspection (data not shown) revealed that all GTML and GTS tumors affiliated with Group 3 human MB.

Despite both tumor models affiliating with Group 3 MB, a differential gene expression analysis suggested substantial transcriptional differences between them, with 698 genes significantly upregulated in GTS and 165 genes significantly downregulated in GTS as compared to GTML. In order to investigate overall transcriptional differences between the primary and recurrent tumors we next compared their expression profiles through pre-ranked Gene Set Enrichment Analyses (GSEAs). Key differences between the two tumor classes were significant enrichment of gene sets associated with MYC pathway, cell cycle and DNA repair mechanisms in the GTML; and a significant enrichment of gene sets associated with inflammatory responses, JAK/STAT and NfKB signaling in GTS (Fig. 17B). The difference in mRNA levels of MYCN and SOX9 between GTML and GTS tumors was not dramatically altered. Still, MYCN was specifically repressed in forebrain (fGTS) tumors and there was an increase in SOX9 mRNA levels in GTS tumors regardless of their location (Fig. 24B). Typical immune escape markers like Galectin 9 (LGALS9) and PD-L1 (CD274) were upregulated significantly in fGTS. Further, proteins implicated in extravasation and dissemination, like CCL2 and CCR2, were found to be upregulated in fGTS as well as hGTS recurrences (Figs. 24B and 24C). In line with the increased migratory behavior of the GTS tumor cells, we also found that genes with both increased ATAC-Seq signals and upregulated RNA-seq expression in GTS tumors significantly overlapped with a cell locomotion (GO_LOCOMOTION) related gene set (Fig. 17C).

Among the GSEA results, we identified upregulated gene sets of cytokine pathway activation, e.g., KEGG_CYTOKINE_CYTOKINE_RECEPTOR_INTERACTION, in where 20 out of 267 genes were upregulated in GTS tumors as compared to GTML tumors (Fig. 24C). Accordingly, as transcriptional changes appeared to affect many cytokine receptors or ligands, we used an array with more than 100 selected cytokines to identify differentially regulated or activated cytokine proteins in GTML and GTS tumor cells. Interestingly, while VEGF and IGFBP2 were dramatically reduced, IL12B and IGFBP5 were increased more than three-fold in recurrent cells (Figs. 17D and 24D). Compared to all other cytokines assayed, IGFBP5 was upregulated in GTS cells but correspondingly elevated in supernatants of GTS cells as well. IGFBP5 expression correlates with increased inflammation, IL6/STAT3 signaling and IFN-gamma levels, and induces cell death and senescence in a manner dependent on the p53 pathway and on the DNA damage response. When studying the levels of IGFBP5 in Group 3 and Group 4 patients it was significantly correlated with poor prognosis (Fig. 17E) suggesting that our observation in our mouse tumor recurrence model is of clinical significance.

In order to see if the expression profile in SOX9-positive recurring cells and SOX9-positive cells in primary GTML we compared scRNA-Seq data of SOX9-positive recurrent cells to those of primary tumors. Evidently, SOX9-positive cells were more numerous in recurrent GTS cell lines (~39 %) as compared to primary GTML cell lines (~5 %) (Fig. 13F); and high SOX9 expression appeared again to correlate with the non-proliferating cells in the G0/G1 cell cycle phase (Fig. 24E) and mostly mutually exclusive from high MYCN expression (Fig. 17F). The most SOX9-positive recurrent cells, similar to the most SOX9-positive primary cells (as shown in Figs. 13G and 13H), also showed a more quiescent signature than low-SOX9/high-MYCN or cells with intermediary expression between SOX9 and MYCN (Figs. 24F and 24G). However, IGFBP5 was found to be significantly enriched only in SOX9-positive recurrent cells in line with the results from our cytokine assay (Fig. 17D).

### SOX9 inhibits MYC in therapy-resistant cells and is essential for driving recurrence

Culturing GTS cells in dox, forcing overexpression of MYCN from the SOX9 promoter, is tolerated but slows down the growth of GTS tumor cells in vitro (Fig. 25A). To overcome the problems with poor MYCN antibodies for IHC, we decided to study tumor heterogeneity at the mRNA level. We used the RNAscope technique to stain specific RNA sequences for mouse SOX9 mRNA and for human MYCN mRNA (the MYCN gene in the TML transgene is the human copy) *in situ.* Both primary GTML and relapsed GTS tumors stained strongly for MYCN and fewer cells were positive for SOX9 (Figs. 18A and 18B). Similar to results found from our scRNA-Seq analysis, cells that were distinctly positive for SOX9 mRNA rarely expressed high levels of MYCN RNA unlike the SOX9-negative cells surrounding them (Figs. 18A and 18B).

In order to see if this heterogeneity is also seen in human MB cells, we introduced lentiviruses with puromycin resistance that can express GFP under control of the SOX9 promoter in MYC amplified Group 3 MB002 cells. Again, a small percentage of MB002 cells selected with puromycin were SOX9-positive (Fig. 25B). We sorted the GFP-positive population from the GFP negative population. With qPCR analysis we found that SOX9 levels were significantly increased together with the mRNA levels of cell cycle regulator p27 (CDKN1B) in SOX9 expressing GFP-positive sorted cells. MYC expression was on the other hand significantly reduced in this cell population (Fig. 25C). This further suggests that SOX9-positive MB cells have lower levels of MYC and an inhibited cell cycle.

Next, we checked if SOX9 is responsible for downregulation of MYC in MYC amplified human tumor cells. We overexpressed inducible wild-type (WT) SOX9 and mutationally stabilized (CPDmt) SOX9 in MYC-amplified MB002 primary Group 3 MB cells cultured in serum-free media. Strikingly, induction of SOX9 downregulated MYC protein already after 8 hours of treatment and brought it to almost undetectable levels after 24 h. The mutationally stabilized form of SOX9 decreased MYC levels even faster (Fig. 18C). However, the effect of the SOX9-driven suppression of MYC was reversible as dox release reactivated MYC in the cells after a few days in normal culture conditions (Fig. 18D). In order to study the regulation of SOX9 and MYC proteins *in vivo,* we introduced a cohort of GTS mice where mice did not receive 30 days of dox treatment after primary tumor development but instead were continually treated with dox for 6 months, resulting in tumor regression and no recurrences in these animals (n=5; Fig. 25D). This suggests that continuous SOX9 expression promotes quiescence over MYCN-driven tumor proliferation *in vivo* and implies that SOX9 expression release is a necessity for MYCN-driven tumor recurrence as similarly shown in SOX9-driven breast cancer metastatic progression.

In order to know if MYC was directly regulated by SOX9 we checked mRNA levels and the methylation status of MYC after 8 h of SOX9 overexpression in MB002 cells. First, we studied our old data generated after stable overexpression of SOX9 in MB002 cells. Neither overexpression of wild-type (WT) nor stabilized (CPDmt) SOX9 showed any immediate decrease in MYC levels (Fig. 25E). The MYC promoter (0-1.5 kb upstream of TSS) was further not methylated following 8 hours of SOX9 expression as seen using EPIC array analyses (Fig. 25F). However, at 24 hours of SOX9 induction (Fig. 25G) MYC mRNA was significantly downregulated as seen using qPCR analyses (Fig. 25H) perhaps suggesting an indirect regulation. We next checked if SOX9 regulates MYC post-translationally. However, SOX9 overexpression followed by cycloheximide chase assays showed no difference in MYC protein stability (Figs. 25l and 25J) although it suppressed MYC phosphorylation (at residues T58 and 252), total MYC levels and PCNA levels (proliferation marker) (Fig. 25K). To summarize, we found that forced SOX9 expression is indeed decreasing the MYC levels in MB cells, but this is likely not a direct regulation but perhaps rather a consequence of anti-proliferative signaling.

We hypothesized that few SOX9-positive cells in the primary GTML tumor survived the dox treatment and caused the less dox sensitive recurrence in GTS tumors. We tested this *in vitro* by treating GTML and pre-GT20 and pre-GT21 cells (cells from two individual primary tumors in GTS system) with dox for 5 days and measured proliferation every other day for an extended time without dox. After 8-10 days in culture, GTML cells had completely stopped proliferating and after 12-14 days pre-GTS cells had started to recover (Fig. 18E). While GTML cells died, dox-treated pre-GTS cells completely recovered from this temporary dox treatment, could be serially passaged for several generations and further showed cell proliferation similar to parental pre-GTS cell lines (not shown). The results further ruled out the possibility that SOX9-positive cells were non-tumorigenic normal brain cells that have infiltrated the brain tumor bulk. In order to see if SOX9 was required for tumor relapse, we used CRISPR knock-out and depleted SOX9 in pre-GT20 cells. A single gSOX9 clone (pre-GT20 gSOX9) was compared to two single clones (pre-GT20 Ctrl1 and Ctrl2) in where a CRISPR-Cas9 control was used (Fig. 25L). When transplanted into the mouse brain these three lines all generated brain tumors at a similar time point (around three weeks after injecting 100 000 cells) *in vivo.* However, when culturing these three lines to study their recurrence capabilities, we found that the pre-GT20 gSOX9 line did not survive the dox rescue (Fig. 18F) proving that SOX9 is in fact essential for rescuing cells during this dox-driven recurrence.

After dox treatment, GTML tumor cells are dying quickly and already at 48 h viability are more than halved. This is also a critical time point in where SOX9-positive cells will avoid treatment and start developing resistant cell clones. To detect early changes in surviving recurrent cell clones we compared RNA-Seq profiles of pre-GTS and GTML cells treated with dox for 48 hours to untreated control cells. MYCN together with general cell cycle markers (e.g., Mki67, Aurka and Ccne1) were quickly downregulated in both GTML and pre-GTS cells as compared to SOX9 that was upregulated in both dox-treated GTML and pre-GTS cells (Fig. 18G). Strikingly, gene sets that were differentially enriched in dox-treated pre-GTS cells as compared to dox-treated GTML cells after 48 h dox included hallmarks for apoptosis, hypoxia, Notch pathway signaling, and EMT (Fig. 18G). As we showed that SOX9-positive cells survive dox treatment we expected to find genes involved in inhibition of cell death. For example, the anti-apoptotic regulator Bcl2 was differentially expressed in dox-treated pre-GTS cells but downregulated in dox-treated GTML cells (Figs. 18G and 18H). Further, IGFBP5, found in the gene set of negative regulation of cell proliferation and acting downstream of the IL-6/JAK/STAT3 pathway was upregulated in pre-GTS cells which correlated well with its upregulation in SOX9-positive GTS cells in our scRNA-Seq data and cytokine array analysis (Figs. 18G and 18H). The Notch pathway and Twist1, which are drivers of leptomeningeal dissemination of these tumors, were also significantly elevated in dox treated pre-GTS cells (Figs. 18G and 18H).

In GTML cells, cell cycle genes/mitotic markers were instead enriched (Figs. 18G and 18H). When comparing RNA-Seq data from dox-treated GTML vs pre-GTS cells we found that the loss of DNA-repair genes was downregulated (similar to results in Fig. 17B), except for MGMT that was upregulated, in pre-GTS cells (Fig. 18H). All in all, our data suggests that rare SOX9-positive tumor cells are starting to reconstitute recurrent cell clones already 48 hours after oncogene targeted dox treatment.

### MB recurrences respond to MGMT inhibition and to doxorubicin therapy

High SOX9 levels correlated to drug resistance in MYC-driven MB. Therefore we treated pre-GTS tumor spheres with cisplatin and saw decreased cell survival and massive apoptotic cell death; however we also found a significant upregulation and increase in the number of SOX9-positive cells that were surviving the treatment after 72 h in culture (Fig. 19A). SOX9 expression was similarly upregulated in MB biopsies from patients given standard therapy as compared to expression in treatment-naive patients (Fig. 26A).

MGMT is a dealkylating DNA methyltransferase important in DNA repair. It is implicated in treatment resistance in both MB and in glioblastoma (GBM) and is significantly upregulated in recurrent GBMs as compared to matched primary patient samples. When MGMT is silenced due to promoter methylation, alkylating agents such as Temozolomide are not counteracted by the cancer cells' inherent capability to repair its DNA. However, in GTS cells our GSEA analysis (Fig. 17B) revealed a significant downregulation of Trp53 (Fig. 26B) that has a central role in the DNA repair response and is lost in MB recurrence. Typical DNA repair gene sets involved in common DNA repair pathways (Base excision repair, Nucleotide excision repair, Recombinational repair and Mismatch repair) were exemplified by significant downregulation of genes like Brca2, Rad51, Parp1, Xrcc2, Fancb and Fancc in GTS recurrences (Figs. 26C to 26H). By contrast, MGMT that is involved in a direct reversal of DNA repair pathway was instead found to be significantly elevated in recurrent GTS tumors compared to GTML tumors, and especially upregulated in fGTS tumors (Fig. 19B). High MGMT levels further results in a significantly worse overall survival in Group 3 gamma and Group 4 alpha (Fig. 19C) MB subtypes, typically driven by MYC and MYCN amplifications respectively. When overexpressing SOX9 in human MB cells we also found a fast upregulation of MGMT, suggesting that SOX9 is promoting its expression (Fig. 19D). We decided to use ATAC-Seq to study the genes regulated by forebrain metastatic spread in parallel with RNA-Seq data. We could see that fGTS tumors were more migratory and less proliferative, correlating with enrichment of pro-migratory and less mitotic gene sets. The MGMT promoter region was opened up in the more SOX9-positive fGTS cells (GT21) (Fig. 26I) as compared to hGTS cells (GT20) as shown with ATAC-Seq. (Fig. 19E).

In order to further explore the mechanism by which mRNA expression of MGMT is regulated, we investigated its methylation status in a set of 30 (11 Group 3 and 19 Group 4) paired primary and relapsed MB samples. Particularly, we focused on two CpG probes, which have previously been used to study the association between MGMT promoter methylation and gene expression. Based on these probes we found that 29 out of the 30 MB patients displayed hypomethylation already in the primary tumors, while the single patient with hypermethylated MGMT promoter underwent demethylation upon recurrence (data not shown). Lacking a methylation-based mechanism to target in order to downregulate MGMT in MB, we thus focused on targeting MGMT on the protein level. Intriguingly, a clinically relevant and potent inhibitor of MGMT, Lomeguatrib, significantly decreased proliferation of primarily GTS cells but did not show any efficacy in GTML cells *in vitro* (Fig. 19F). In combination with the alkylating agent ThioTEPA, commonly used in high-risk Group 3 MB therapy, Lomeguatrib decreased proliferation of GTS tumor cells even further (Fig. 26J).

A GSEA against a database of genetic and chemical perturbations revealed several drug signatures differentially enriched between forebrain and hindbrain GTS tumors and suggested that forebrain GTS might be less sensitive to Gefinitib inhibition and doxorubicin treatment (Fig. 19G). These findings were strengthened by ATAC-Seq data, which also indicated that genes with more open chromatin in forebrain GTS-2 cells significantly overlapped with corresponding doxorubicin-related (APOPTOSIS BY DOXORUBICIN_DN) gene sets (Fig. 19H). As mentioned, the EGFR inhibitor Gefitinib was suggested to affect forebrain tumors with increased dissemination but was however not specific for targeting forebrain GTS cells in our hands (not shown). Similarly, the anthracycline doxorubicin was suggested to be a specific drug for recurrent cells and when fGTS cells were treated with 200 nM doxorubicin, they were completely depleted after 72 h as compared to GTML and hGTS cells where nearly half of the cells were still viable (Fig. 19I). Combined, these observations suggest that rare SOX9-positive cells in Group 3 and Group 4 MB show a distinct set of signaling pathways reflecting a cancer clone that is more resistant to standard drugs. These clones contain malignant, dormant cells critical to further explore when modelling recurrence processes or for understanding treatment resistance mechanisms.

### Discussion

Recurrent tumors and metastasis are associated with poor prognosis in MB. It was recently shown that SOX2 and OLIG2 positive cells driven by Ptch1 loss were capable of propagating SHH-dependent brain cancer and in inducing recurrence. However, SOX2 and OLIG2 do not correlate with poor prognosis in non-WNT/non-SHH MBs. In the present study we focus on recurrence mechanisms of the more numerous Group 3 and Group 4 tumors that represent approximately two thirds of all MB cases and that are clearly different from WNT or SHH MB when analyzed with methylation profiling.

Non-proliferative SOX9-positive tumor cells have previously been implicated in head and neck tumor dormancy by a SOX9-driven quiescence program dependent on the retinoic acid pathway or by SOX9-driven breast tumor latency indirectly driven by suppression of MYC proteins. Based on the differentially expressed genes we found pathways that are overrepresented in the recurrent GTS tumors as well as in patient samples. Pathways involved in cell migration, motility, adhesion and differentiation were overexpressed in the recurrent tumors. The upregulation of the chemokine ligand CCL2 and its receptor CCR2 in GTS tumors is striking and correlates well with recent reports of MB metastasizing through circulating tumor cells in the blood. Similarly, the upregulation of typical immune escape markers like PD-L1 and Lgals9 suggest that recurrences indeed try to protect themselves against the immune system in this fully immunocompetent model.

We show that metastases compared to primary tumors have much higher levels of SOX9 and are less proliferative. Such silent cells avoid conventional chemotherapy and could eventually cause relapse. The downregulation of p53 and numerous DNA repair pathways observed in GTS tumors are most likely an escape route for tumors to avoid getting trapped by the DNA damage repair machinery. By contrast, MGMT that is involved in the direct reversal repair pathway was found to be highly expressed in GTS recurrences as compared to GTML cells, and MGMT further correlated with worse prognosis in Group 3 and Group 4 patients. MGMT seemed to have a functioning role in recurrent tumors as its inhibition successfully potentiated the effects of standard therapy given to high risk MYC amplified Group 3 patients. Our GSEA from our combined sequencing efforts also predicted doxorubicin as a prominent therapy for the more metastatic relapses and it indeed showed to be selectively targeting recurrent tumor cells over primary brain tumor cells.

The approach of combining a TetOFF system representing the bulk cells of the tumor with a TetON system representing distinct clones of dormant tumor cells can be applied for studying heterogeneity in other cancer models and especially for evaluating the properties of defined cell types within the tumor. By comparing the relapsed GTS tumors from the combined TetOFF-TetON mouse MB model with paired primary-recurrent human MB samples we identified fundamental cancer proteins upregulated specifically in relapsing tumor cells. Our data confirms that relapsing cells avoid therapy by entering into a dormant quiescent state. We also concluded that the SOX9-positive, resistant tumor cells are plastic and change their appearance when they are involved in the relapse process. This specifically categorize the SOX9-positive cells, that release chemokines like IGFBP5, which correlates with poor prognosis in Group 3 and Group 4 tumors and might give tumors a survival advantage. However, by identifying specific recurrence pathways, we have shown that relapsing tumor cells can become vulnerable to doxorubicin and MGMT inhibitors.

### EXAMPLE 2

The SOX9 response element (S9RE) reporter uses four copies of a 49-bp chondrocyte specific intronic enhancer segment from the human type II collagen alpha1 (*Col2a1*) gene with a minimal promoter to express luciferase. The minimal promoter alone delivers negligible expression, but when downstream of S9REs, it drives the expression of luciferase when SOX9 is expressed. The result is the ability to measure SOX9 expression by luciferase activity.

### Results

In cultured HEK-293T cells co-transfected with S9RE reporter gene construct and SOX9, SOX9 was able to induce luciferase activity (about 2000-fold) compared to the minimal promoter alone (control). In the absence of the putative SOX9 binding site (mutS9RE), SOX9 lost the ability to induce reporter gene expression. Other SOX family members, such as SOX6, were unable to activate the S9RE reporter construct (Fig. 27). The S9RE reporter cell-based assay can detect a range of luciferase activity (Fig. 28).

Human primary medulloblastoma MB002 cells express very low levels of endogenous SOX9. Treatment with doxycycline induced expression of mutant SOX9-T236/240A in MB002 SOX9-CPDmt cells (EMBO J (2016) 35(20): 2192-2212). These mutations in the CDP motif result in loss of interaction with FBXW7 and stabilization of SOX9 (EMBO J (2016) 35(20): 2192-2212). Induction of SOX9 in these medulloblastoma cells, strongly activated the S9RE reporter (Fig. 29), whereas there was no induction of the minimal promoter alone (control) even in the presence of doxycycline.

In breast cancer cell lines, expression of transcription factor SOX9 was much higher in the more malignant estrogen-resistant MDA-MB-231 (triple negative breast cancer cells) cells than in estrogen-sensitive MCF-7 cells (luminal, ER-positive breast cancer cells) (Fig. 30A). In the matched pair of Group 4 medulloblastoma cell line, the recurrent CHLA-01R-MED expresses more SOX9 than the primary CHLA-01-MED cell line (Fig. 30B). Both cell lines were derived from the original tumor (CHLA-01-MED) and the pleural effusion recurrence (CHLA-01R-MED) of the same patient.

### Materials and Methods

### Cell Culture

HEK-293T, MDA-MB-231 and MCF-7 cells were maintained in Dulbecco's modified Eagle's medium (DMEM) (Gibco), supplemented with 10% FBS (Gibco) and penicillin/streptomycin (Sigma-Alderich). Subculturing of cells was carried out using 0.25% trypsin-EDTA solution (Sigma-Alderich).

Doxycycline-inducible MB002 SOX9-CPDmt (SOX9-T236/240A) cells were cultured as described previously (EMBO J (2016) 35(20): 2192-2212). Expression of the SOX9 was induced by addition of 100 ng/mL doxycycline.

CHLA-01-MED and CHLA-01R-MED were cultured n DMEM:F12 medium (Gibco) with 20 ng/mL human epidermal growth factor (Peprotech), 20 ng/mL human basic fibroblast growth factor (Peprotech) and 2% (v/v) B-27 supplement (Gibco).

### Transfection and luciferase assay

For luciferase assays, HEK-293T cells were plated into 96-well plates 24 hours before transfection. Each well was transfected with 30 ng of luciferase reporter construct, 1 ng Renilla luciferase construct, along with 30 ng (if not stated otherwise) of expression plasmids using TranslT-LT1 transfection reagent (Mirus). pU19 DNA was used as filler to equalize the total amount of DNA transfected per well. 48 hours after transfection, cells were lysed and Firefly luciferase and Renilla luciferase activity were measured using Dual-Glo luciferase assay system (Promega) by a luminometer (Wallac 1420 Victor Plate Reader, Perkin-Elmer or CLARIOstar Plus plate reader, BMGLabtech). The Firefly luciferase activity was normalized against Renilla luciferase activity. All experiments were performed in triplicate. For Western blot analysis, transfections were scaled up using 12-well plates and 10 times the amount of each plasmid (as described above).

The Neon Transfection System (Life Technologies) was used to transfect MB002 cells. For each replicate, 1 µg of plasmid DNA was delivered with Neon program #18 (950V/30 ms/2 pulses) to 200000 cells per 10 µL tip in Buffer R.

### Western Blot analysis

Whole cell lysates were separated on a NuPAGE 4-12% Bis-Tris protein gel (Invitrogen) and blotted onto a nitrocellulose membrane. The membrane was blocked with 5% Skim milk for 1 hour at RT, and subsequently exposed to specific primary antibodies [anti-SOX9 (1:500; R&D Systems), anti-SOX6 (1:1000, Santa Cruz Biotechnology] overnight at 4°C. anti-Cyclophilin B (1:1000, Abcam) was used to normalize for protein loading. The respective HRP-conjugated secondary antibodies and chemiluminescence detection kit (Thermo Fisher) was used for detection. The blots were imaged using Amersham 680 blot and gel imager

The embodiments described above are to be understood as illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible. The scope of the present invention is, however, defined by the appended claims.

## Claims

1. A viral vector comprising a suicidal gene under transcriptional control of a SOX9 promoter or of a promoter operatively linked to an enhancer element comprising at least one SOX9 binding site.

2. The viral vector according to claim 1, wherein the suicidal gene encodes a protein selected from the group consisting of herpes simplex virus-1 thymidine kinase (HSV-TK), cytosine deaminase, nitroreductase, caboxypeptidase G2, cytochrome P450, purine nucleoside phosphorylase, and a combination thereof, preferably HSV-TK.

3. The viral vector according to claim 1 or 2, wherein the viral vector is a viral vector selected from the group consisting of a lentivirus virus and an adeno-associated viral (AAV) vector, preferably an AAV serotype 9 (AAV9) vector.

4. The viral vector according to any of the claims 1 to 3, wherein the SOX9 binding site has the nucleotide sequence CATTCAT.

5. The viral vector according to any of the claims 1 to 4, wherein the enhancer element comprises at least one copy of a 49-bp chondrocyte specific intronic enhancer segment from type II collagen alpha 1 (*Col2a1*) gene comprising the SOX9 binding site, preferably wherein the enhancer element comprises, preferably consists of, the nucleotide sequence as defined in SEQ ID NO: 12, 13 or 14.

6. The viral vector according to any of the claims 1 to 5, wherein the enhancer element comprises multiple copies of the SOX9 binding site, preferably at least three copies of the SOX9 binding site and more preferably at least four copies of the SOX9 binding site, preferably wherein the enhancer element comprises, preferably consists of, the nucleotide sequence as defined in SEQ ID NO: 13 or 14.

7. The viral vector according to any of the claims 1 to 6, wherein the promoter operatively linked to the enhancer element is selected from the group consisting of human elongation factor 1α (hEF1α) promoter, cytomegalovirus (CMV) promoter, simian virus 40 early (SV40) promoter, human Ubiquitin C (UBC) promoter, phosphoglycerate kinase 1 (PGK) promoter, tetracycline-responsive element (TRE) promoter and CMV early enhancer/chicken β-Actin (CAG) promoter.

8. A medicament comprising a viral vector according to any of the claims 1 to 7 and a prodrug for use in treatment of a cancer disease in a subject, wherein the suicidal gene encodes a prodrug activating protein capable of converting the prodrug or a metabolite thereof into a cytotoxic agent.

9. The medicament for use according to claim 8, wherein
the suicidal gene encodes herpes simplex virus-1 thymidine kinase (HSV-TK); and
the prodrug is selected from the group consisting of ganciclovir (GCV), a prodrug of GCV, preferably valganciclovir (ValGCV), and a combination thereof.

10. The medicament for use according to claim 8 or 9, wherein
the viral vector is formulated for administration to the subject prior to, during and/or following exposing the subject to a cancer treatment; and
the prodrug is formulated for administration to the subject following administration of the viral vector to the subject.

11. The medicament for use according to claim 10, wherein the cancer treatment is selected from the group consisting of surgery, chemotherapy, radiation therapy, immunotherapy, hormonal therapy, and a combination thereof, preferably selected from the group consisting of surgery, chemotherapy, radiation therapy, and a combination thereof.

12. The medicament for use according to any of the claims 8 to 11, wherein the subject is suffering from a solid tumor cancer disease, preferably selected from the group consisting of a brain cancer, a lung cancer, a colon cancer, a skin cancer, a breast cancer, a renal cancer, a thyroid cancer, a gastrointestinal cancer, a pancreatic cancer, a liver cancer, a colorectal cancer, a bladder cancer, a gastric cancer, an ovarian cancer and a prostate cancer, preferably wherein the subject is suffering from a brain cancer, preferably a malignant brain cancer, and more preferably wherein the subject is suffering from a cancer disease selected from the group consisting of medulloblastoma (MB) and glioblastoma (GBM).

13. An *in vitro* method of predicting a response to a cancer treatment, the method comprising the steps of:
introducing (S40) *ex vivo* i) a nucleic acid molecule comprising a reporter gene under transcriptional control of a SOX9 promoter or of a promoter operatively linked to an enhancer element comprising at least one SOX9 binding site and a suicidal gene under transcriptional control of a *SOX9* promoter or of a promoter operatively linked to an enhancer element comprising at least one SOX9 binding site or ii) a first nucleic acid molecule comprising the reporter gene under transcriptional control of a SOX9 promoter or of a promoter operatively linked to an enhancer element comprising at least one SOX9 binding site and a second nucleic acid molecule comprising the suicidal gene under transcriptional control of a SO*X9* promoter or of a promoter operatively linked to an enhancer element comprising at least one SOX9 binding site into cancer cells obtained from a tumor sample from a subject, the suicidal gene encodes a prodrug activating protein capable of converting a prodrug or a metabolite thereof into a cytotoxic agent;
exposing (S41) *ex vivo* the cancer cells to a cancer treatment;
determining (S42) a first amount of SOX9 positive cancer cells following cancer treatment based on expression of the reporter gene;
adding (S43) *ex vivo* the prodrug to the cancer cells;
determining (S44) a second amount of SOX9 positive cancer cells following addition of the prodrug based on expression of the reporter gene; and
predicting (S45) a response of the subject to a cancer treatment based on the determined first and second amounts.

14. The *in vitro* method according to claim 13, wherein the introducing (S40) step comprises:
transducing (S50) *ex vivo* the cancer cells with a first viral vector comprising the first nucleic acid molecule; and
transducing (S51) *ex vivo* the cancer cells with a second viral vector according to any of the claims 1 to 7.

15. A kit for use in the *in vitro* method according to anyone 13 or 14, the kit comprises:
i) a nucleic acid molecule comprising a reporter gene under transcriptional control of a *SOX9* promoter or of a promoter operatively linked to an enhancer element comprising at least one SOX9 binding site and a suicidal gene under transcriptional control of a SOX9 promoter or of a promoter operatively linked to an enhancer element comprising at least one SOX9 binding site; or
ii) a first nucleic acid molecule comprising the reporter gene under transcriptional control of a *SOX9* promoter or of a promoter operatively linked to an enhancer element comprising at least one SOX9 binding site and a second nucleic acid molecule comprising the suicidal gene under transcriptional control of a SOX9 promoter or of a promoter operatively linked to an enhancer element comprising at least one SOX9 binding site; and
a prodrug, wherein the suicidal gene encodes a prodrug activating protein capable of converting the prodrug or a metabolite thereof into a cytotoxic agent.
